# EUROPEAN PATENT APPLICATION

(11) **EP 4 310 093 A1**
(43) Date of publication of application: **24.01.2024**
(21) Application number: 21931802.9
(22) Date of filing: 06.04.2021
(51) Int. Cl.: C07J 43/00, C07J 41/00, A61K 31/58, A61K 31/575, A61P 31/14, A61P 31/20, A23L 33/10

(54) **AMINO ACID DERIVATIVE, PREPARATION METHOD THEREFOR, AND PHARMACEUTICAL COMPOSITION FOR TREATING HEPATITIS, COMPRISING SAME**

(30) Priority: 19.03.2021 KR 20210035805
(71) Applicant: Prazertherapeutics Inc., Seoul 02455 (KR)
(72) Inventor: INN, Kyung-Soo, Goyang-si Gyeonggi-do 10491 (KR); KIM, Nam-Jung, Seoul 06356 (KR); LEE, Jong Kil, Seoul 02447 (KR); KIM, Kyeojin, Seoul 06356 (KR); KIM, Donghwan, Seoul 02803 (KR); KIM, Soyoung, Yongin-si Gyeonggi-do 16817 (KR); KIM, Hee Jin, Seoul 07982 (KR)
(74) Representative: dompatent von Kreisler Selting Werner - Partnerschaft von Patent- und Rechtsanwälten mbB
(86) International application number: PCT/KR2021/004291
(87) International publication number: WO 2022/196858

(57) **Abstract**

The present invention relates to a novel amino acid derivative, a preparation method therefor, and a pharmaceutical composition for treating hepatitis B or D, comprising same. A compound represented by chemical formula 1, according to the present invention, has the excellent effect of inhibiting the binding of HBV preS 1 peptide by binding to NTCP, so as to have the effect of inhibiting the intracellular entry and cell adhesion of HBV, and thus is usable as a composition for preventing, treating, or alleviating diseases caused by HBV and HDV infection.

## Description

### Technical Field

The present disclosure relates to a novel amino acid derivative, a preparation method thereof, and a pharmaceutical composition for treating hepatitis including the same.

### Background Art

Since the discovery in 1967 by Blumberg (awarded the Nobel Prize for Medicine for this contribution in 1976), hepatitis B virus (HBV) has been known to be one of viruses that cause damage to human in the highest infection rate in the world. HBV infection is accompanied by a diverse clinical course of transition to asymptomatic infection, chronic infection, cirrhosis, and hepatocellular carcinoma, thus increasing morbidity and mortality of chronic diseases. Chronic hepatitis, cirrhosis, and hepatocellular carcinoma caused by HBV infection are the No. 1 cause of death in men in their 50s in Korea, and the mortality rate of hepatocellular carcinoma, which is mostly caused by HBV infection, ranks 17^{th} in the world, overwhelmingly ranking first among OECD countries.

Hepatitis D virus (HDV) is a disease caused by hepatitis D virus. In 1977, Rizzetto et al. in Italy first discovered hepatitis D virus in the hepatocyte nucleus of patients having chronic hepatitis B. HDV is a defective RNA virus, very unusual in that it has a circular RNA genome that is only found in plant viruses. The nucleocapsid composed of RNA genes and HDV antigens is surrounded by an envelope composed of hepatitis B surface antigen (HBsAg), and proliferation of the virus is based on the double rolling circle mechanism. Acute hepatitis D develops in the form of co-infection of HDV and HBV, or in the form of polyinfection with HDV in people previously having chronic hepatitis B. Thus, HDV infection may occur in subjects with incidental HBV infection. In the case of polyinfection with HDV, it is known that the degree of liver damage is more severe than that of single infection by HBV, and it quickly progresses to cirrhosis. As such, for HBV infection, the apparent unmet medical needs are even more urgent in subjects having co-infection with HBV and HDV.

For these reasons, there is an urgent demand for effective treatment strategies for hepatitis B and hepatitis D.

Although the development of vaccines has made partial prevention of hepatitis B possible, current treatments for hepatitis B involve the use of nucleos(t)ide analog reverse transcriptase inhibitors (NRTIs) and interferon alpha. Unlike the therapeutic agents for hepatitis C, which can lead to complete cure, NRTIs target the later stages of the viral life cycle, and thus, they do not provide a fundamental cure due to the persistence of viral cccDNA. Furthermore, interferon also carries a high risk of side effects and resistance due to prolonged administration without achieving complete recovery. As a result, a new treatment strategy is urgently needed. Clinical trials have been continuously conducted to archive higher efficacy through co-administration of existing NRTI and interferon, but no combination therapy using existing therapeutic agents has reached high efficacy compared to mono therapy.

On the other hand, it has recently been demonstrated that Na+ Taurocholate Cotransporting Peptide (NTCP) functions as the host receptor during HBV infection, and by confirming that genetic removal of NTCP prevents viral infection, and the inhibitory effect of endogenous ligands on virus attachment, those makes NTCP is a target for suppressing viral infection. In the case of NTCP inhibitors, preS 1 peptides of viruses or TLR7 agonists for boosting the host immune function are currently under development, but there is no clinically applied therapeutic agent until now, and in particular, there has been no report on the development of novel synthetic low molecular weight compounds that target the binding between NTCP and PreS 1.

Therefore, in order to develop a fundamental treatment method through a therapeutic agent showing a novel mechanism targeting the early stage of viral life cycle different from the existing therapeutic agent to inhibit the same, the present inventors discovered, as a result of research on substances exhibiting antiviral effects in a new mechanism that inhibits the cell invasion process which is the earliest stage of viral infection, novel substances capable of inhibiting the intracellular entry of virus by blocking the NTCP binding of PreS 1, a viral receptor-binding peptide, thereby completing the present disclosure by determining that the substances above may exhibit an inhibitory ability that is tens of times higher than that of endogenous bile acid that is previously reported to inhibit PreS 1 binding.

### Disclosure of the Invention

### Technical Goals

An object of the present disclosure is to provide a novel compound having a therapeutic efficacy on hepatitis.

Another object of the present disclosure is to provide a pharmaceutical composition for preventing or treating hepatitis.

Another object of the present disclosure is to provide a health functional food composition for preventing or alleviating hepatitis.

### Technical Solutions

In order to achieve the above objects,

the present disclosure provides a compound in which amino acids are bound to bile acid, an enantiomer thereof, a diastereomer thereof, or a pharmaceutically acceptable salt thereof.

Further, the present disclosure provides a pharmaceutical composition for preventing or treating hepatitis, including a compound in which amino acids are bound to bile acid, an enantiomer thereof, a diastereomer thereof, or a pharmaceutically acceptable salt thereof.

Moreover, the present disclosure provides a health functional food composition for preventing or alleviating hepatitis, including a compound in which amino acids are bound to bile acid, an enantiomer thereof, a diastereomer thereof, or a food-acceptable carrier thereof. "

In addition, the present disclosure provides a method of treating hepatitis, including administering a therapeutically effective amount of a compound in which amino acids are bound to bile acid, an enantiomer thereof, a diastereomer thereof, or a pharmaceutically acceptable salt thereof to a patient in need of hepatitis treatment. Advantageous Effects

A compound represented by Chemical Formula 1 according to the present disclosure has an excellent effect of inhibiting the binding of HBV or HDV PreS1 peptides by binding to NTCP to inhibit intracellular entry and attachment of HBV or HDV, and thus is useful as a composition for preventing, treating, or alleviating diseases by HBV and HDV infection.

### Brief Description of Drawings

FIG. 1 is graphs showing results of identifying an inhibitory ability to inhibit the binding between PreS1 and NTCP expressing cell lines, for compounds in Example 1, Examples 38 to 56, and Comparative Example 1 according to the present disclosure.
FIG. 2 is graphs showing results of identifying an ability to inhibit the binding between PreS1 and NTCP expressing cell lines for compounds in Example 1 (LCA-Ile), Example 50 (LCA-Trp), Example 52 (LCA-Tyr), and Example 44 (LCA-Val) according to the present disclosure.
FIG. 3 is graphs showing results of identifying an ability to inhibit the binding between PreS1 and NTCP expressing cell lines, for compounds in Examples 1 to 3, Examples 6 to 11, Examples 14 to 15, Example 44, and Comparative Examples 1 to 6 according to the present disclosure.
FIG. 4 is graphs showing a binding inhibitory ability of a fluorescent-labeled preS1 peptide through fluorescence imaging, for compounds of Example 1 (LCA-Ile), Example 44 (LCA-Val), Example 2 (UDCA-Ile), and Example 3 (UDCA-Val) according to the present disclosure.
FIG. 5 is graphs showing results of identifying toxicity at a cellular level, for compounds in Example 1 (LCA-Ile), Example 44 (LCA-Val), Example 2 (UDCA-Ile), and Example 3 (UDCA-Val) according to the present disclosure.
FIG. 6 shows an experimental protocol for viral infection to evaluate an ability to inhibit intracellular entry (invasion) of HBV virus by performing Enzyme-linked immunosorbent assay (ELISA) analysis and Southern blot analysis for HBeAg.
FIG. 7 is a graph showing a result of identifying an HBeAg absorbance (O.D.) level through HBeAg ELISA analysis, for compounds in Example 1 (LCA-Ile), Example 44 (LCA-Val), Example 52 (LCA-Tyr), Example 51 (LCA-Ser), Example 55 (LCA-Glu), Example 2 (UDCA-Ile), Example 3 (UDCA-Val), Example 25 (LCA-Val-Trp), Example 10 (DCA-Ile), Comparative Example 1 (LCA), and Comparative Example 2 (UDCA) according to the present disclosure.
FIG. 8 is a graph showing results of identifying HBeAg DNA and relative replication rate (%) of quantified HBeAg DNA through Southern blot analysis, for compounds in Example 1 (LCA-Ile), Example 44 (LCA-Val), Example 52 (LCA-Tyr), Example 51 (LCA-Ser), Example 55 (LCA-Glu), Example 2 (UDCA-Ile), Example 3 (UDCA-Val), Example 25 (LCA-Val-Trp), Example 10 (DCA-Ile), Comparative Example 1 (LCA), and Comparative Example 2 (UDCA) according to the present disclosure.
FIG. 9 is graphs showing results of identifying an inhibitory ability to inhibit the binding between PreS1 and NTCP expressing cell lines, for compounds in Examples 4 to 5, Examples 12 to 13, Examples 16 to 17, and Comparative Examples 2 (UDCA), and Comparative Examples 5 (DCA) and 6 (CDCA) according to the present disclosure.
FIG. 10 is graphs showing results of identifying an ability to inhibit the binding between PreS1 and NTCP expressing cell lines by concentration for compound in Example 4 (UDCA-Trp) according to the present disclosure.

### Best Mode for Carrying Out the Invention

Hereinafter, the present disclosure will be described in detail.

### A compound, an enantiomer thereof, a diastereomer thereof, or a pharmaceutically acceptable salt thereof

The present disclosure provides a compound represented by the following Chemical Formula 1, in which amino acids are bound to bile acid, an enantiomer thereof, a diastereomer thereof, or a pharmaceutically acceptable salt thereof:

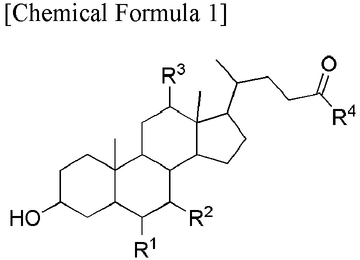

in the Chemical Formula 1,
R¹, R², and R³ are independently hydrogen or hydroxy;
R⁴ is 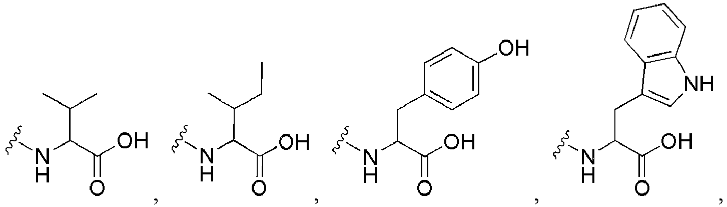 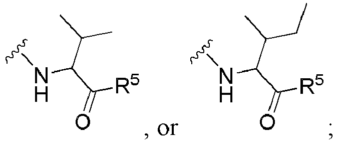 and
R⁵ is

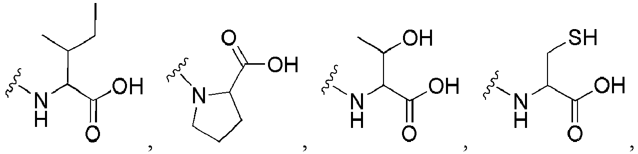

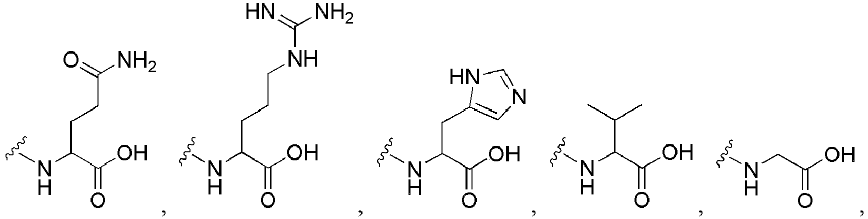

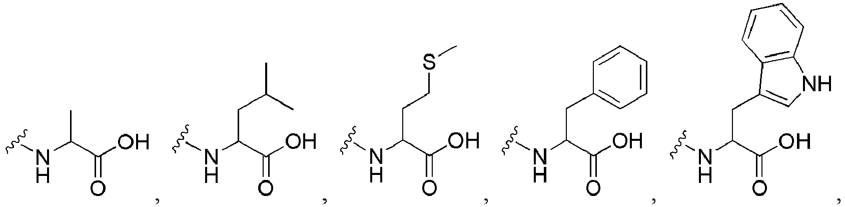

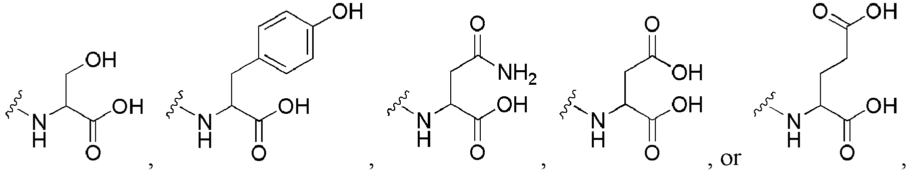

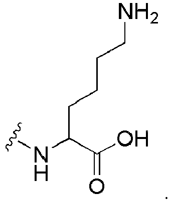

Preferably,
the R¹, R², and R³ are independently hydrogen or hydroxy;
the R⁴ is 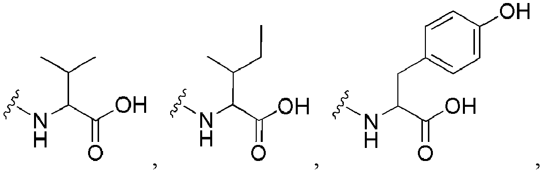 or 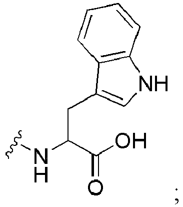 and
more preferably, one or more of the R¹, R², and R³ may be hydrogen.

In an example embodiment according to the present disclosure,
the R¹, R², and R³ are independently hydrogen;
the R⁴ is 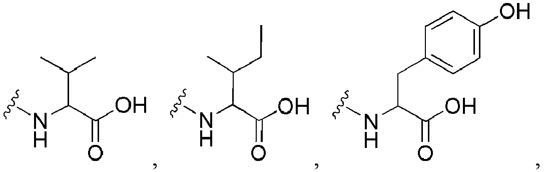 or 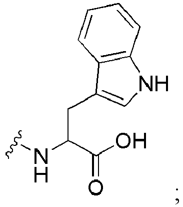 and
in an example embodiment according to the present disclosure, the bile acid is lithocholic acid (LCA), ursodeoxycholic acid (UDCA), cholic acid (CA), hyodeoxycholic acid (HDCA), deoxycholic acid (DCA), or chenodeoxycholic acid (CDCA).

In an example embodiment according to the present disclosure, a preferred example of the compound represented by Chemical Formula 1 includes the following group of compounds, and skeletal structures thereof are shown in Tables 1 and 2 below.
1) (2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Ile);
2) (2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (UDCA-Ile);
3) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (UDCA-Val);
4) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (UDCA-Trp);
5) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (UDCA-Tyr);
6) (2S,3R)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)pentanoic acid (CA-Ile);
7) (S)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)butanoic acid (CA-Val);
8) (2S,3R)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylpentanoic acid (HDCA-Ile);
9) (S)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylbutanoic acid (HDCA-Val);
10) (2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid (DCA-Ile);
11) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylbutanoic acid (DCA-Val);
12) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(1H-indol-3-yl)propanoic acid (DCA-Trp);
13) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(4-hydroxyphenyl)propanoic acid (DCA-Tyr);
14) (2S,3R)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (CDCA-Ile);
15) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (CDCA-Val);
16) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (CDCA-Trp);
17) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (CDCA-Tyr);
18) 2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)acetic acid (LCA-Val-Gly);
19) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ala);
20) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylbutanoic acid (LCA-Val-Val);
21) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-methylpentanoic acid (LCA-Val-Leu);
22) (2S,3R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylpentanoic acid (LCA-Val-Ile);
23) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-(methylthio)butanoic acid (LCA-Val-Met);
24) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-phenylpropanoic acid (LCA-Val-Phe);
25) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-indol-3-yl)propanoic acid (LCA-Val-Trp);
26) 1-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoyl)pyrrolidine-2-carboxylic acid (LCA-Val-Pro);
27) (S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ser);
28) (2S,3S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)butanoic acid (LCA-Val-Thr);
29) (R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-mercaptopropanoic acid (LCA-Val-Cys);
30) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(4-hydroxyphenyl)propanoic acid (LCA-Val-Tyr);
31) (S)-4-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-oxobutanoic acid (LCA-Val-Asn);
32) (S)-5-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-5-oxopentanoic acid (LCA-Val-Gln);
33) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)succinic acid (LCA-Val-Asp);
34) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanedioic acid (LCA-Val-Glu);
35) (S)-6-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)hexanoic acid (LCA-Val-Lys);
36) (S)-5-guanidino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanoic acid (LCA-Val-Arg); and
37) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-imidazol-4-yl)propanoic acid (LCA-Val-His).

The compound represented by Chemical Formula 1 of the present disclosure may be used in the form of a pharmaceutically acceptable salt, and as a salt, an acid additive salt formed by a pharmaceutically acceptable free acid is useful. The term pharmaceutically acceptable salt as used herein refers to any organic or inorganic additive salt of a base compound of Chemical Formula 1, in which the adverse effects caused by the salt do not degrade the beneficial efficacy of the base compound of Chemical Formula 1 in a concentration having an effective effect relatively non-toxic and harmless to a patient. For these salts, inorganic acids and organic acids may be used as free acids, wherein hydrochloric acid, bromic acid, nitric acid, sulfuric acid, perchloric acid, and phosphoric acid may be used as the inorganic acid, and citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, or malonic acid may be used as the organic acid. In addition, these salts include alkali metal salts (sodium salt, potassium salt, etc) and alkaline earth metal salts (calcium salt, magnesium salt, etc). For example, acid additive salt may include acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methyl sulfate, naphthylate, 2-naphsylate, nicotinate, nitrate, orotate, oxalate, palmitate, famoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, and zinc salt, of which hydrochloride or trifluoroacetate is preferred.

In addition, the compound represented by Chemical Formula 1 of the present disclosure includes not only pharmaceutically acceptable salts, but also all salts, isomers, hydrates, and solvates that may be prepared by conventional methods.

The additive salt according to the present disclosure may be prepared in a conventional manner, for example, the compound of Chemical Formula 1 may be prepared by dissolving in a water-miscible organic solvent, such as acetone, methanol, ethanol, or acetonitrile, adding an excess of organic acid or adding an aqueous acidic solution of an inorganic acid, and then performing precipitation or crystallization. Subsequently, preparation may be performed by evaporating the solvent or excess acid from the mixture and then drying the same to obtain an additive salt or undergoing suction filtration using the precipitated salt.

The present disclosure relates to any of all the tautomers of the compound represented by Chemical Formula 1 having anti-HBV activity, and it may be understood that the particular compound represented by Chemical Formula 1 may exist in a solvate form and a non-solvate form such as hydrated form. It may be understood that the present disclosure encompasses all such solvated forms having anti-HBV activity.

### Preparation Method of Compound

As shown in the following Scheme 1,
the present disclosure provides a preparation method of the compound represented by Chemical Formula 1, including:
dissolving compound 2 and compound 3 in an organic solvent and carrying out a reaction to obtain compound 4 (step 1); and
reacting compound 4 with sodium hydroxide or trifluoroacetic acid to obtain compound 1 (step 2); 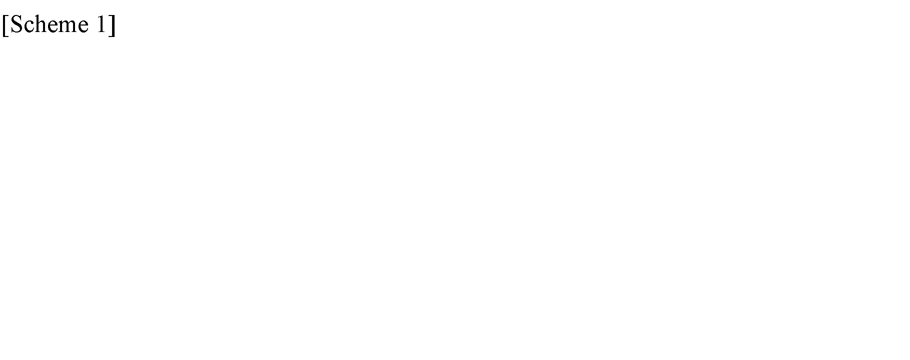 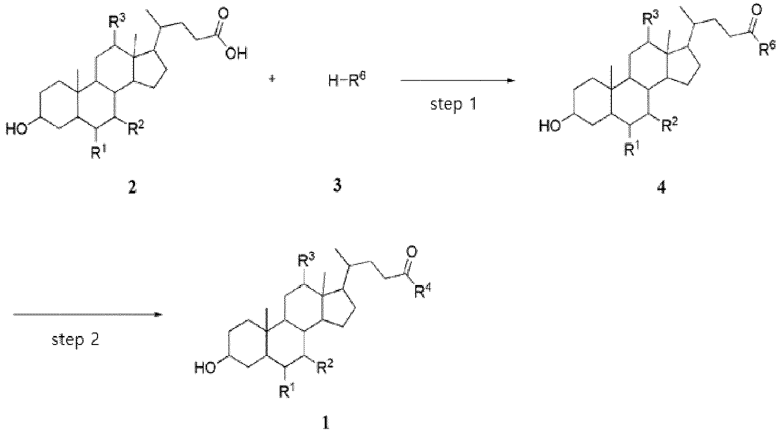
   in Scheme 1,
   R¹, R², and R³ are as defined above;
   R⁴ is R⁵;
   R⁵ is as defined above; and
   R⁶ is 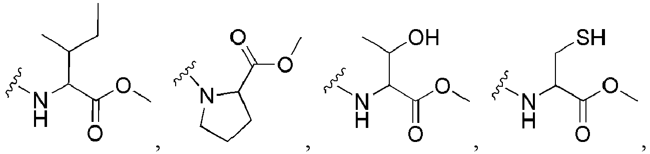 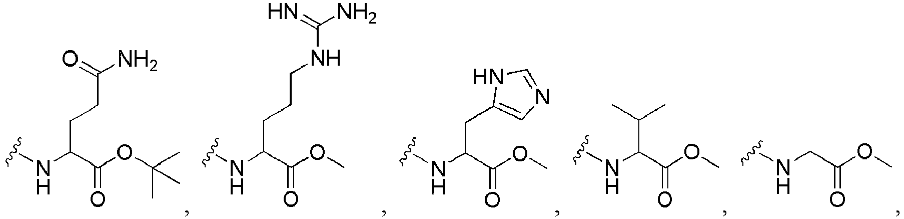 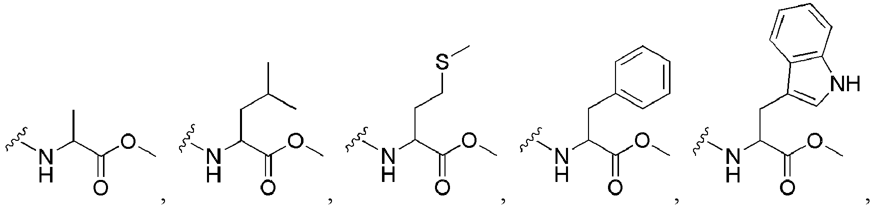 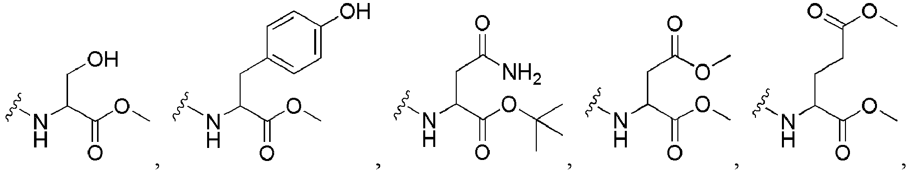 or

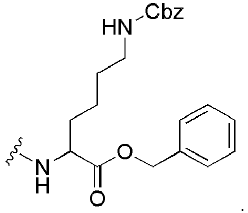

In the preparation method according to the present disclosure, preferably, one or more types of the R¹, R², and R³ may be hydrogen.

In the preparation method according to the present disclosure, the organic solvent in step 1 may be one or more types selected from the group consisting of dichloromethane (DCM), dimethylformamide (DMF), ethanol, tetrahydrofuran (THF), benzene, KOH/MeOH, MeOH, toluene, hexane, dimethylacetamide (DMA), diisopropyl ether, diethyl ether, dioxane, dimethyl sulfoxide (DMSO), acetone, and chlorobenzene, preferably, it may be one or more types selected from the group consisting of dichloromethane (DCM) and dimethylformamide (DMF).

In the preparation method according to the present disclosure, the step 1 may include further adding one or more types selected from the group consisting of N-methylmorpholine (NMM), 1-(3-dimethylaminopropyl)-3-ethylcarbodiimide (EDC), and 4-dimethylaminopyridine (DMAP) at the time of reaction.

In the preparation method according to the present disclosure, the reaction in step 2 may be performed in one or more types of solvents selected from the group consisting of dichloromethane (DCM), dimethylformamide (DMF), ethanol, tetrahydrofuran (THF), benzene, KOH/MeOH, MeOH, toluene, hexane, dimethylacetamide (DMA), diisopropyl ether, diethyl ether, dioxane, dimethyl sulfoxide (DMSO), acetone, and chlorobenzene, preferably, it may be performed in one or more types of solvents selected from the group consisting of dichloromethane (DCM) and ethanol.

In addition, as shown in the following Scheme 2,
the present disclosure provides a preparation method of the compound represented by Chemical Formula 1, including:
dissolving compound 5 and compound 3 in an organic solvent and carrying out a reaction to obtain compound 6 (step 1); and
reacting compound 6 with sodium hydroxide, trifluoroacetic acid or palladium on carbon (Pd/C) and hydrogen gas (H₂ gas) to obtain compound 1 (step 2):

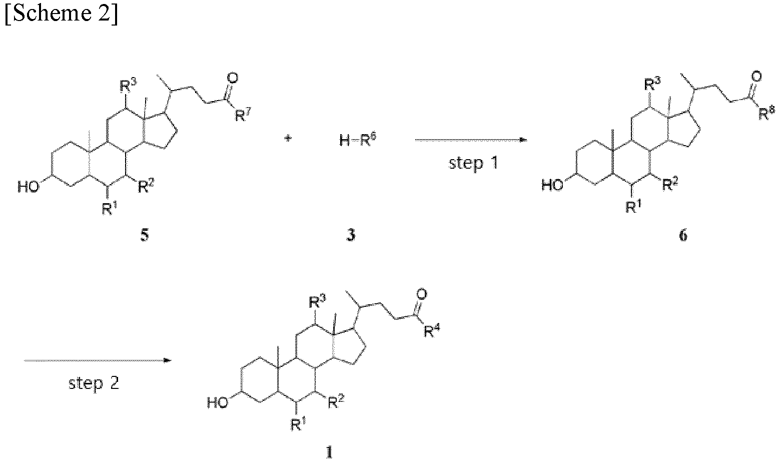

   in Scheme 2,
   R¹, R², and R³ are as defined above;
   R⁴ is 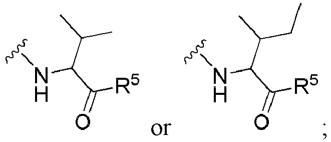
   R⁵ is as defined above;
   R⁶ is 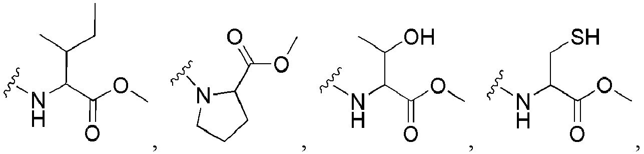 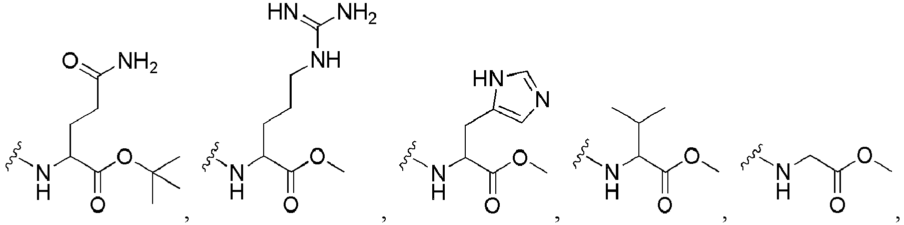 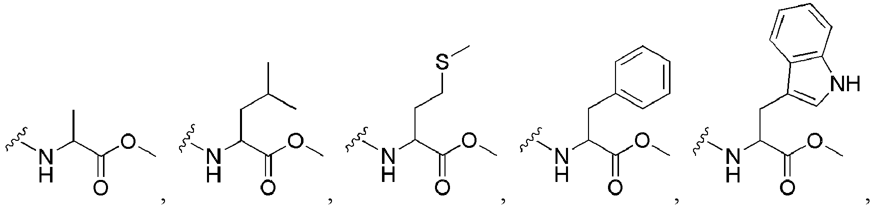 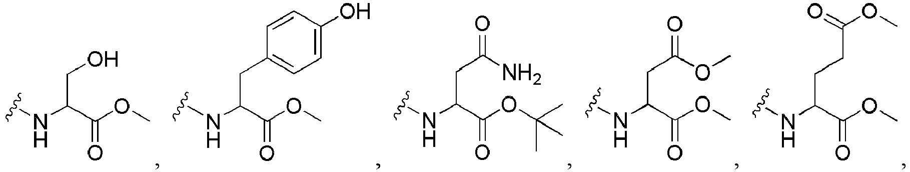 or 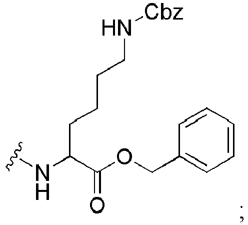
   R⁷ is 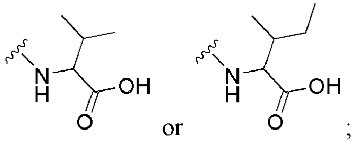 and
   R⁸ is

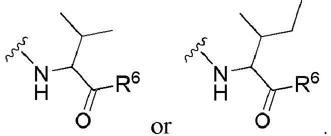

In the preparation method according to the present disclosure, preferably, one or more types of the R¹, R², and R³ may be hydrogen.

More preferably, the R¹, R², and R³ may be independently hydrogen.

In the preparation method according to the present disclosure, the organic solvent in step 1 of Scheme 2 may be one or more types selected from the group consisting of dichloromethane (DCM), dimethylformamide (DMF), ethanol, tetrahydrofuran (THF), benzene, KOH/MeOH, MeOH, toluene, hexane, dimethylacetamide (DMA), diisopropyl ether, diethyl ether, dioxane, dimethyl sulfoxide (DMSO), acetone, and chlorobenzene, preferably, it may be one or more types selected from the group consisting of dichloromethane (DCM) and dimethylformamide (DMF).

In the preparation method according to the present disclosure, the step 1 in Scheme 2 may include further adding one or more types of substances selected from the group consisting of 1-hydroxybenzotriazole (HOBt), hexafluorophosphate azabenzotriazole tetramethyl uronium (HATU), and N, N-diisopropylethylamine (DIPEA) at the time of reaction.

In the preparation method according to the present disclosure, the reaction in step 2 of Scheme 2 may be performed in one or more types of solvents selected from the group consisting of dichloromethane (DCM), dimethylformamide (DMF), ethanol, tetrahydrofuran (THF), benzene, KOH/MeOH, MeOH, toluene, hexane, dimethylacetamide (DMA), diisopropyl ether, diethyl ether, dioxane, dimethyl sulfoxide (DMSO), acetone, and chlorobenzene, preferably, it may be performed in one or more types of solvents selected from the group consisting of dichloromethane (DCM) and ethanol.

### Pharmaceutical Composition for Preventing or Treating Hepatitis

The present disclosure provides a pharmaceutical composition for preventing or treating hepatitis, including a compound represented by the following Chemical Formula 1, in which amino acids are bound to bile acid, an enantiomer thereof, a diastereomer thereof, or a pharmaceutically acceptable salt thereof:

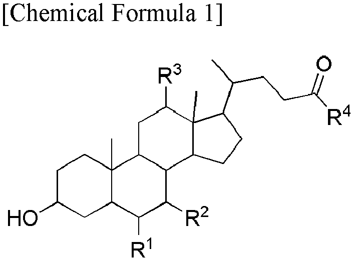

in the Chemical Formula 1,
R¹, R², and R³ are independently hydrogen or hydroxy;
R⁴ is R⁵, 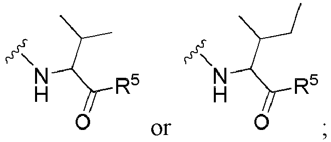 and
R⁵ is 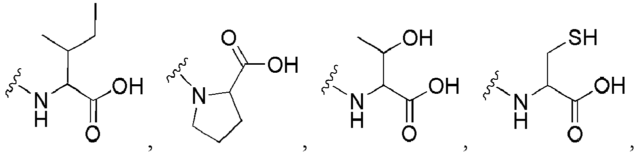 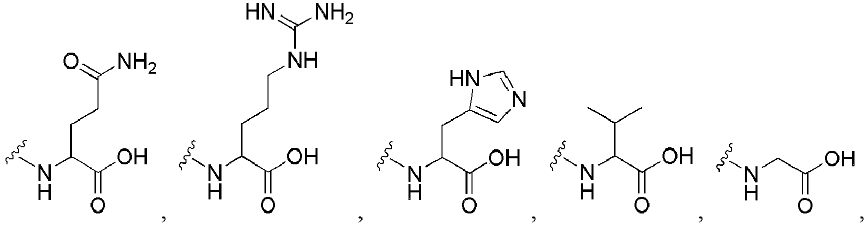 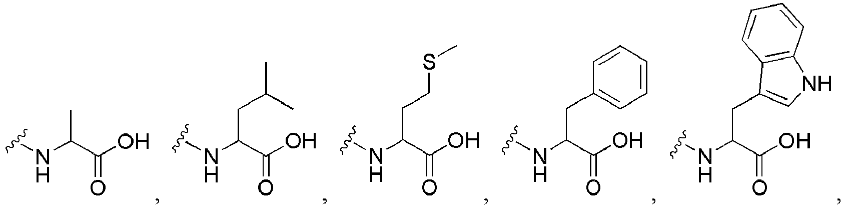 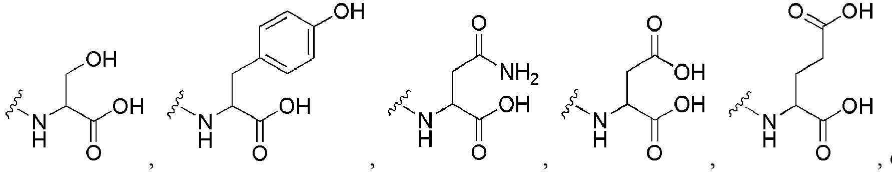 or

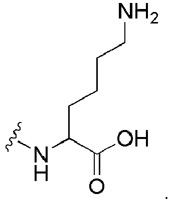

In the pharmaceutical composition according to the present disclosure, preferably, one or more types of the R¹, R², and R³ may be hydrogen.

A preferred example of the compound represented by Chemical Formula 1 may include the following group of compounds, and chemical structures thereof are shown in Tables 1 to 3 below.
1) (2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Ile);
2) (2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (UDCA-Ile);
3) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (UDCA-Val);
4) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (UDCA-Trp);
5) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (UDCA-Tyr);
6) (2S,3R)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)pentanoic acid (CA-Ile);
7) (S)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)butanoic acid (CA-Val);
8) (2S,3R)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylpentanoic acid (HDCA-Ile);
9) (S)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylbutanoic acid (HDCA-Val);
10) (2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid (DCA-Ile);
11) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylbutanoic acid (DCA-Val);
12) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(1H-indol-3-yl)propanoic acid (DCA-Trp);
13) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(4-hydroxyphenyl)propanoic acid (DCA-Tyr);
14) (2S,3R)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (CDCA-Ile);
15) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (CDCA-Val);
16) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (CDCA-Trp);
17) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (CDCA-Tyr);
18) 2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)acetic acid (LCA-Val-Gly);
19) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ala);
20) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylbutanoic acid (LCA-Val-Val);
21) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-methylpentanoic acid (LCA-Val-Leu);
22) (2S,3R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylpentanoic acid (LCA-Val-Ile);
23) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-(methylthio)butanoic acid (LCA-Val-Met);
24) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-phenylpropanoic acid (LCA-Val-Phe);
25) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-indol-3-yl)propanoic acid (LCA-Val-Trp);
26) 1-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoyl)pyrrolidine-2-carboxylic acid (LCA-Val-Pro);
27) (S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ser);
28) (2S,3S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)butanoic acid (LCA-Val-Thr);
29) (R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-mercaptopropanoic acid (LCA-Val-Cys);
30) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(4-hydroxyphenyl)propanoic acid (LCA-Val-Tyr);
31) (S)-4-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-oxobutanoic acid (LCA-Val-Asn);
32) (S)-5-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-5-oxopentanoic acid (LCA-Val-Gln);
33) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)succinic acid (LCA-Val-Asp);
34) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanedioic acid (LCA-Val-Glu);
35) (S)-6-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)hexanoic acid (LCA-Val-Lys);
36) (S)-5-guanidino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanoic acid (LCA-Val-Arg);
37) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-imidazol-4-yl)propanoic acid (LCA-Val-His);
38) 1-((R)-4-((3R,5R,8R,9S,10S,13R,14S,17R)-3-hydroxy-10,13-dimethyl-hexadecahydro-1H-cyclopenta[a]phenanthren-17-yl)pentanoyl)pyrrolidine-2-carboxylic acid (LCA-Pro);
39) (2S,3S)-3-hydroxy-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)butanoic acid (LCA-Thr);
40) (R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-mercaptopropanoic acid (LCA-Cys);
41) (S)-5-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-5-oxopentanoic acid (LCA-Gln);
42) (S)-5-guanidino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)pentanoic acid (LCA-Arg);
43) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(1H-imidazol-5-yl)propanoic acid (LCA-His);
44) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoic acid (LCA-Val);
45) 2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-acetic acid (LCA-Gly);
46) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-propanoic acid (LCA-Ala);
47) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Leu);
48) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-4-(methylthio)butanoic acid (LCA-Met);
49) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-phenylpropanoic acid (LCA-Phe);
50) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(1H-indol-3-yl)propanoic acid (LCA-Trp);
51) (S)-3-hydroxy-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)propanoic acid (LCA-Ser);
52) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(4-hydroxyphenyl)propanoic acid (LCA-Tyr);
53) (S)-4-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-4-oxobutanoic acid (LCA-Asn);
54) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)succinic acid (LCA-Asp);
55) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)pentanedioic acid (LCA-Glu); and
56) (S)-6-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)hexanoic acid (LCA-Lys).

In the pharmaceutical composition according to the present disclosure, the hepatitis may be hepatitis B or hepatitis D.

The compound represented by Chemical Formula 1 according to the present disclosure ensures excellent effects of inhibiting the binding between HBV PreS1 (Myr-GTNLSVPNPLGFFPDHQLDPAFGANSNNPDWDFNPNKDHWPEANQV-Lys(FITC)) and NTCP(Na⁺ taurocholate cotransporting peptide) expressing cell lines and/or inhibiting intracellular invasion (entry) and infection of HBV virus in a HepG2-NTCP cell, a human NTCP-expressing cell line (see Experimental Examples 1 to 4).

In the pharmaceutical composition according to the present disclosure,
the R¹, R², and R³ may be independently hydrogen or hydroxy;
the R⁴ may be 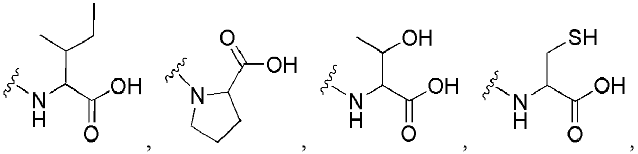 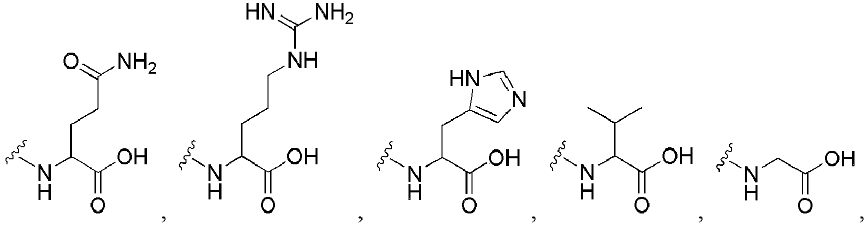 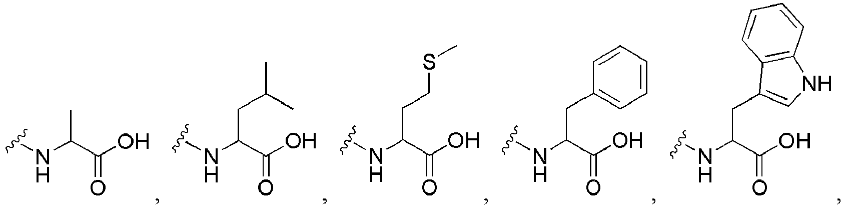 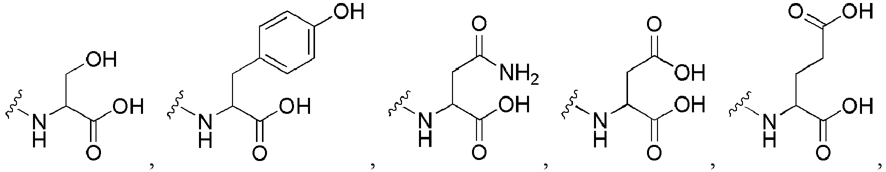 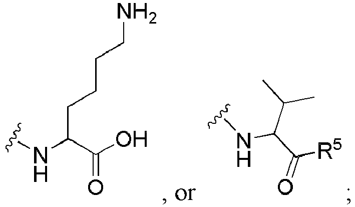 and
R⁵ may be

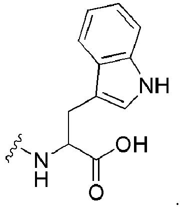

The present inventors identified effects in which the compounds inhibit the binding between HBV PreS1 (Myr-GTNLSVPNPL GFFPDHQLDPAFGANSNNPDWDFNPNKDHWPEANQV-Lys(FITC)) peptide and Na⁺ taurocholate cotransporting peptide (NTCP) and/or inhibit intracellular invasion (entry) and infection of HBV viruses (see Experimental Examples 1 to 4).

In the pharmaceutical composition according to the present disclosure,
preferably, the R¹, R², and R³ may be independently hydrogen or hydroxy;
at least two types of the R¹, R², and R³ may be hydrogen; and
the R⁴ may be

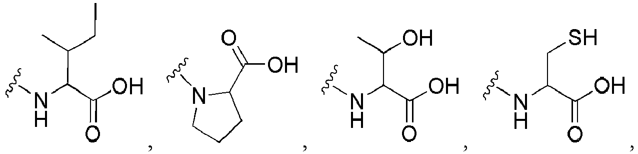

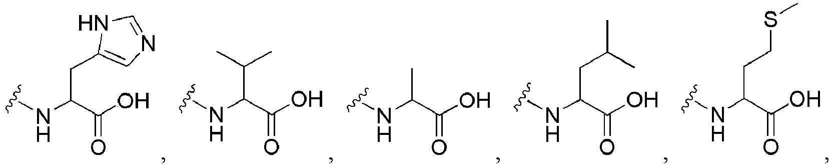

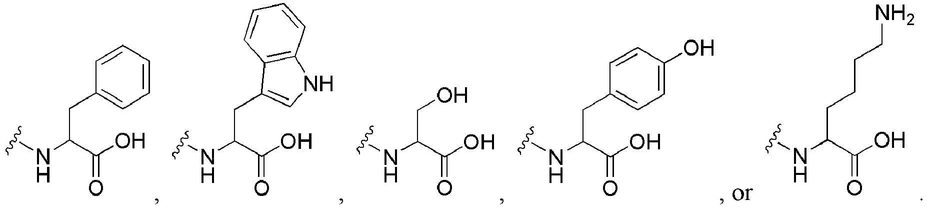

In this case, the compound represented by Chemical Formula 1 may exhibit, along with effects of inhibiting intracellular invasion (entry) and infection of HBV virus (see Experimental Example 4), effects of inhibiting the binding between HBV PreS1 peptide and NTCP, superior to existing lithocholic acid and derivatives thereof (see Experimental Examples 1 to 3).

In the pharmaceutical composition according to the present disclosure,
more preferably, the R¹, R², and R³ may be independently hydrogen or hydroxy;
at least two types of the R¹, R², and R³ may be hydrogen; and
the R⁴ may be

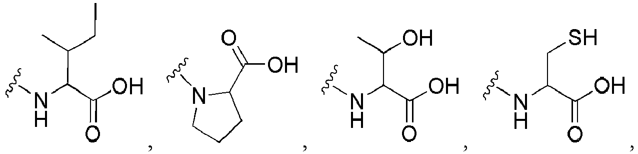

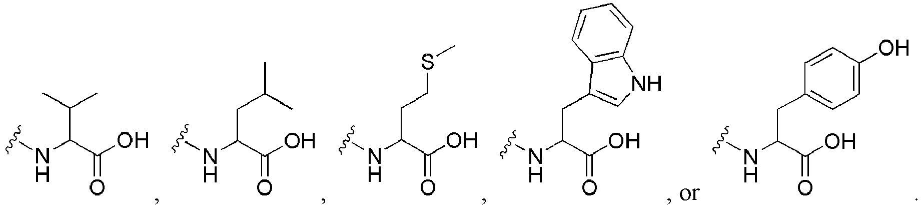

In this case, the compound represented by Chemical Formula 1 may have, along with effects of inhibiting intracellular invasion (entry) and infection of HBV virus (see Experimental Example 4), improved ability to inhibit the binding between HBV PreS 1 peptide and NTCP to derive inhibitory effects to a level less than 40% compared to a group solely treated with PreS1 (See Experimental Examples 1 to 3).

In the pharmaceutical composition according to the present disclosure,
more preferably, the R¹, R², and R³ may be independently hydrogen or hydroxy;
two or more types of the R¹, R², and R³ may be hydrogen; and
the R⁴ may be

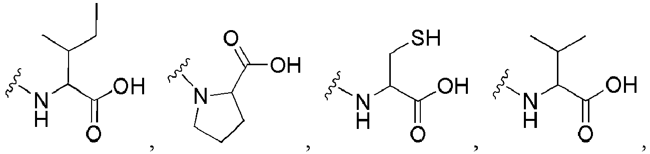

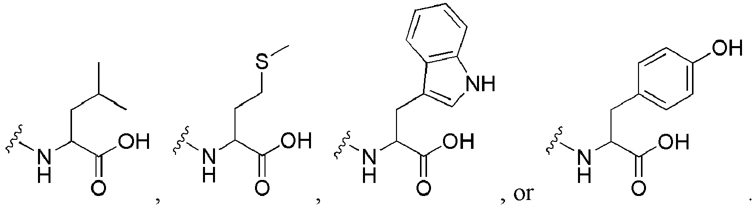

In this case, the compound represented by Chemical Formula 1 may have, along with effects of inhibiting intracellular invasion (entry) and infection of HBV virus (see Experimental Example 4), improved ability to inhibit the binding between HBV PreS 1 peptide and NTCP to derive inhibitory effects to a level less than 20% compared to a group solely treated with PreS1 (See Experimental Examples 1 to 3).

In the pharmaceutical composition according to the present disclosure,
more preferably, the R¹, R², and R³ may be independently hydrogen or hydroxy;
two or more types of the R¹, R², and R³ may be hydrogen; and
the R⁴ may be 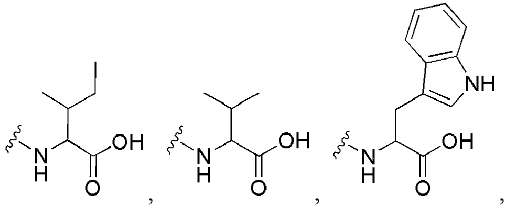 or

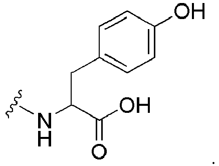

In this case, the compound represented by Chemical Formula 1 may have, along with effects of inhibiting intracellular invasion (entry) and infection of HBV virus (see Experimental Example 4), improved ability to inhibit the binding between HBV PreS 1 peptide and NTCP to derive inhibitory effects to a level less than 5% compared to a group solely treated with PreS 1 (See Experimental Examples 1 to 3).

In the pharmaceutical composition according to the present disclosure,
more preferably, the R¹ may be hydrogen;
the R² and R³ may be independently hydrogen or hydroxy;
one or more types of the R² and R³ may be hydrogen; and
R⁴ may be

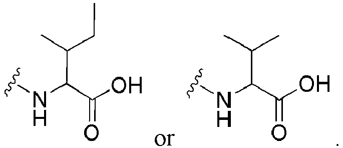

In this case, the compound represented by Chemical Formula 1 may have remarkably improved effects of inhibiting the binding between HBV PreS1 peptide and NTCP and inhibiting intracellular invasion (entry) and infection of HBV virus, compared to HBV-induced group and other derivatives having similar structures (see Experimental Examples 1 to 4).

According to an example embodiment of the present disclosure, it is particularly preferred that the compound represented by Chemical Formula 1 is one or more types of the following compounds:
1) (2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Ile);
2) (2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (UDCA-Ile);
3) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (UDCA-Val);
4) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (UDCA-Trp);
10) (2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid (DCA-Ile);
12) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(1H-indol-3-yl)propanoic acid (DCA-Trp); and
16) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (CDCA-Trp).

Therefore, the compound represented by Chemical Formula 1 according to the present disclosure has an excellent effect of inhibiting the binding of HBV PreS1 peptide by binding to NTCP and an effect of inhibiting intracellular entry and attachment of HBV virus through the effect above, such that the compound may be useful as a pharmaceutical composition for preventing or treating diseases caused by HBV infection.

The compound represented by Chemical Formula 1 according to the present disclosure may be used for treatment and progression inhibition in patients with chronic hepatitis B or hepatitis D, and in particular, it may be used to suppress new infections in patients who have undergone liver transplantation due to cirrhosis or hepatocellular carcinoma, and through a mechanism that targets the early stages of viral infection by NTCP targeting and inhibition of intracellular entry of HBV, it may be used for preventing or treating liver dysfunction, hepatitis, hepatocirrhosis, and hepatocellular carcinoma, associated with chronic hepatitis B or hepatitis D caused by HBV or HDV hepatitis.

In addition, the compound represented by Chemical Formula 1 according to the present disclosure may be used alone or in combination with existing therapeutic agents for disease treatment and inhibition of disease progression in patients with chronic hepatitis B or hepatitis D.

When the composition of the present disclosure is used as a medical product, the pharmaceutical composition including the compound represented by Chemical Formula 1, enantiomer thereof, diastereomer thereof, or pharmaceutically acceptable salt thereof as an active ingredient may be administered by being formulated in the form of various following oral or parenteral administration forms upon clinical administration, but is not limited thereto.

Formulations for oral administration include, for example, tablets, pills, hard/soft capsules, liquids, suspensions, emulsifiers, syrups, granules, and elixirs, and these formulations include diluents (e.g., lactose, dextrose, sucrose, mannitol, sorbitol, cellulose and/or glycine) and lubricants (e.g., silica, talc, stearic acid and magnesium or calcium salts thereof, and/or polyethylene glycol) in addition to the active ingredient. The tablet may also include binders such as magnesium aluminum silicate, starch paste, gelatin, methylcellulose, sodium carboxymethylcellulose and/or polyvinylpyrrolidine, and in some cases, it may include disintegrants such as starch, agar, alginic acid, or sodium salts thereof or effervescent mixtures and/or absorbents, coloring agents, flavoring agents, and sweetening agents.

In addition, the dosage of the compound represented by Chemical Formula 1 according to the present disclosure to the human body may vary depending on the age, weight, sex, dosage form, health status, and degree of disease of a patient, and based on an adult patient weighing 70 kg, it may generally be 0.1 ~ 1,000 mg/day, preferably 1 ~ 500 mg/day, wherein the dosage may be administered once or in several divided doses a day at a predetermined time interval according to the judgment of a doctor or pharmacist.

### Health Functional Food Composition for Preventing or Alleviating Hepatitis

The present disclosure provides a health functional food composition for preventing or alleviating hepatitis, including a compound represented by the following Chemical Formula 1, in which amino acids are bound to bile acid, an enantiomer thereof, a diastereomer thereof, or a food-acceptable carrier thereof:

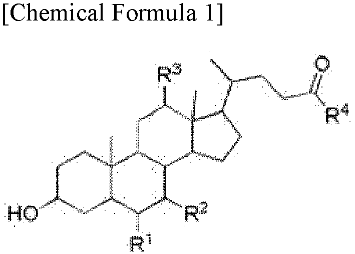

in the Chemical Formula 1,
R¹, R², and R³ are independently hydrogen or hydroxy;
R⁴ is R⁵, 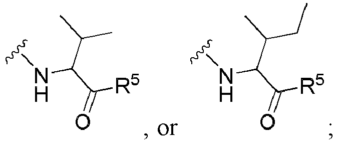 and
R⁵ is 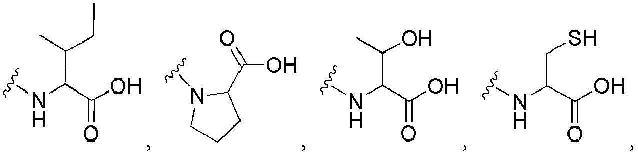 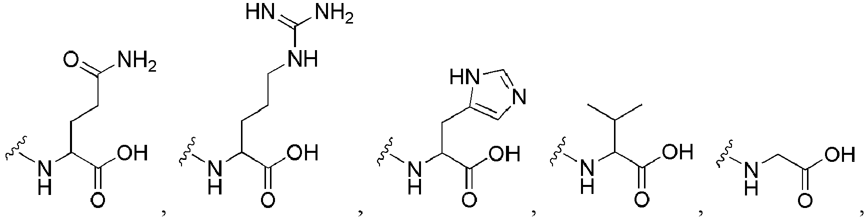 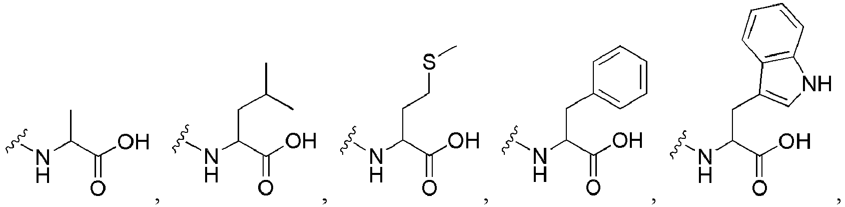 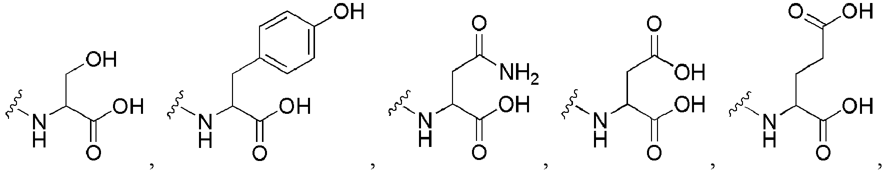 or

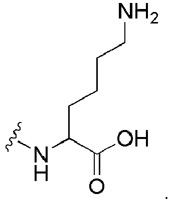

In the health functional food composition according to the present disclosure, preferably, one or more types of the R¹, R², and R³ may be hydrogen.

In the health functional food composition according to the present disclosure, the hepatitis may be hepatitis B or hepatitis D.

When the composition of the present disclosure is used as a health functional food, there is no particular limitation on the type of food. Examples of foods to which the composition of the present disclosure may be added include drinks, meats, sausages, bread, biscuits, rice cakes, chocolates, candies, snacks, confectionery, pizza, ramen, other noodles, chewing gums, dairy products including ice cream, various soups, beverages, alcoholic beverages and vitamin complexes, dairy products, and processed dairy products, including all the health functional foods in the usual sense.

The health functional food composition including the compound represented by Chemical Formula 1 of the present disclosure may be added to food as it is or used in combination with other foods or food ingredients and used appropriately according to conventional methods. The mixed amount of the active ingredient may be suitably determined according to the purpose of use thereof (for prevention or alleviation). In general, the composition in health functional food may be added in an amount of 0.1 to 90 parts by weight of a total weight of food. However, in the case of long-term intake for the purpose of maintaining health or controlling health, the amount may be below the above range, and the active ingredient may also be used in an amount beyond the range since there is no problem in terms of safety.

The health functional food composition including the compound represented by Chemical Formula 1 of the present disclosure has, as an essential ingredient in the indicated ratio, no particular limitation on other ingredients other than including the composition of the present disclosure and may include various flavoring agents or natural carbohydrates as additional ingredients like ordinary beverages. Examples of natural carbohydrates described above are monosaccharides, such as glucose and fructose; disaccharides, such as maltose and sucrose; and polysaccharides, such as conventional sugars such as dextrin and cyclodextrin, and sugar alcohols such as xylitol, sorbitol, and erythritol. Natural flavoring agents (thaumatin, stevia extract (e.g., rebaudioside A, glycyrrhizin, etc.)) and synthetic flavoring agents (saccharin, aspartame, etc.) may be advantageously used as flavoring agents other than foregoing ingredients. The ratio of natural carbohydrates is generally about 1 to 20 g, preferably about 5 to 12 g, per 100 of the health functional food composition of the present disclosure.

In addition to the above, the health functional food composition including the compound represented by Chemical Formula 1 of the present disclosure may include various nutritional supplements, vitamins, minerals (electrolytes), flavor agents such as synthetic flavor agents and natural flavor agents, colorants and enhancer (cheese, chocolate, etc.), pectic acid and salts thereof, alginic acid and salts thereof, organic acids, protective colloidal thickeners, pH adjusters, stabilizers, preservatives, glycerin, alcohol, and carbonating agent used in carbonated beverages. In addition, the health functional food composition of the present disclosure may include pulp for preparation of natural fruit juice, fruit juice beverages, and vegetable beverages.

These ingredients may be used independently or in combination. The ratio of these additives is not so important, but it is general to be selected in a range of 0.1 to about 20 parts by weight per 100 parts by weight of the health functional food composition including the effective substance of the present disclosure.

Hereinafter, the present disclosure will be described in more detail according to the following example embodiments. However, the following example embodiments are only illustration of the present disclosure, and the contents of the present disclosure are not limited by the following example embodiments.

### Modes for Carrying Out the Invention

Hereinafter, the present disclosure will be described in detail by the example embodiments. However, the following

example embodiments are merely for illustration of the present disclosure, and the content of the present disclosure is not limited by the following example embodiments.

### <Preparation Example> Instruments and Reagents

All starting materials and reagents used in Examples were used by purchasing from Aldrich, Alfa Aesar, TCI and the like. Various glasses used for reactions were dried in an oven at 80°C. Reactions dealing with compounds sensitive to air and moisture were carried out by injecting Ar gas. The progression pattern of reactions was observed by thin-layer-chromatography, UV light, p-anisaldehyde, and ninhydrin. Column chromatography was performed using silica gel 60 (230-400 mesh) in combination with solvents such as hexane, ethyl acetate, dichloromethane, and methanol.

¹H-NMR spectra were analyzed using Bruker Avance 400 (400 MHz for ¹H) spectrometers. ¹H NMR chemical shift value was expressed in parts per million (ppm) using tetramethylsilane (TM) as an internal reference. NMR signals were indicated as s (singlet), m (multiplet), d (doublet), t (triplet), q (quartet), and dd (doublet of doublets), and coupling constant was expressed in hertz (Hz).

### <Example 1> Preparation of (2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Ile)

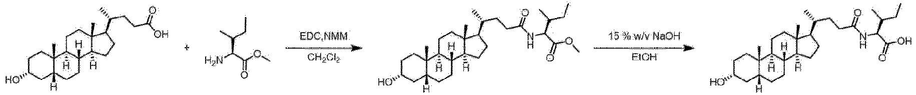

Starting materials including lithocholic acid (100 mg, 0.264 mmol) and L-isoleucine methyl ester hydrochloride (53 mg, 0.290 mmol), and N-methylmorpholine (NMM) (0.476 mmol) were dissolved in 6 mL of dry DCM. Thereafter, 1-(3-dimethylaminopropyl)-3-ethylcarbodiimide (EDC) (0.264 mmol) was added and a reaction was carried out overnight at room temperature after filling with Ar gas. When it was detected that the starting material disappeared using TLC, the reaction was stopped with 2N-HCl, and dilution was performed with DCM solvent. The organic layer was washed and extracted with DCM and H₂O. The extracted organic layer was dried using MgSO₄, and the solvent was removed using an evaporator. The reaction mixture was purified by column chromatography to obtain an intermediate (100 mg, 49%). To remove the protected part, the intermediate (91.5 mg, 0.182 mmol) was dissolved in 9 mL of ethanol, 6 mL of 15% w/v sodium hydroxide was added, and a reaction was carried out for 30 minutes. When it was detected that the starting material disappeared using TLC, ethanol was removed using an evaporator, and acidification was performed with concentrated hydrochloric acid at 0°C. The resulting precipitate was filtered through a filter and washed with water to obtain a final compound (Example 1; LCA-Ile) (86 mg, 97%).

(2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid; ¹H-NMR (400 MHz, DMSO-d6) δ = 0.60 (s, 3H, CH3), 0.80-0.90 (m, 13H), 0.99-1.22 (m, 11H), 1.29-1.39 (m, 7H), 1.46-1.55 (m, 2H), 1.58-1.82 (m, 6H), 1.82-1.88 (m, 1H), 1.99-2.08 (m, 1H), 2.15-2.23 (m, 1H), 3.32-3.35 (m, 1H), 4.15 (t, J = 7.0 Hz, 1H, CHCHCH3CH2), 4.45 (s, 1H, OH), 7.94 (d, J = 8.4 Hz, 1H, NH). 12.50 (bs, 1H, COOH).

### <Example 2> Preparation of (2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (UDCA-Ile)

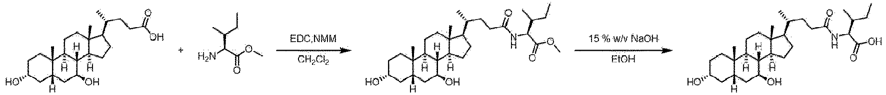

Starting materials including ursodeoxycholic acid (100 mg, 0.255 mmol) and L-isoleucine methyl ester hydrochloride (51 mg, 0.281 mmol), and N-methylmorpholine (NMM) (0.459 mmol) were dissolved in 5 mL of dry DCM. Thereafter, 1-(3-dimethylaminopropyl)-3-ethylcarbodiimide (EDC) (0.281 mmol) was added, and an intermediate was obtained in the same manner as the compound in Example 1 (121 mg, 91%). In order to remove the protected part, the intermediate (30 mg, 0.058 mmol) was dissolved in 2 mL of ethanol, 2 mL of 15% w/v sodium hydroxide was added, and a reaction was carried out for 30 minutes. Thereafter, deprotection was performed in the same manner as the compound in Example 1 to obtain a final compound (Example 2; UDCA-Ile) (19 mg, 66%).

(2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid; ¹H-NMR (400 MHz, DMSO-d6) δ = 0.61 (s, 3H, CH3), 0.79-0.94 (m, 13H), 1.08-1.25 (m, 9H), 1.28-1.41 (m, 7H), 1.42-1.51 (m, 3H), 1.60-1.86 (m, 6H), 1.88-1.98 (m, 1H), 1.98-2.11 (m, 1H), 2.14-2.26 (m, 1H), 3.87 (d, J = 6.6 Hz ,1H, OH), 4.15 (t, J = 7.3 Hz, 1H, CHCHCH3CH2), 4.44 (s, 1H, OH), 7.91 (d, J = 8.4 Hz, 1H, NH). 12.45 (bs, 1H, COOH).

### <Example 3> Preparation of (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (UDCA-Val)

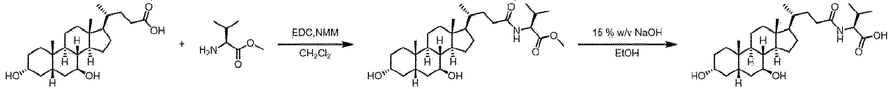

Starting materials including ursodeoxycholic acid (100 mg, 0.255 mmol) and L-valine methyl ester hydrochloride (50 mg, 0.281 mmol), and N-methylmorpholine (NMM) (0.459 mmol) were dissolved in 5 mL of dry DCM. Thereafter, 1-(3-dimethylaminopropyl)-3-ethylcarbodiimide (EDC) (0.281 mmol) was added, and an intermediate was obtained in the same manner as the compound in Example 1 (110 mg, 86%). In order to remove the protected part, the intermediate (30 mg, 0.058 mmol) was dissolved in 2 mL of ethanol, 2 mL of 15% w/v sodium hydroxide was added, and a reaction was carried out for 30 minutes. Thereafter, deprotection was performed in the same manner as the compound in Example 1 to obtain a final compound (Example 3; UDCA-Val) (28 mg, 98%).

(S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid; ¹H-NMR (400 MHz, DMSO-d6) δ = 0.61 (s, 3H, CH3), 0.82-0.93 (m, 13H), 1.05-1.25 (m, 8H), 1.26-1.41 (m, 7H), 1.42-1.52 (m, 3H), 1.60-1.70 (m, 3H), 1.70-1.89 (m, 2H), 1.89-1.98 (m, 1H), 1.98-2.11 (m, 3H), 2.15-2.27 (m, 1H), 3.87 (d, J = 7.0 Hz, 1H, OH), 4.11 (t, J = 7.0 Hz, 1H, CHCHCH3CH2), 4.45 (s, 1H, OH), 7.89 (d, J = 9.9 Hz, 1H, NH). 12.40 (bs, 1H, COOH).

### <Example 4> Preparation of (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (UDCA-Trp)

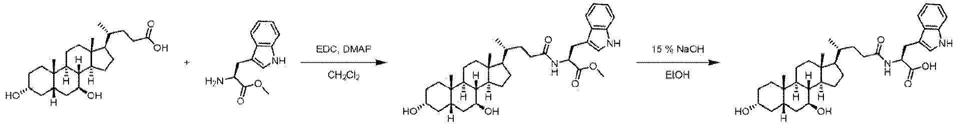

Starting materials including ursodeoxycholic acid (50 mg, 0.127 mmol) and L-tryptophan methyl ester hydrochloride (39 mg, 0.152 mmol), and 4-dimethylaminopyridine (1.5 mg, 0.013 mmol) were dissolved in 3 mL of dry DCM. Thereafter, 1-(3-dimethylaminopropyl)-3-ethylcarbodiimide (EDC) (0.153 mmol) was added, and an intermediate was obtained in the same manner as the compound in Example 1 (59mg, 83%). In order to remove the protected part, the intermediate (50 mg, 0.084 mmol) was dissolved in 9 mL of ethanol, 6 mL of 15% w/v sodium hydroxide was added, and a reaction was carried out for 30 minutes. Thereafter, deprotection was performed in the same manner as the compound in Example 1 to obtain a final compound (Example 4; UDCA-Trp) (24 mg, 40%).

¹H-NMR (500 MHz, DMSO-d6)δ= 0.59 (s, 3H), 0.83-0.87 (m, 7H), 0.87-0.99 (m, 2H), 1.00-1.50 (m, 15H), 1.53-1.74 (m, 4H), 1.77-1.86 (m, 1H), 1.88-2.01 (m, 2H), 2.03-2.11 (m, 1H), 2.94-3.00 (m, 1H), 3.11-3.17 (m, 1H), 3.23-3.35 (m, 2H), 3.88 (d, J = 5.6 Hz, 1H), 4.39-4.44 (m, 1H), 4.45 (d, J = 3.6 Hz, 1H), 6.94-6.98 (m, 1H), 7.03-7.07 (m, 1H), 7.09-7.11 (m, 1H), 7.32 (d. J = 6.4 Hz, 1H), 7.51 (d, J = 6.4 Hz, 1H), 7.98-8.02 (m, 1H), 10.83 (s, 1H), 12.58 (bs, 1H).

### <Example 5> Preparation of (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (UDCA-Tyr)

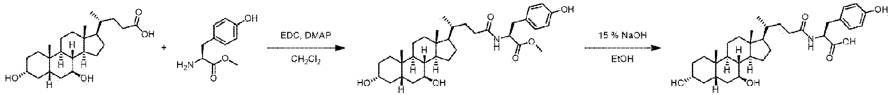

Starting materials including ursodeoxycholic acid (100 mg, 0.255 mmol) and L-tyrosine methyl ester hydrochloride (71 mg, 0.306 mmol) were dissolved in 3 mL of dry DCM. Thereafter, 1-(3-dimethylaminopropyl)-3-ethylcarbodiimide (EDC) (0.153 mmol) was added, and an intermediate was obtained in the same manner as the compound in Example 1 (118 mg, 81%). In order to remove the protected part, the intermediate (50 mg, 0.084 mmol) was dissolved in 9 mL of ethanol, 6 mL of 15% w/v sodium hydroxide was added, and a reaction was carried out for 30 minutes. Thereafter, deprotection was performed in the same manner as the compound in Example 1 to obtain a final compound (Example 5; UDCA-Tyr) (27 mg, 57%).

¹H-NMR (500 MHz, DMSO-d6)δ = 0.60 (s, 3H), 0.82-0.88 (m, 6H), 0.89-1.01 (m, 2H), 1.02-1.24 (m, 6H), 1.25-1.50 (m, 10H), 1.53-1.60 (m, 1H), 1.61-1.76 (m, 3H), 1.79-1.87 (m, 1H), 1.89-1.98 (m, 2H), 2.02-2.09 (m, 1H), 2.67-2.74 (m, 1H), 2.86-2.92 (m, 1H), 3.24 (bs, 2H), 3.88 (d, J = 5.6 Hz, 1H), 4.25-4.31 (m, 1H), 4.45 (d, J = 3.6 Hz, 1H), 6.63 (d, J = 6.8 Hz, 2H), 6.99 (d, J = 6.8 Hz, 2H), 8.02 (d, J = 6.4 Hz, 1H), 9.20 (s, 1H), 12.55 (bs, 1H).

### <Example 6> Preparation of (2S,3R)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)pentanoic acid (CA-Ile)

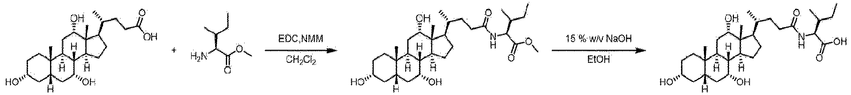

Starting materials including cholic acid (300 mg, 0.742 mmol) and L-isoleucine methyl ester hydrochloride (162 mg, 0.890 mmol), and N-methylmorpholine (NMM) (1.113 mmol) were dissolved in 4 mL of dry DCM. Thereafter, 1-(3-dimethylaminopropyl)-3-ethylcarbodiimide (EDC) (0.965 mmol) was added, and an intermediate was obtained in the same manner as the compound in Example 1 (349 mg, 90%). In order to remove the protected part, the intermediate (30 mg, 0.056 mmol) was dissolved in 2.7 mL of ethanol, 1.8 mL of 15% w/v sodium hydroxide was added, and a reaction was carried out for 30 minutes. Thereafter, deprotection was performed in the same manner as the compound in Example 1 to obtain a final compound (Example 6; CA-Ile) (24 mg, 79%).

(2S,3R)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)pentanoic acid; ¹H-NMR (400 MHz, DMSO-d6) δ = 0.58 (s, 3H, CH3), 0.76-0.88 (m, 10H), 0.89-0.96 (m, 3H), 1.08-1.48 (m, 14H), 1.56-1.68 (m, 3H), 1.68-1.82 (m, 4H), 1.92-2.07 (m, 2H), 2.08-2.27 (m, 3H), 3.12-3.24 (m, 1H, CH), 3.60 (s,1H, CH), 3.78 (s, 1H, CH), 4.15 (t, J = 6.6 Hz, 1H, CHCHCH3CH2), 7.91 (d, J = 8.2 Hz, 1H, NH). 12.34 (bs, 1H, COOH).

### <Example 7> Preparation of (S)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)butanoic acid (CA-Val)

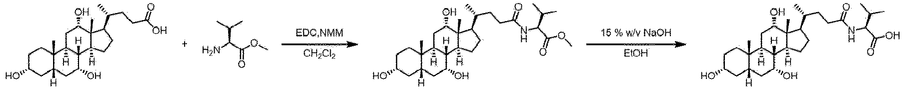

Starting materials including cholic acid (300 mg, 0.742 mmol) and L-valine methyl ester hydrochloride (149 mg, 0.890 mmol), and N-methylmorpholine (NMM) (1.113 mmol) were dissolved in 4 mL of dry DCM. Thereafter, 1-(3-dimethylaminopropyl)-3-ethylcarbodiimide (EDC) (0.965 mmol) was added, and an intermediate was obtained in the same manner as the compound in Example 1 (333 mg, 88%). In order to remove the protected part, the intermediate (50 mg, 0.096 mmol) was dissolved in 4.5 mL of ethanol, 3 mL of 15% w/v sodium hydroxide was added, and a reaction was carried out for 30 minutes. Thereafter, deprotection was performed in the same manner as the compound in Example 1 to obtain a final compound (Example 7; CA-Val) (31 mg, 64%).

(S)-3-methyl-2-((R)-4-((3R,5S,7R,8R,9S,10S,12S,13R,14S,17R)-3,7,12-trihydroxy-10,13-dimethyl-hexadecahydro-1H-cyclopenta[a]phenanthren-17-yl)pentanamido)butanoic acid; ¹H-NMR (400 MHz, DMSO-d6) δ = 0.58 (s, 3H, CH3), 0.73-0.90 (m, 10H), 0.90-1.01 (m, 3H), 1.06-1.51 (m, 11H), 1.53-1.69 (m, 3H), 1.69-1.84 (m, 3H), 1.90-2.08 (m, 3H), 2.08-2.30 (m, 3H), 3.10-3.17 (m, 1H, CH), 3.60 (s, 1H, CH), 3.78 (s, 1H, CH), 4.11 (t, J = 5.6 Hz, 1H, CHCHCH3CH2), 7.90 (d, J = 7.5 Hz, 1H, NH). 12.46 (bs, 1H, COOH).

### <Example 8> Preparation of (2S,3R)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylpentanoic acid (HDCA-Ile)

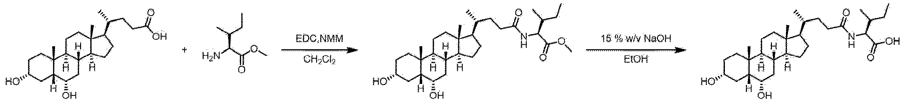

Starting materials including hyodeoxycholic acid (300 mg, 0.764 mmol) and L-isoleucine methyl ester hydrochloride (167 mg, 0.917 mmol), and N-methylmorpholine (NMM) (1.146 mmol) were dissolved in 4 mL of dry DCM. Thereafter, 1-(3-dimethylaminopropyl)-3-ethylcarbodiimide (EDC) (0.993 mmol) was added, and an intermediate was obtained in the same manner as the compound in Example 1 (300 mg, 76%). In order to remove the protected part, the intermediate (30 mg, 0.058 mmol) was dissolved in 2.7 mL of ethanol, 1.8 mL of 15% w/v sodium hydroxide was added, and a reaction was carried out for 30 minutes. Thereafter, deprotection was performed in the same manner as the compound in Example 1 to obtain a final compound (Example 8; HDCA-Ile) (15 mg, 52%).

(2S,3R)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylpentanoic acid; ¹H-NMR (400 MHz, DMSO-d6) δ = 0.59 (s, 3H, CH3), 0.78-0.91 (m, 12H), 0.92-1.25 (m, 13H), 1.29-1.54 (m, 9H), 1.59-1.69 (m, 2H), 1.69-1.81 (m, 2H), 1.87-1.95 (m, 1H), 1.97-2.07 (m, 1H), 2.15-2.24 (m, 1H), 3.77-3.85 (m, 1H, CH), 4.15 (t, J = 8.0 Hz, 1H, CHCHCH3CH2), 4.25 (s, 1H, OH), 4.44 (s, 1H, OH), 7.92 (d, J = 8.6 Hz, 1H, NH). 12.32 (bs, 1H, COOH).

### <Example 9> Preparation of (S)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylbutanoic acid (HDCA-Val)

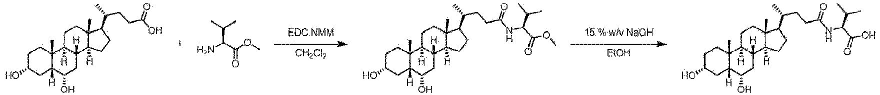

Starting materials including hyodeoxycholic acid (300 mg, 0.764 mmol) and L-valine methyl ester hydrochloride (154 mg, 0.917 mmol), and N-methylmorpholine (NMM) (1.146 mmol) were dissolved in 4 mL of dry DCM. Thereafter, 1-(3-dimethylaminopropyl)-3-ethylcarbodiimide (EDC) (0.993 mmol) was added, and an intermediate was obtained in the same manner as the compound in Example 1 (355 mg, 92%). In order to remove the protected part, the intermediate (40 mg, 0.079 mmol) was dissolved in 3.7 mL of ethanol, 2.5 mL of 15% w/v sodium hydroxide was added, and a reaction was carried out for 30 minutes. Thereafter, deprotection was performed in the same manner as the compound in Example 1 to obtain a final compound (Example 9; HDCA-Val) (30 mg, 77%).

(S)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylbutanoic acid; ¹H-NMR (400 MHz, DMSO-d6) δ = 0.59 (s, 3H, CH3), 0.80-0.92 (m, 12H), 0.91-1.25 (m, 11H), 1.26-1.56 (m, 8H), 1.60-1.70 (m, 2H), 1.71-1.86 (m, 2H), 1.88-1.95 (m, 1H), 1.97-2.08 (m, 2H), 2.16-2.26 (m, 1H), 3.77-3.86 (m, 1H, CH), 4.11 (t, J = 7.1 Hz, 1H, CHCHCH3CH2), 4.24 (s, 1H, OH), 4.42 (s, 1H, OH), 7.91 (d, J = 7.2 Hz, 1H, NH). 12.48 (bs, 1H, COOH).

### <Example 10> Preparation of (2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid (DCA-Ile)

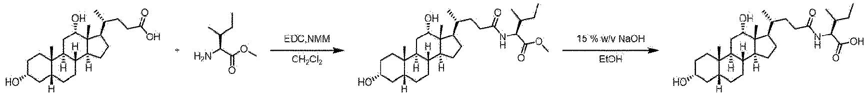

Starting materials including deoxycholic acid (200 mg, 0.509 mmol) and L-isoleucine methyl ester hydrochloride (110 mg, 0.611 mmol), and N-methylmorpholine (NMM) (0.764 mmol) were dissolved in 4 mL of dry DCM. Thereafter, 1-(3-dimethylaminopropyl)-3-ethylcarbodiimide (EDC) (0.764 mmol) was added, and an intermediate was obtained in the same manner as the compound in Example 1 (208 mg, 79%). In order to remove the protected part, the intermediate (50 mg, 0.096 mmol) was dissolved in 2.7 mL of ethanol, 1.8 mL of 15% w/v sodium hydroxide was added, and a reaction was carried out for 30 minutes. Thereafter, deprotection was performed in the same manner as the compound in Example 1 to obtain a final compound (Example 10; DCA-Ile) (44mg, 90%).

(2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid; ¹H-NMR (400 MHz, DMSO-d6) δ = 0.59 (s, 3H, CH3), 0.78-0.88 (m, 10H), 0.88-0.95 (m, 3H), 0.96-1.22 (m, 6H), 1.21-1.41 (m, 9H), 1.41-1.56 (m, 3H), 1.56-1.68 (m, 3H), 1.69-1.86 (m, 5H), 1.96-2.09 (m, 1H), 2.13-2.25 (m, 1H), 3.75-3.81 (m, 1H, CH), 4.13 (s, 1H, OH), 4.18 (t, J = 4.2 Hz, 1H, CHCHCH3CH2), 4.46 (s, 1H, OH), 7.91 (d, J = 8.0 Hz, 1H, NH). 12.46 (bs, 1H, COOH).

### <Example 11> Preparation of (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylbutanoic acid (DCA-Val)

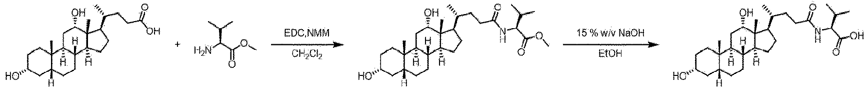

Starting materials including deoxycholic acid (200 mg, 0.509 mmol) and L-valine methyl ester hydrochloride (102 mg, 0.611 mmol), and N-methylmorpholine (NMM) (0.764 mmol) were dissolved in 4 mL of dry DCM. Thereafter, 1-(3-dimethylaminopropyl)-3-ethylcarbodiimide (EDC) (0.764 mmol) was added, and an intermediate was obtained in the same manner as the compound in Example 1 (204 mg, 79%). In order to remove the protected part, the intermediate (50 mg, 0.099 mmol) was dissolved in 4.6 mL of ethanol, 3.1 mL of 15% w/v sodium hydroxide was added, and a reaction was carried out for 30 minutes. Thereafter, deprotection was performed in the same manner as the compound in Example 1 to obtain a final compound (Example 11; DCA-Val) (40 mg, 82%).

(S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylbutanoic acid; ¹H-NMR (400 MHz, DMSO-d6) δ = 0.59 (s, 3H, CH3), 0.80-0.89 (m, 10H), 0.89-0.96 (m, 3H), 0.97-1.21 (m, 5H), 1.23-1.40 (m, 8H), 1.40-1.56 (m, 3H), 1.56-1.67 (m, 3H), 1.69-1.85 (m, 4H), 1.96-2.06 (m, 2H), 2.15-2.27 (m, 1H), 3.74-3.82 (m, 1H, CH), 4.11 (t, J = 7.3 Hz, 1H, CHCHCH3CH2), 4.18 (s, 1H, OH), 4.47 (s, 1H, OH), 7.90 (d, J = 8.4 Hz, 1H, NH). 12.49 (bs, 1H, COOH).

### <Example 12> Preparation of (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(1H-indol-3-yl)propanoic acid (DCA-Trp)

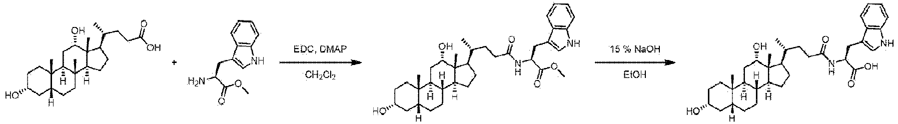

Starting materials including deoxycholic acid (50 mg, 0.127 mmol) and L-tryptophan methyl ester hydrochloride (39 mg, 0.152 mmol) were dissolved in 3 mL of dry DCM. Thereafter, 1-(3-dimethylaminopropyl)-3-ethylcarbodiimide (EDC) (0.153 mmol) was added, and an intermediate was obtained in the same manner as the compound in Example 1 (62 mg, 83%). In order to remove the protected part, the intermediate (50 mg, 0.084mmol) was dissolved in 9 mL of ethanol, 6 mL of 15% w/v sodium hydroxide was added, and a reaction was carried out for 30 minutes. Thereafter, deprotection was performed in the same manner as the compound in Example 1 to obtain a final compound (Example 12; DCA-Trp) (24 mg, 39%).

¹H-NMR (500 MHz, DMSO-d6)δ = 0.57 (s, 3H), 0.79-0.90 (m, 7H), 0.90-1.49 (m, 16H), 1.53-1.83(m, 6H), 1.86-1.92 (m, 1H), 1.93-2.02 (m, 1H), 2.04-2.12 (m, 1H), 2.94-3.00 (m, 1H), 3.11-3.21 (m, 2H), 3.59-3.64 (m, 1H), 4.14 (d, J = 2.8 Hz, 1H), 4.33 (d, J = 3.6 Hz, 1H), 4.45-4.39 (m, 1H), 6.94-6.98 (m, 1H), 7.03-7.07 (m, 1H), 7.10-7.12 (m, 1H), 7.32 (d, J = 6.4 Hz, 1H), 7.51 (d, , J = 6.4 Hz, 1H), 8.05 (d, J = 6 Hz, 1H), 10.84 (s, 1H), 12.59 (bs, 1H).

### <Example 13> Preparation of (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(4-hydroxyphenyl)propanoic acid (DCA-Tyr)

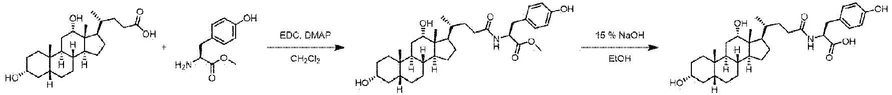

Starting materials including deoxycholic acid (100 mg, 0.255 mmol) and L-tyrosine methyl ester hydrochloride (71 mg, 0.306 mmol) were dissolved in 3 mL of dry DCM. Thereafter, 1-(3-dimethylaminopropyl)-3-ethylcarbodiimide (EDC) (0.153 mmol) was added, and an intermediate was obtained in the same manner as the compound in Example 1 (110 mg, 76%). In order to remove the protected part, the intermediate (50 mg, 0.088 mmol) was dissolved in 9 mL of ethanol, 6 mL of 15% w/v sodium hydroxide was added, and a reaction was carried out for 30 minutes. Thereafter, deprotection was performed in the same manner as the compound in Example 1 to obtain a final compound (Example 13; DCA-Tyr) (24 mg, 49%).

¹H-NMR (500 MHz, DMSO-d6)δ = 0.57 (s, 3H), 0.84 (s, 3H), 0.85-0.90 (m, 4H), 0.91-1.13 (m, 4H), 1.13-1.38 (m, 9H), 1.41-1.65 (m, 6H), 1.65-1.83 (m 4H), 1.90-1.98 (m, 1H), 2.02-2.10 (m, 1H), 2.67-2.73 (m, 1H), 2.86-2.92 (m, 1H), 3.77 (bs, 1H), 4.19 (d, J = 3.2 Hz, 1H), 4.24-4.30 (m, 1H), 4.47 (d, J = 3.6 Hz, 1H), 6.63 (d, J = 6.8 Hz, 2H), 6.99 (d, J = 6.8 Hz, 2H), 8.02 (d, J = 6.4 Hz, 1H), 9.19 (s, 1H), 12.56 (bs. 1H).

### <Example 14> Preparation of (2S,3R)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (CDCA-Ile)

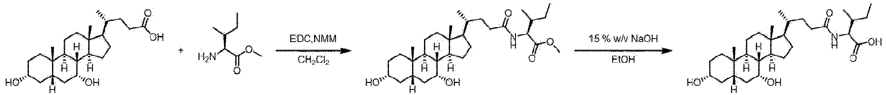

Starting materials including chenodeoxycholic acid (200 mg, 0.509 mmol) and L-isoleucine methyl ester hydrochloride (110 mg, 0.611 mmol), and N-methylmorpholine (NMM) (0.916 mmol) were dissolved in 5 mL of dry DCM. Thereafter, 1-(3-dimethylaminopropyl)-3-ethylcarbodiimide (EDC) (0.764 mmol) was added, and an intermediate was obtained in the same manner as the compound in Example 1 (206mg, 78%). In order to remove the protected part, the intermediate (50 mg, 0.096 mmol) was dissolved in 4.5 mL of ethanol, 3 mL of 15% w/v sodium hydroxide was added, and a reaction was carried out for 30 minutes. Thereafter, deprotection was performed in the same manner as the compound in Example 1 to obtain a final compound (Example 14; CDCA-Ile) (44 mg, 90%).

(2S,3R)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid; ¹H-NMR (400 MHz, DMSO-d6) δ = 0.55 (s, 3H, CH3), 0.64-0.92 (m, 13H), 1.04-1.28 (m, 8H), 1.29-1.50 (m, 7H), 1.59-1.85 (m, 6H), 1.87-1.94 (m, 1H), 1.96-2.10 (m, 2H), 2.14-2.26 (m, 2H), 3.13-3.23 (m, 1H, CH), 3.59-3.65 (m, 1H, CH), 4.10 (t, J = 4.2 Hz, 1H, CHCHCH3CH2), 4.12 (s, 1H, OH), 4.31 (s, 1H, OH), 7.90 (d, J = 9.1 Hz, 1H, NH). 12.47 (bs, 1H, COOH).

### <Example 15> Preparation of (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (CDCA-Val)

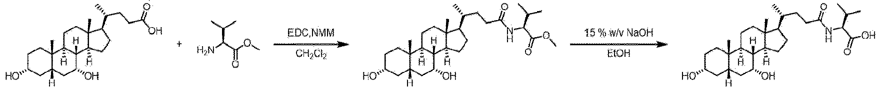

Starting materials including chenodeoxycholic acid (200 mg, 0.509 mmol) and L-valine methyl ester hydrochloride (102 mg, 0.611 mmol), and N-methylmorpholine (NMM) (0.916 mmol) were dissolved in 4 mL of dry DCM. Thereafter, 1-(3-dimethylaminopropyl)-3-ethylcarbodiimide (EDC) (0.764 mmol) was added, and an intermediate was obtained in the same manner as the compound in Example 1 (199 mg, 77%). In order to remove the protected part, the intermediate (50 mg, 0.099 mmol) was dissolved in 4.6 mL of ethanol, 3.1 mL of 15% w/v sodium hydroxide was added, and a reaction was carried out for 30 minutes. Thereafter, deprotection was performed in the same manner as the compound in Example 1 to obtain a final compound (Example 15; CDCA-Val) (45 mg, 87%).

(S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid; ¹H-NMR (400 MHz, DMSO-d6) δ = 0.59 (s, 3H, CH3), 0.77-0.92 (m, 13H), 1.03-1.27 (m, 9H), 1.28-1.51 (m, 8H), 1.59-1.84 (m, 7H), 1.86-1.95 (m, 1H), 1.99-1.21 (m, 1H), 2.12-2.26 (m, 2H), 3.11-3.23 (m, 1H, CH), 3.58-3.65 (m, 1H, CH), 4.11 (s, 1H, OH), 4.14 (t, J = 7.2 Hz, 1H, CHCHCH3CH2), 4.31 (s, 1H, OH), 7.91 (d, J = 8.8 Hz, 1H, NH). 12.46 (bs, 1H, COOH).

### <Example 16> Preparation of (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (CDCA-Trp)

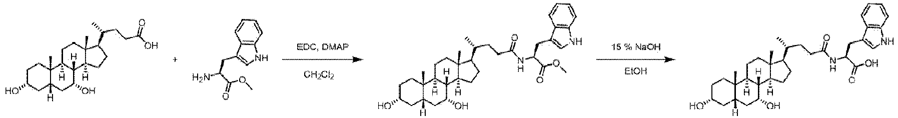

Starting materials including chenodeoxycholic acid (50 mg, 0.127 mmol) and L-tryptophan methyl ester hydrochloride (39 mg, 0.152 mmol) were dissolved in 3 mL of dry DCM. Thereafter, 1-(3-dimethylaminopropyl)-3-ethylcarbodiimide (EDC) (0.153 mmol) was added, and an intermediate was obtained in the same manner as the compound in Example 1 (50 mg, 66%). In order to remove the protected part, the intermediate (50 mg, 0.084mmol) was dissolved in 9 mL of ethanol, 6 mL of 15% w/v sodium hydroxide was added, and a reaction was carried out for 30 minutes. Thereafter, deprotection was performed in the same manner as the compound in Example 1 to obtain a final compound (Example 16; CDCA-Trp) (26 mg, 53%).

¹H-NMR (500 MHz, DMSO-d6)δ = 0.56 (s, 3H), 0.81-0.90 (m, 8H), 0.91-1.36 (m, 14H), 1.41-1.64 (m, 4H), 1.65-1.82 (m, 2H), 1.92-2.00 (m, 1H), 2.04-2.11 (m, 1H), 2.94-3.00 (m, 1H), 3.11-3.16 (m, 1H), 3.34-3.39 (m, 1H), 3.77 (bs, 1H), 4.19 (bs, 1H), 4.37-4.44 (m, 1H), 4.47 (d, J = 3.6 Hz, 1H), 6.94-6.98 (m, 1H), 7.03-7.07 (m, 1H), 7.10-7.12(m, 1H), 7.31 (d, J = 6.4 Hz, 2H), 7.51 (d, J = 6.4 Hz, 2H), 8.01-8.06 (m, 1H), 10.83 (s, 1H), 12.59 (bs, 1H).

### <Example 17> Preparation of (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (CDCA-Tyr)

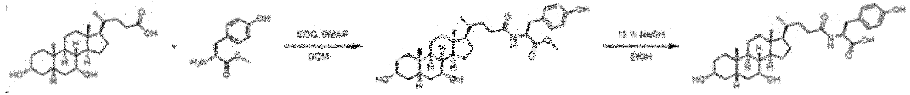

Starting materials including chenodeoxycholic acid (100 mg, 0.255 mmol) and L-tyrosine methyl ester hydrochloride (71 mg, 0.306 mmol) were dissolved in 3 mL of dry DCM. Thereafter, 1-(3-dimethylaminopropyl)-3-ethylcarbodiimide (EDC) (0.153 mmol) was added, and an intermediate was obtained in the same manner as the compound in Example 1 (123 mg, 85%). In order to remove the protected part, the intermediate (50 mg, 0.088 mmol) was dissolved in 9 mL of ethanol, 6 mL of 15% w/v sodium hydroxide was added, and a reaction was carried out for 30 minutes. Thereafter, deprotection was performed in the same manner as the compound in Example 1 to obtain a final compound (Example 17; CDCA-Tyr) (27 mg, 57%).

¹H-NMR (500 MHz, DMSO-d6)δ = 0.58 (s, 3H), 0.80-0.91 (m, 6H), 0.91-1.49 (m, 16H), 1.52-1.83 (m, 6H), 1.85-2.00 (m, 2H), 2.02-2.10 (m, 1H), 2.14-2.23 (m, 1H), 2.67-2.73 (m, 1H), 2.86-2.92 (m, 1H), 3.13-3.22 (m, 1H), 3.62 (s, 1H), 4.12 (d, J = 2.4 Hz, 1H), 4.24-4.30 (m, 1H), 4.32 (d, J = 4 Hz, 1H), 6.62 (d, J = 6.8 Hz, 2H), 6.99 (d, J = 6.8 Hz, 2H), 8.01 (d, J = 6.4 Hz, 1H), 9.19 (s, 1H), 12.58 (bs, 1H).

The chemical structures of Examples 1 to 17 are shown in Table 1 below.

**TABLE 1**

| Example | Chemical structure | Example | Chemical structure |
|---|---|---|---|
| 1 (LCA-Ile) | 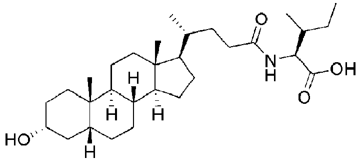 | 10 (DCA-Ile) | 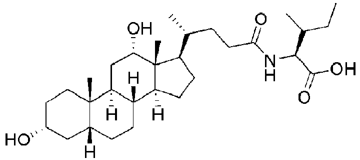 |
| 2 (UDCA-Ile) | 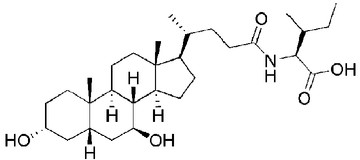 | 11 (DCA-Val) | 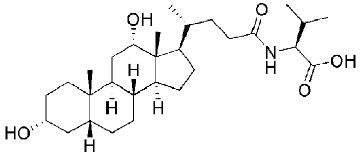 |
| 3 (UDCA-Val) | 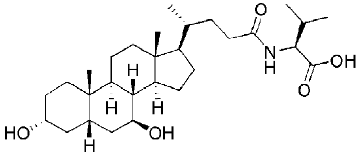 | 12 (DCA-Trp) | 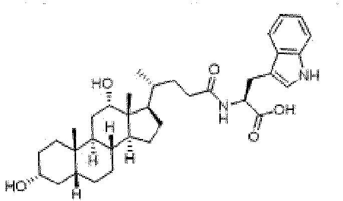 |
| 4 (UDCA-Trp) | 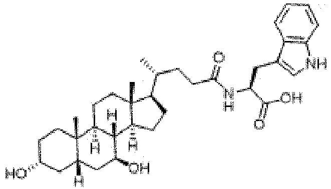 | 13 (DCA-Tyr) | 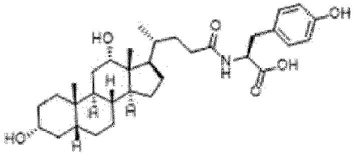 |
| 5 (UDCA-Tyr) | 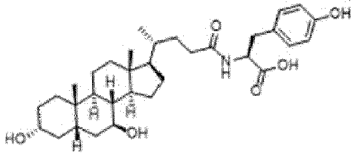 | 14 (CDCA-Ile) | 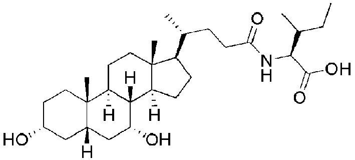 |
| 6 (CA-Ile) | 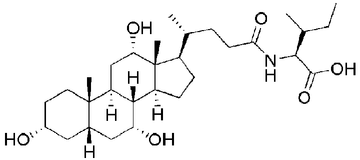 | 15 (CDCA-Val) | 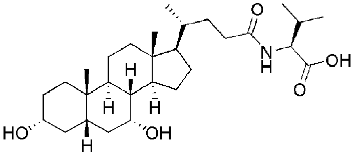 |
| 7 (CA-Val) | 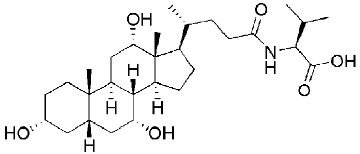 | 16 (CDCA-Trp) | 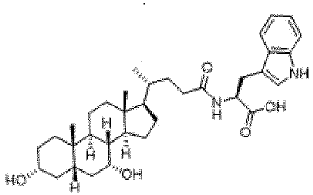 |
| 8 (HDCA-Ile) | 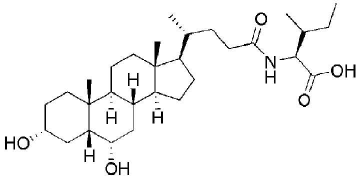 | 17 (CDCA-Tyr) | 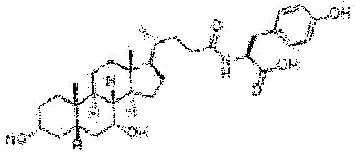 |
| 9 (HDCA-Val) | 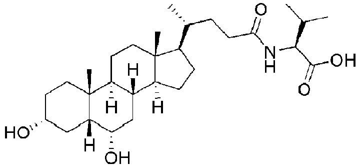 | | |

### <Example 18> Preparation of 2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)acetic acid (LCA-Val-Gly)

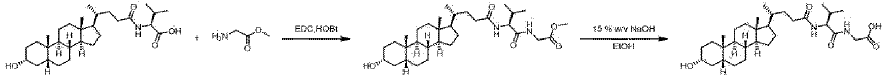

Starting materials including lithocholic acid-Val (100 mg, 0.210 mmol) and L-glycine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (110 mg, 95%). In order to remove the protected part, the intermediate (50 mg, 0.091 mmol) was dissolved in 5 mL of ethanol, 3 mL of 15% w/v sodium hydroxide was added, and a reaction was carried out for 30 minutes. Thereafter, deprotection was performed in the same manner as the compound in Example 1 to obtain a final compound (Example 18; LCA-Val-Gly) (38 mg, 79%).

2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)acetic acid; ¹H-NMR (400 MHz, DMSO-d6) δ = 0.60 (s, 3H, CH3), 0.79-0.90 (m, 13H), 0.97-1.08 (m, 4H), 1.12-1.24 (m, 6H), 1.27-1.39 (m, 7H), 1.46-1.55 (m, 2H), 1.57-1.70 (m, 3H), 1.73-1.84 (m, 2H), 1.90-2.06 (m, 3H), 2.16-2.24 (m, 1H), 3.67 (d, J = 16.24 Hz, 1H, CH), 4.16 (t, J = 7.62 Hz, 2H, CH2), 4.45 (s, 1H, OH), 7.82 (d, J = 8.44 Hz, 1H, NH), 8.12 (s, 1H, NH).

### <Example 19> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ala)

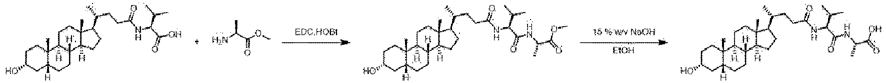

Starting materials including lithocholic acid-Val (50 mg, 0.105 mmol) and L-alanine methyl ester hydrochloride (0.126 mmol), and 1-hydroxybenzotriazole hydrate (0.126 mmol) were dissolved in 3 mL of dry DCM. Thereafter, 1-(3-dimethylaminopropyl)-3-ethylcarbodiimide (EDC) (0.126 mmol) was added, and an intermediate was obtained in the same manner as the compound in Example 1 (43mg, 81%). In order to remove the protected part, the intermediate (33 mg, 0.058 mmol) was dissolved in 3 mL of ethanol, 2 mL of 15% w/v sodium hydroxide was added, and a reaction was carried out for 30 minutes. Thereafter, deprotection was performed in the same manner as the compound in Example 1 to obtain a final compound (Example 19; LCA-Val-Ala) (26.5 mg, 83%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid; ¹H-NMR (400 MHz, DMSO-d6) δ = 0.59 (s, 3H, CH3), 0.79-0.91 (m, 13H), 0.97-1.21 (m, 10H), 1.24-1.38 (m, 10H), 1.45-1.56 (m, 2H), 1.58-1.70 (m, 3H), 1.73-1.84 (m, 2H), 1.86-1.96 (m, 2H), 1.97-2.06 (m, 1H), 2.14-2.24 (m, 1H), 4.10-4.23 (m, 2H, CHCH3, CH), 7.80 (d, J = 8.88 Hz, 1H, NH), 8.23 (d, J = 6.04 Hz, 1H, NH), 12.47 (bs, 1H, COOH).

### <Example 20> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylbutanoic acid (LCA-Val-Val)

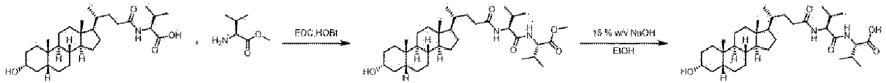

Starting materials including lithocholic acid-Val (50 mg, 0.105 mmol) and L-valine methyl ester hydrochloride (0.126 mmol), and 1-hydroxybenzotriazole hydrate (0.126 mmol) were dissolved in 3 mL of dry DCM. Thereafter, 1-(3-dimethylaminopropyl)-3-ethylcarbodiimide (EDC) (0.126 mmol) was added, and an intermediate was obtained in the same manner as the compound in Example 1 (59 mg, 95%). In order to remove the protected part, the intermediate (50 mg, 0.085 mmol) was dissolved in 2 mL of ethanol, 2 mL of 15% w/v sodium hydroxide was added, and a reaction was carried out for 30 minutes. Thereafter, deprotection was performed in the same manner as the compound in Example 1 to obtain a final compound (Example 2; LCA-Val-Val) (40 mg, 82%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylbutanoic acid; ¹H-NMR (400 MHz, DMSO-d6) δ = 0.59 (s, 3H, CH3), 0.79-0.90 (m, 19H), 0.97-1.23 (m, 10H), 1.27-1.38 (m, 7H), 1.45-1.54 (m, 2H), 1.58-1.70 (m, 3H), 1.73-1.83 (m, 2H), 1.88-1.96 (m, 2H), 1.99-2.08 (m, 2H), 2.14-2.23 (m, 1H), 4.08 (dd, J = 7.72, 5.80 Hz,1H, CH), 4.24 (t, J = 8.20 Hz, 1H, CH), 4.45 (s, 1H, OH), 7.81 (d, J = 8.80 Hz, 1H, NH), 7.90 (d, J = 8.00 Hz, 1H, NH), 12.56 (bs, 1H, COOH).

### <Example 21> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-methylpentanoic acid (LCA-Val-Leu)

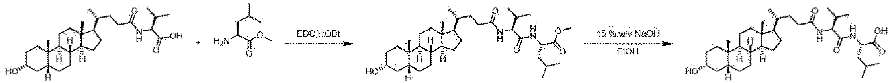

Starting materials including lithocholic acid-Val (100 mg, 0.210 mmol) and L-leucine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (109 mg, 82%). In order to remove the protected part, the intermediate (50 mg, 0.081 mmol) was dissolved in 3 mL of ethanol, 2 mL of 15% w/v sodium hydroxide was added, and a reaction was carried out for 30 minutes. Thereafter, deprotection was performed in the same manner as the compound in Example 1 to obtain a final compound (Example 21; LCA-Val-Leu) (40 mg, 85%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-methylpentanoic acid; ¹H-NMR (400 MHz, DMSO-d6) δ = 0.60 (s, 3H, CH3), 0.79-0.91 (m, 19H), 0.96-1.23 (m, 10H), 1.27-1.39 (m, 7H), 1.45-1.54 (m, 4H), 1.58-1.70 (m, 4H), 1.73-1.83 (m, 2H), 1.87-1.96 (m, 2H), 1.99-2.09 (m, 1H), 2.12-2.22 (m, 1H), 4.16 (q, J = 7.33 Hz, 2H, CH, CHCH2), 4.45 (bs, 1H, OH), 7.79 (d, J = 8.84 Hz, 1H, NH), 8.07 (d, J = 7.04 Hz, 1H, NH), 12.52 (bs, 1H, COOH).

### <Example 22> Preparation of (2S,3R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylpentanoic acid (LCA-Val-Ile)

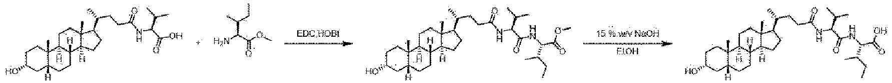

Starting materials including lithocholic acid-Val (100 mg, 0.210 mmol) and L-isoleucine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (121 mg, 82%). In order to remove the protected part, the intermediate (50 mg, 0.083 mmol) was dissolved in 3 mL of ethanol, 2 mL of 15% w/v sodium hydroxide was added, and a reaction was carried out for 30 minutes. Thereafter, deprotection was performed in the same manner as the compound in Example 1 to obtain a final compound (Example 22; LCA-Val-Ile) (37 mg, 75%).

(2S,3R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylpentanoic acid; ¹H-NMR (400 MHz, DMSO-d6) δ = 0.60 (s, 3H, CH3), 0.80-0.89 (m, 19H), 0.97-1.23 (m, 13H), 1.28-1.38 (m, 7H), 1.45-1.55 (m, 2H), 1.57-1.70 (m, 3H), 1.71-1.83 (m, 3H), 1.87-1.96 (m, 2H), 1.99-2.08 (m, 1H), 2.13-2.22 (m, 1H), 4.11 (t, J = 7.12 Hz, 1H, CH), 4.23 (t, J = 7.76 Hz, 1H, CH), 4.46 (d, J = 4.52 Hz, 1H, OH), 7.80 (d, J = 8.72 Hz, 1H, NH), 7.92 (d, J = 8.40 Hz, 1H, NH), 12.54 (bs, 1H, COOH).

### <Example 23> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-(methylthio)butanoic acid (LCA-Val-Met)

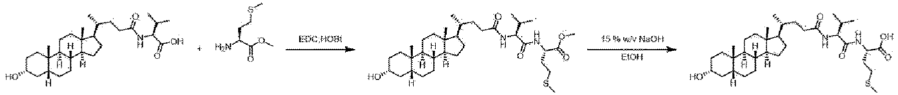

Starting materials including lithocholic acid-Val (100 mg, 0.210 mmol) and L-methionine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (100 mg, 79%). In order to remove the protected part, the intermediate (40 mg, 0.066 mmol) was dissolved in 2 mL of ethanol, 1 mL of 15% w/v sodium hydroxide was added, and a reaction was carried out for 30 minutes. Thereafter, deprotection was performed in the same manner as the compound in Example 1 to obtain a final compound (Example 23; LCA-Val-Met) (31 mg, 79%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-(methylthio)butanoic acid; ¹H-NMR (400 MHz, DMSO-d6) δ = 0.60 (s, 3H, CH3), 0.80-0.90 (m, 13H), 0.96-1.23 (m, 11H), 1.28-1.38 (m, 7H), 1.28-1.38 (m, 7H), 1.46-1.71 (m, 5H), 1.74-1.96 (m, 6H), 2.00-2.10 (m, 5H), 2.12-2.21 (m, 1H), 4.14 (t, J = 7.86 Hz, 1H, CH), 4.21-4.29 (m, 1H, CHCH2), 4.46 (d, J = 3.68 Hz, 1H, OH), 7.82 (t, J = 8.26 Hz, 1H, NH), 8.19 (dd, J = 27.68, 7.72 Hz, 1H, NH), 12.64 (bs, 1H, COOH).

### <Example 24> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-phenylpropanoic acid (LCA-Val-Phe)

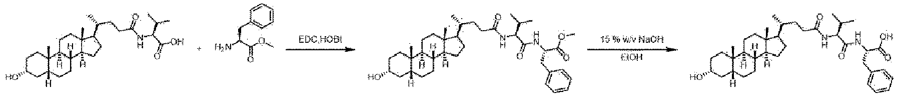

Starting materials including lithocholic acid-Val (100 mg, 0.210 mmol) and L-phenylalanine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (119 mg, 89%). In order to remove the protected part, the intermediate (50 mg, 0.076 mmol) was dissolved in 5 mL of ethanol, 3 mL of 15% w/v sodium hydroxide was added, and a reaction was carried out for 30 minutes. Thereafter, deprotection was performed in the same manner as the compound in Example 1 to obtain a final compound (Example 24; LCA-Val-Phe) (33.2 mg, 69%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-phenylpropanoic acid; ¹H-NMR (400 MHz, DMSO-d6) δ = 0.59 (s, 3H, CH3), 0.75-0.90 (m, 13H), 0.97-1.09 (m, 4H), 1.10-1.25 (m, 6H), 1.27-1.38 (m, 7H), 1.46-1.54 (m, 2H), 1.56-1.70 (m, 3H), 1.73-1.83 (m, 2H), 1.87-2.03 (m, 3H), 2.88 (q, J = 7.65 Hz, 1H, CH3), 3.02 (q, J = 6.37 Hz, 1H, CH3), 4.16 (t, J = 8.20 Hz, 1H, CH), 4.38 (q, J = 7.19 Hz, 1H, CHCH2), 4.46 (d, J = 4.40 Hz, 1H, OH), 7.16-7.27 (m, 5H, Ar), 7.73 (d, J = 9.28 Hz, 1H, NH), 8.18 (d, J = 8.60 Hz, 1H, NH), 12.68 (bs, 1H, COOH).

### <Example 25> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-indol-3-yl)propanoic acid (LCA-Val-Trp)

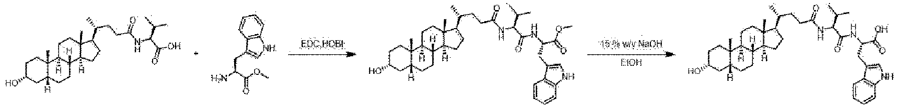

Starting materials including lithocholic acid-Val (100 mg, 0.210 mmol) and L-tryptophan methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (134 mg, 94%). In order to remove the protected part, the intermediate (50 mg, 0.073 mmol) was dissolved in 2 mL of ethanol, 1 mL of 15% w/v sodium hydroxide was added, and a reaction was carried out for 30 minutes. Thereafter, deprotection was performed in the same manner as the compound in Example 1 to obtain a final compound (Example 25; LCA-Val-Trp) (38.6 mg, 80%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-indol-3-yl)propanoic acid; ¹H-NMR (400 MHz, DMSO-d6) δ = 0.58 (s, 3H, CH3), 0.78-0.90 (m, 13H), 0.97-1.22 (m, 10H), 1.26-1.39 (m, 7H), 1.44-1.54 (m, 2H), 1.56-1.70 (m, 4H), 1.72-1.83 (m, 2H), 1.88-2.02 (m, 2H), 2.13-2.23 (m, 1H), 3.02 (q, J = 7.28 Hz, 1H, CH2), 3.14 (q, J = 7.13 Hz, 1H, CH2), 4.20 (t, J = 7.36 Hz, 1H, CH), 4.45 (m, 2H, OH, CH), 6.96 (t, J = 7.28 Hz, 1H, Ar), 7.05 (t, J = 7.08 Hz, 1H, Ar), 7.16 (s, 1H, Ar), 7.31 (d, J = 8.24 Hz, 1H, Ar), 7.51 (d, J = 8.24 Hz, 1H, Ar), 7.75 (d, J = 8.24 Hz, 1H, NH), 10.85 (s, 1H, NH), 12.55 (bs, 1H, COOH).

### <Example 26> Preparation of 1-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoyl)pyrrolidine-2-carboxylic acid (LCA-Val-Pro)

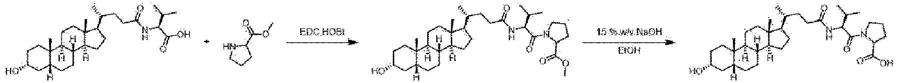

Starting materials including lithocholic acid-Val (70 mg, 0.147 mmol) and L-proline methyl ester hydrochloride (0.176 mmol), and 1-hydroxybenzotriazole hydrate (0.176 mmol) were dissolved in 2 mL of dry DCM. Thereafter, 1-(3-dimethylaminopropyl)-3-ethylcarbodiimide (EDC) (0.176 mmol) was added, and an intermediate was obtained in the same manner as the compound in Example 1 (66 mg, 76%). In order to remove the protected part, the intermediate (50 mg, 0.085 mmol) was dissolved in 2 mL of ethanol, 2 mL of 15% w/v sodium hydroxide was added, and a reaction was carried out for 30 minutes. Thereafter, deprotection was performed in the same manner as the compound in Example 1 to obtain a final compound (Example 26; LCA-Val-Pro) (25 mg, 52%).

1-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoyl)pyrrolidine-2-carboxylic acid; ¹H-NMR (400 MHz, DMSO-d6) δ = 0.59 (s, 3H, CH3), 0.80-0.93 (m, 13H), 0.97-1.22 (m, 10H), 1.27-1.38 (m, 7H), 1.46-1.71 (m, 5H), 1.73-2.02 (m, 7H), 2.07-2.20 (m, 2H), 3,31-3.40 (m, 1H), 1.46-1.71 (m, 5H), 3.56 (q, J = 7.29 Hz, 1H, CH2), 3.81 (q, J = 7.64 Hz, 1H, CH2), 4.20 (s, 1H, CH), 4.27 (t, J = 8.74 Hz, 1H, CH), 4.66 (bs, 1H, OH), 8.04 (d, J = 8.28 Hz, 1H, NH).

### <Example 27> Preparation of (S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ser)

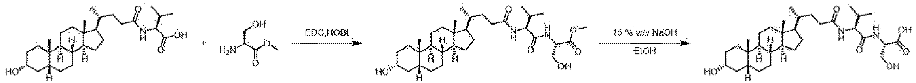

Starting materials including lithocholic acid-Val (100 mg, 0.210 mmol) and L-serine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (113 mg, 91%). In order to remove the protected part, the intermediate (40 mg, 0.069 mmol) was dissolved in 2 mL of ethanol, 2 mL of 15% w/v sodium hydroxide was added, and a reaction was carried out for 30 minutes. Thereafter, deprotection was performed in the same manner as the compound in Example 1 to obtain a final compound (Example 27; LCA-Val-Ser) (35.2 mg, 91%).

(S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid; ¹H-NMR (400 MHz, DMSO-d6) δ = 0.60 (s, 3H, CH3), 0.79-0.91 (m, 13H), 0.97-1.24 (m, 11H), 1.27-1.39 (m, 7H), 1.45-1.70 (m, 6H), 1.73-1.85 (m, 2H), 1.88-2.07 (m, 2H), 2,17-2.26 (m, 1H), 3.58-3.70 (m, 2H), 4.18-4.27 (m, 2H), 4.45 (d, J = 4.72 Hz, 1H, OH), 7.82 (d, J = 9.08 Hz, 1H, NH), 8.04 (d, J = 7.92 Hz, 1H, NH).

### <Example 28> Preparation of (2S,3S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)butanoic acid (LCA-Val-Thr)

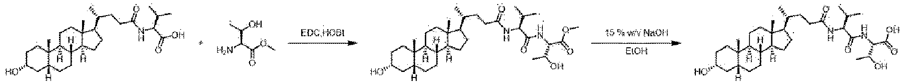

Starting materials including lithocholic acid-Val (100 mg, 0.210 mmol) and L-threonine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (115 mg, 93%). In order to remove the protected part, the intermediate (51.2 mg, 0.087 mmol) was dissolved in 3 mL of ethanol, 2 mL of 15% w/v sodium hydroxide was added, and a reaction was carried out for 30 minutes. Thereafter, deprotection was performed in the same manner as the compound in Example 1 to obtain a final compound (Example 28; LCA-Val-Thr) (50 mg, 99%).

(2S,3S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)butanoic acid; ¹H-NMR (400 MHz, DMSO-d6) δ = 0.59 (s, 3H, CH3), 0.80-0.91 (m, 13H), 0.97-1.23 (m, 13H), 1.27-1.39 (m, 7H), 1.45-1.71 (m, 5H), 1.73-1.85 (m, 2H), 1.89-2.09 (m, 3H), 2,14-2.24 (m, 1H), 4.08-4.21 (m, 2H), 4.25 (q, J = 6.61 Hz, 1H, CH), 4.45 (s, 1H, OH), 4.87 (s, 1H), 7.73 (t, J = 6.04 Hz, 1H, NH), 7.86 (t, J = 6.18 Hz, 1H, NH), 12.50 (bs, 1H, COOH).

### <Example 29> Preparation of (R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-mercaptopropanoic acid (LCA-Val-Cys)

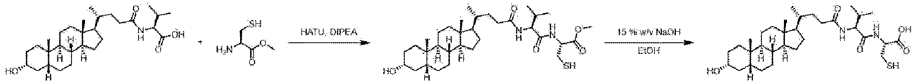

Starting materials including lithocholic acid-Val (50 mg, 0.105 mmol) and L-cysteine methyl ester hydrochloride (0.126 mmol), and HATU (0.126 mmol) and N,N-diisopropylethylamine (DIPEA) (0.126 mmol) were dissolved in 3 mL of dry DCM. Thereafter, an intermediate was obtained in the same manner as the compound in Example 1 (10 mg, 17%). In order to remove the protected part, the intermediate (10 mg, 0.017 mmol) was dissolved in 1 mL of ethanol, 1 mL of 15% w/v sodium hydroxide was added, and a reaction was carried out for 30 minutes. Thereafter, deprotection was performed in the same manner as the compound in Example 1 to obtain a final compound (Example 29; LCA-Val-Cys) (1.7 mg, 17%).

(R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-mercaptopropanoic acid; ¹H-NMR (400 MHz, DMSO-d6) δ = 0.60 (s, 3H, CH3), 0.81-0.91 (m, 13H), 0.98-1.24 (m, 10H), 1.27-1.39 (m, 7H), 1.45-1.55 (m, 2H), 1.58-1.71 (m, 3H), 1.72-1.85 (m, 2H), 1.88-2.08 (m, 3H), 2.16-2.26 (m, 1H), 2.70-2.89 (m, 2H), 4.17 (t, J = 7.44 Hz, 1H, CH), 4.32-4.38 (m, 1H, CH), 4.45 (d, J = 4.28 Hz, 1H, OH), 7.87 (d, J = 9.12 Hz, 1H, NH), 8.16 (d, J = 7.56 Hz, 1H, NH), 12.85 (bs, 1H, COOH).

### <Example 30> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(4-hydroxyphenyl)propanoic acid (LCA-Val-Tyr)

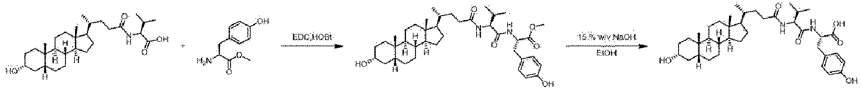

Starting materials including lithocholic acid-Val (100 mg, 0.210 mmol) and L-tyrosine methyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (100 mg, 73%). In order to remove the protected part, the intermediate (45 mg, 0.069 mmol) was dissolved in 3 mL of ethanol, 2 mL of 15% w/v sodium hydroxide was added, and a reaction was carried out for 30 minutes. Thereafter, deprotection was performed in the same manner as the compound in Example 1 to obtain a final compound (Example 30; LCA-Val-Tyr) (33 mg, 75%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(4-hydroxyphenyl)propanoic acid; ¹H-NMR (400 MHz, DMSO-d6) δ = 0.60 (s, 3H, CH3), 0.76-0.91 (m, 12H), 0.96-1.23 (m, 10H), 1.27-1.39 (m, 7H), 1.45-1.55 (m, 2H), 1.57-1.71 (m, 3H), 1.74-1.94 (m, 3H), 1.95-2.04 (m, 1H), 2.14-2.23 (m, 1H), 2.71-2.92 (m, 2H), 4.13-4.21 (m, 1H), 4.26-4.34 (m, 1H), 4.46 (s, 1H, OH), 6.62 (d, J = 6.64 Hz, 2H, Ar), 6.99 (d, J = 6.64 Hz, 2H, Ar), 7.72 (d, J = 9.00 Hz, 1H, NH), 8.07-8.12 (m, 1H, NH), 9.20 (s, 1H, Ar-OH), 12.57 (bs, 1H, COOH).

### <Example 31> Preparation of (S)-4-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-oxobutanoic acid (LCA-Val-Asn)

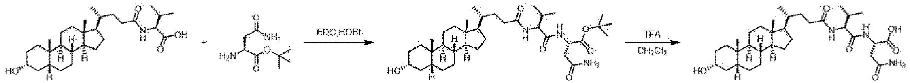

Starting materials including lithocholic acid-Val (100 mg, 0.210 mmol) and L-asparagine tert-butyl ester hydrochloride (0.252 mmol), and 1-hydroxybenzotriazole hydrate (0.252 mmol) were dissolved in 1 mL of dry DCM. Thereafter, 1-(3-dimethylaminopropyl)-3-ethylcarbodiimide (EDC) (0.252 mmol) was added, and an intermediate was obtained in the same manner as the compound in Example 1 (117 mg, 86%). In order to remove the protected part, the intermediate (50 mg, 0.077 mmol) was dissolved in 2 mL of DCM, 1 mL of TFA was added, and a reaction was carried out for 30 minutes. When it was detected that the starting material disappeared using TLC, a solvent was removed using an evaporator, removal was repeated 3 times by dissolving in methanol, and a reaction mixture was purified by column chromatography to obtain a final compound (Example 31; LCA-Val-Asn) (26 mg, 57%).

(S)-4-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-oxobutanoic acid; ¹H-NMR (400 MHz, DMSO-d6) δ = 0.61 (s, 3H, CH3), 0.78-0.92 (m, 13H), 0.97-1.27 (m, 11H), 1.30-1.41 (m, 5H), 1.45-1.55 (m, 3H), 1.57-1.67 (m, 2H), 1.71-1.85 (m, 3H), 1.87-2.04 (m, 3H), 2.18-2.27 (m, 1H), 2.38-2.57 (m, 2H) 4.16 (dd, J = 8.66, 6.74 Hz, 1H, CH), 4.40 (d, J = 6.49 Hz, 1H, OH), 4.88-4.98 (m, 1H, CH), 6.91 (s, 1H, NHH), 7.45 (s, 1H, NHH), 7.83 (d, J = 8.96 Hz, 1H, NH), 7.99 (s, 1H, NH), 12.53 (bs, 1H, COOH).

### <Example 32> Preparation of (S)-5-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-5-oxopentanoic acid (LCA-Val-Gln)

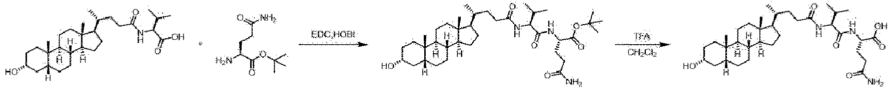

Starting materials including lithocholic acid-Val (100 mg, 0.210 mmol) and L-glutamine tert-butyl ester hydrochloride (0.252 mmol), and 1-hydroxybenzotriazole hydrate (0.252 mmol) were dissolved in 3 mL of dry DCM/1 mL of DMF. Thereafter, 1-(3-dimethylaminopropyl)-3-ethylcarbodiimide (EDC) (0.252 mmol) was added, and an intermediate was obtained in the same manner as the compound in Example 1 (126 mg, 91%). In order to remove the protected part, the intermediate (60 mg, 0.083 mmol) was dissolved in 2 mL of DCM, 1 mL of TFA was added, and a reaction was carried out. When it was detected that the starting material disappeared using TLC, a solvent was removed using an evaporator, removal was repeated 3 times by dissolving in methanol, and a reaction mixture was purified by column chromatography to obtain a final compound (Example 32; LCA-Val-Gln) (26 mg, 57%).

(S)-5-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-5-oxopentanoic acid; ¹H-NMR (400 MHz, DMSO-d6) δ = 0.60 (s, 3H, CH3), 0.78-0.90 (m, 13H), 0.97-1.24 (m, 10H), 1.27-1.39 (m, 7H), 1.44-1.70 (m, 5H), 1.71-1.83 (m, 3H), 1.87-1.99 (m, 3H), 2.05-2.16 (m, 2H), 2.18-2.29 (m, 2H), 3.32-3.41 (m, 1H), 4.05-4.28 (m, 3H), 6.78 (s, 1H), 7.26 (s, 1H), 7.74-7.84 (m, 1H), 8.14-8.25 (m, 1H), 12.46 (bs, 1H, COOH).

### <Example 33> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)succinic acid (LCA-Val-Asp)

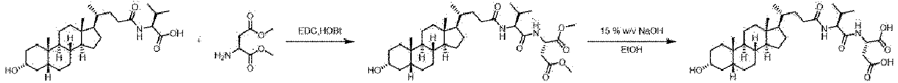

Starting materials including lithocholic acid-Val (100 mg, 0.210 mmol) and L-aspartic acid dimethyl ester hydrochloride (0.252 mmol), and 1-hydroxybenzotriazole hydrate (0.252 mmol) were dissolved in 3 mL of dry DCM. Thereafter, 1-(3-dimethylaminopropyl)-3-ethylcarbodiimide (EDC) (0.252 mmol) was added, and an intermediate was obtained in the same manner as the compound in Example 1 (93.6 mg, 72%). In order to remove the protected part, the intermediate (40 mg, 0.065 mmol) was dissolved in 3 mL of ethanol, 2 mL of 15% w/v sodium hydroxide was added, and a reaction was carried out for 30 minutes. Thereafter, deprotection was performed in the same manner as the compound in Example 1 to obtain a final compound (Example 33; LCA-Val-Asp) (36.4 mg, 95%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)succinic acid; ¹H-NMR (400 MHz, DMSO-d6) δ = 0.59 (d, J = 5.04 Hz, 3H, CH3), 0.77-0.91 (m, 12H), 0.95-1.23 (m, 11H), 1.27-1.40 (m, 7H), 1.44-1.55 (m, 2H), 1.56-1.71 (m, 3H), 1.72-1.84 (m, 2H), 1.87-2.06 (m, 3H), 2.09-2.27 (m, 2H), 2.51-2.72 (m, 2H), 4.16-4.23 (m, 1H), 4.47-4.55 (m, 1H, OH), 7.76-7.85 (m, 1H, NH), 8.26 (dd, J = 22.24, 8.00 Hz, 1H, NH), 12.67 (bs, 1H, COOH), 12.80 (bs, 1H, COOH).

### <Example 34> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanedioic acid (LCA-Val-Glu)

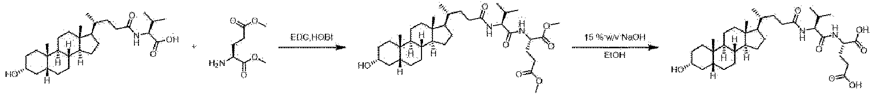

Starting materials including lithocholic acid-Val (100 mg, 0.210 mmol) and dimethyl L-glutamate hydrochloride (0.252 mmol), and 1-hydroxybenzotriazole hydrate (0.252 mmol) were dissolved in 2 mL of dry DCM. Thereafter, 1-(3-dimethylaminopropyl)-3-ethylcarbodiimide (EDC) (0.252 mmol) was added, and an intermediate was obtained in the same manner as the compound in Example 1 (62.8 mg, 94%). In order to remove the protected part, the intermediate (10 mg, 0.016 mmol) was dissolved in 2 mL of ethanol, 1 mL of 15% w/v sodium hydroxide was added, and a reaction was carried out for 30 minutes. Thereafter, deprotection was performed in the same manner as the compound in Example 1 to obtain a final compound (Example 34; LCA-Val-Glu) (9 mg, 92%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanedioic acid; ¹H-NMR (400 MHz, DMSO-d6) δ = 0.59 (d, J = 3.2 Hz, 3H, CH3), 0.79-0.91 (m, 13H), 0.96-1.24 (m, 11H), 1.26-1.39 (m, 7H), 1.45-1.71 (m, 5H), 1.72-1.84 (m, 3H), 1.87-2.06 (m, 3H), 2.10-2.30 (m, 3H), 4.13-4.26 (m, 3H), 4.45 (bs, 1H, OH), 7.77-7.83 (m, 1H, NH), 8.20 (dd, J = 26.12, 7.48 Hz, 1H, NH), 12.15 (bs, 1H, COOH), 12.56 (bs, 1H, COOH).

### <Example 35> Preparation of (S)-6-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)hexanoic acid (LCA-Val-Lys)

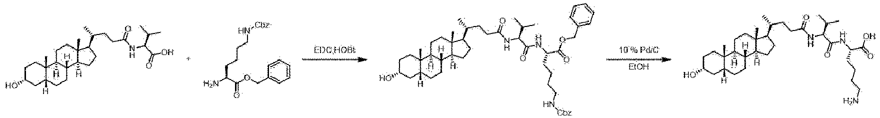

Starting materials including lithocholic acid-Val (100 mg, 0.210 mmol) and Nepsilon-benzyloxycarbonyl-L-lysine benzyl ester hydrochloride (0.252 mmol), and 1-hydroxybenzotriazole hydrate (0.252 mmol) were dissolved in 1 mL of dry DCM. Thereafter, 1-(3-dimethylaminopropyl)-3-ethylcarbodiimide (EDC) (0.252 mmol) was added, and an intermediate was obtained in the same manner as the compound in Example 1 (171 mg, 98%). In order to remove the protected part, the intermediate (50 mg, 0.061 mmol) was dissolved in 5 mL of ethanol, 10% Pd/C was added, and a reaction was carried out. When it was detected that the starting material disappeared using TLC, Pd/C was removed through a celite, ethanol was removed with an evaporator, and a reaction mixture was purified by column chromatography to obtain a final compound (Example 35; LCA-Val-Lys) (37 mg, 87%).

(S)-6-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)hexanoic acid; ¹H-NMR (400 MHz, DMSO-d6) δ = 0.61 (s, 3H, CH3), 0.77-0.91 (m, 13H), 0.97-1.42 (m, 23H), 1.45-1.70 (m, 8H), 1.73-1.85 (m, 3H), 1.89-2.08 (m, 2H), 2.18-2.30 (m, 2H), 3.64-3.72 (m, 1H), 2.18-2.30 (m, 2H), 4.02 (t, J = 6.76 Hz, 1H, CH), 4.45 (bs, 1H, OH), 7.37 (d, J = 5.60 Hz, 1H, NH), 7.95 (d, J = 8.08 Hz, 1H, NH).

### <Example 36> Preparation of (S)-5-guanidino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanoic acid (LCA-Val-Arg)

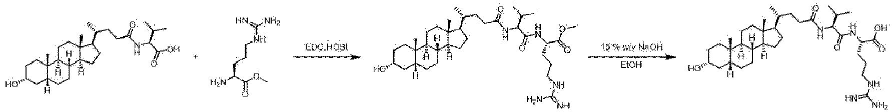

Starting materials including lithocholic acid-Val (100 mg, 0.210 mmol) and L-arginine methyl ester dihydrochloride (0.252 mmol), and N-methylmorpholine (NMM) (0.273 mmol) and 1-hydroxybenzotriazole hydrate (0.273 mmol) were dissolved in 2 mL of dry DMF. Thereafter, 1-(3-dimethylaminopropyl)-3-ethylcarbodiimide (EDC) (0.273 mmol) was added, and an intermediate was obtained in the same manner as the compound in Example 1 (102 mg, 70%). In order to remove the protected part, the intermediate (22 mg, 0.034 mmol) was dissolved in 1 mL of ethanol, 1 mL of 15% w/v sodium hydroxide was added, and a reaction was carried out for 30 minutes. Thereafter, deprotection was performed in the same manner as the compound in Example 1 to obtain a final compound (Example 36; LCA-Val-Arg) (19 mg, 88%).

(S)-5-guanidino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanoic acid; ¹H-NMR (400 MHz, DMSO-d6) δ = 0.60 (s, 3H, CH3), 0.80-0.90 (m, 13H), 0.96-1.25 (m, 12H), 1.27-1.39 (m, 7H), 1.44-1.54 (m, 4H), 1.57-1.84 (m, 6H), 1.88-2.05 (m, 3H), 2.18-2.26 (m, 1H), 3.09 (q, J = 6.36 Hz, 1H), 3.33-3.40 (m, 1H, H-OH), 4.13-4.19 (m, 2H), 6.87 (bs, 1H), 7.32 (bs, 1H), 7.57 (t, J = 5.24 Hz, 1H), 7.84 (d, J = 9.44 Hz, 1H, NH), 8.21 (d, J = 7.48 Hz, 1H, NH), 12.64 (bs, 1H, COOH).

### <Example 37> Preparation of (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-imidazol-4-yl)propanoic acid (LCA-Val-His)

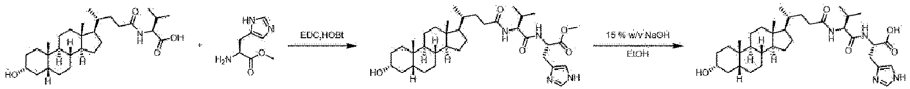

Starting materials including lithocholic acid-Val (100 mg, 0.210 mmol) and L-histidine methyl ester dihydrochloride (0.252 mmol), and N-methylmorpholine (NMM) (0.273 mmol) and 1-hydroxybenzotriazole hydrate (0.273 mmol) were dissolved in 2 mL of dry DMF. Thereafter, 1-(3-dimethylaminopropyl)-3-ethylcarbodiimide (EDC) (0.273 mmol) was added, and an intermediate was obtained in the same manner as the compound in Example 1 (104 mg, 79%). In order to remove the protected part, the intermediate (50 mg, 0.079 mmol) was dissolved in 2 mL of ethanol, 2 mL of 15% w/v sodium hydroxide was added, and a reaction was carried out for 30 minutes. Thereafter, deprotection was performed in the same manner as the compound in Example 1 to obtain a final compound (Example 37; LCA-Val-His) (20 mg, 41%).

(S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-imidazol-4-yl)propanoic acid; ¹H-NMR (400 MHz, DMSO-d6) δ = 0.59 (s, 3H, CH3), 0.80-0.91 (m, 13H), 0.96-1.24 (m, 11H), 1.27-1.38 (m, 7H), 1.45-1.71 (m, 5H), 1.72-1.83 (m, 2H), 1.87-2.05 (m, 3H), 2.16-2.26 (m, 1H), 2.97-3.04 (m, 1H), 3.10-3.17 (m, 1H), 4.06 (t, J = 8.206 Hz, 1H, CH), 4.50-4.58 (m, 1H, OH), 7.42 (s, 1H, CH), 7.91 (d, J = 8.56 Hz, 1H, NH), 8.42 (d, J = 7.60 Hz, 1H, NH), 9.00 (s, 1H, CH), 12.92 (bs, 1H, COOH), 14.18 (bs, 1H, NH).

The chemical structures of Examples 18 to 37 are shown in Table 2 below.

**TABLE 2**

| Example | Chemical structure | Example | Chemical structure |
|---|---|---|---|
| 18 (LCA-Val-Gly) | 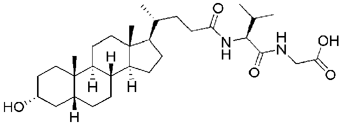 | 28 (LCA-Val-Thr) | 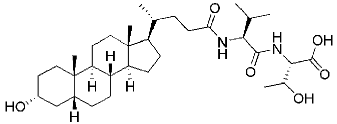 |
| 19 (LCA-Val-Ala) | 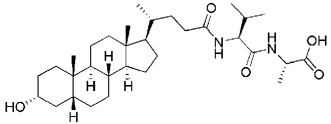 | 29 (LCA-Val-Cys) | 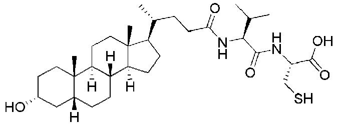 |
| 20 (LCA-Val-Val) | 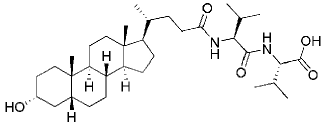 | 30 (LCA-Val-Tyr) | 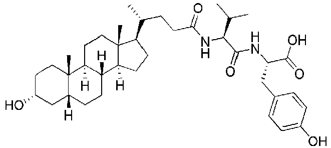 |
| 21 (LCA-Val-Leu) | 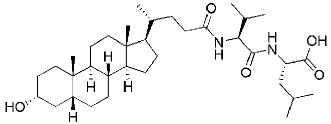 | 31 (LCA-Val-Asn) | 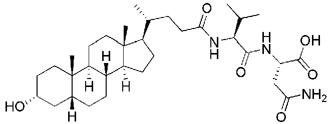 |
| 22 (LCA-Val-Ile) | 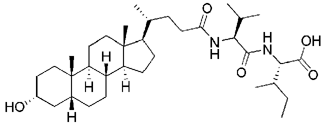 | 32 (LCA-Val-Gln) | 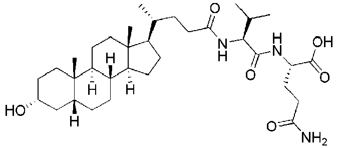 |
| 23 (LCA-Val-Met) | 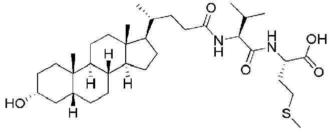 | 33 (LCA-Val-Asp) | 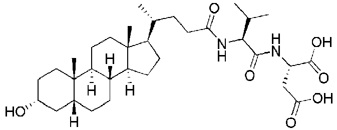 |
| 24 (LCA-Val-Phe) | 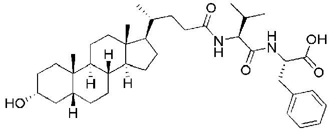 | 34 (LCA-Val-Glu) | 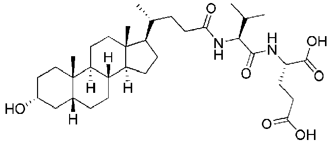 |
| 25 (LCA-Val-Trp) | 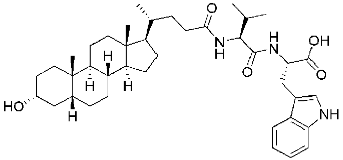 | 35 (LCA-Val-Lys) | 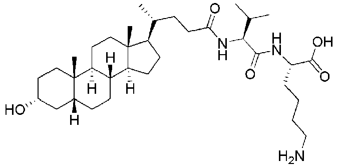 |
| 26 (LCA-Val-Pro) | 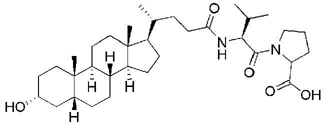 | 36 (LCA-Val-Arg) | 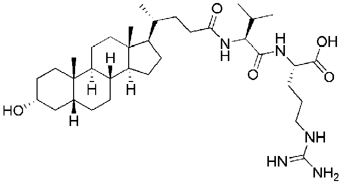 |
| 27 (LCA-Val-Ser) | 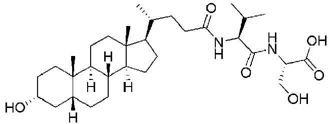 | 37 (LCA-Val-His) | 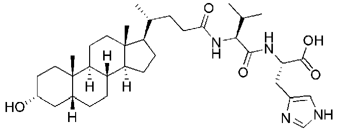 |

### <Example 38> Preparation of 1-((R)-4-((3R,5R,8R,9S,10S,13R,14S,17R)-3-hydroxy-10,13-dimethyl-hexadecahydro-1H-cyclopenta[a]phenanthren-17-yl)pentanoyl)pyrrolidine-2-carboxylic acid (LCA-Pro)

Using lithocholic acid (100 mg, 0.264 mmol) and L-proline methyl ester hydrochloride as starting materials, a final compound (Example 38; LCA-Pro) was obtained in the same manner as the compound 1.

¹H-NMR (400 MHz, DMSO-d6) δ = 0.61 (s, 3H, CH3), 0.82-0.91 (m, 7H), 0.98-1.22 (m, 10H), 1.23-1.39 (m, 7H), 1.45-1.71 (m, 6H), 1.72-1.95 (m, 6H), 1.99-2.31 (m, 4H), 3.34-3.36 (m, 1H), 3.47-3.52 (m, 1H), 4.18 (q, J = 4.17, 1H, CH), 12.33 (bs, 1H, COOH).

### <Example 39> Preparation of (2S,3S)-3-hydroxy-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)butanoic acid (LCA-Thr)

Using lithocholic acid and L-threonine methyl ester hydrochloride as starting materials, a final compound (Example 39; LCA-Thr) was obtained in the same manner as the compound 1.

¹H-NMR (400 MHz, DMSO-d6) δ = 0.60 (s, 3H, CH3), 0.80-0.90 (m, 13H), 0.99-1.22 (m, 11H), 1.29-1.39 (m, 7H), 1.46-1.55 (m, 2H), 1.58-1.82 (m, 6H), 1.82-1.88 (m, 1H), 1.99-2.08 (m, 1H), 2.15-2.23 (m, 1H), 3.32-3.35 (m, 1H), 4.15 (t, J = 7.0 Hz, 1H, CHCHCH3CH2), 4.45 (s, 1H, OH), 7.94 (d, J = 8.4 Hz, 1H, NH). 12.50 (bs, 1H, COOH).

### <Example 40> Preparation of (R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-mercaptopropanoic acid (LCA-Cys)

Using lithocholic acid and L-cysteine methyl ester hydrochloride as starting materials, a final compound (Example 40; LCA-Cys) was obtained in the same manner as the compound 1.

¹H-NMR (400 MHz, DMSO-d6) δ = 0.61 (s, 3H, CH3), 0.82-0.93 (m, 7H), 0.98-1.24 (m, 10H), 1.26-1.40 (m, 7H), 1.44-1.71 (m, 6H), 1.72-1.85 (m, 2H), 1.89-1.96 (m, 1H), 2.00-2.09 (m, 1H), 2.13-2.22 (m, 1H), 2.66-2.77 (m, 1H), 2.78-2.89 (m, 1H), 4.30-4.39 (m, 1H), 4.45 (bs, 1H, OH), 8.13 (d, J = 7.20 Hz, 1H, NH), 12.80 (bs, 1H, COOH).

### <Example 41> Preparation of (S)-5-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-5-oxopentanoic acid (LCA-Gln)

Using lithocholic acid and L-glutamine tert butyl ester hydrochloride as starting materials, a final compound (Example 41; LCA-Gln) was obtained in the same manner as the compound 1.

¹H-NMR (400 MHz, DMSO-d6) δ = 0.61 (s, 3H, CH3), 0.84-0.92 (m, 7H), 1.01-1.12 (m, 5H), 1.16-1.27 (m, 9H), 1.31-1.40 (m, 5H), 1.45-1.56 (m, 4H), 1.70-1.83 (m, 4H), 1.88-1.95 (m, 2H), 2.06-2.13 (m, 2H), 4.07-4.14 (m, 1H), 4.88-4.98 (m, 1H), 6.79 (s, 1H, NHH), 7.30 (s, 1H, NHH), 8.08 (d, J = 7.84 Hz, 1H, NH), 12.51 (bs, 1H, COOH).

### <Example 42> Preparation of (S)-5-guanidino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)pentanoic acid (LCA-Arg)

Using lithocholic acid and L-arginine methyl ester dihydrochloride as starting materials, a final compound (Example 42; LCA-Arg) was obtained in the same manner as the compound 1.

¹H-NMR (400 MHz, DMSO-d6) δ = 0.60 (s, 3H, CH3), 0.83-0.90 (s, 7H), 0.99-1.22 (m, 12H), 1.29-1.39 (m, 8H), 1.45-1.56 (m, 4H), 1.58-1.70 (m, 4H), 1.74-1.83 (m, 2H), 1.94-2.02 (m, 1H), 2.10-2.19 (m, 1H), 3.03 (d, J = 5.08 Hz, 1H), 3.92 (q, J = 6.64 Hz, 1H), 4.44 (s, 1H, OH), 5.76 (s, 1H), 7.17-7.84 (m, 4H), 9.30 (s, 1H).

### <Example 43> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(1H-imidazol-5-yl)propanoic acid (LCA-His)

Using lithocholic acid and L-histidine methyl ester dihydrochloride as starting materials, a final compound (Example 43; LCA-His) was obtained in the same manner as the compound 1.

¹H-NMR (400 MHz, DMSO-d6) δ = 0.58 (s, 3H, CH3), 0.80-0.89 (s, 7H), 0.96-1.22 (m, 10H), 1.26-1.39 (m, 8H), 1.45-1.70 (m, 4H), 1.63-1.70 (m, 1H), 1.72-1.81 (m, 2H), 1.87-1.94 (m, 1H), 1.95-2.02 (m, 1H), 2.04-2.12 (m, 1H), 2.95 (dd, J = 15.24, 9.84 Hz, 1H), 3.11 (dd, J = 15.10, 5.22 Hz, 1H), 4.44-4.56 (m, 2H), 7.35 (s, 1H), 8.23 (d, J = 8.44 Hz, 1H, NH), 8.94 (s, 1H), 13.98 (bs, 1H).

### <Example 44> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoic acid (LCA-Val)

Using lithocholic acid and L-valine methyl ester hydrochloride as starting materials, a final compound (Example 44; LCA-Val) was obtained in the same manner as the compound 1.

¹H-NMR (400 MHz, DMSO-d6) δ = 0.60 (s, 3H), 0.80-0.95 (m, 13H), 0.95-2.27 (m, 28H), 3.30-3.33 (m, 1H), 4.05-4.15 (m, 1H), 4.38-4.48 (m, 1H), 7.83-7.92 (m, 1H). 12.46 (bs, 1H).

### <Example 45> Preparation of 2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-acetic acid (LCA-Gly)

Using lithocholic acid and L-glycine methyl ester hydrochloride as starting materials, a final compound (Example 45; LCA-Gly) was obtained in the same manner as the compound 1.

¹H-NMR (400 MHz, DMSO-d6)δ = 0.60 (s, 3H), 0.83-0.90 (m, 6H), 0.92-2.18 (m, 28H), 3.19-3.33 (m, 1H), 3.62 (d, J = 6 Hz, 2H), 4.40-4.46 (m, 1H), 7.88-7.96 (m, 1H), 12.45 (bs, 1H).

### <Example 46> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-propanoic acid (LCA-Ala)

Using lithocholic acid and L-alanine methyl ester hydrochloride as starting materials, a final compound (Example 46; LCA-Ala) was obtained in the same manner as the compound 1.

¹H-NMR (400 MHz, DMSO-d6)δ = 0.60 (s, 3H), 0.83-0.93 (m, 9H), 0.98-2.16 (m, 28H), 3.19-3.33 (m, 1H), 3.56-3.68 (m, 1H), 4.40-4.46 (m, 1H), 7.88-7.96 (m, 1H), 12.45 (bs, 1H).

### <Example 47> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Leu)

Using lithocholic acid and L-leucine methyl ester hydrochloride as starting materials, a final compound (Example 47; LCA-Leu) was obtained in the same manner as the compound 1.

¹H-NMR (400 MHz, DMSO-d6)δ = 0.60 (s, 3H), 0.83-0.90 (m, 13H), 0.92-2.18 (m, 30H), 3.29-3.36 (m, 1H), 4.13-4.23 (m, 1H), 4.40-4.47 (m, 1H), 8.00-8.20 (m, 1H), 12.43 (bs, 1H).

### <Example 48> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-4-(methylthio)butanoic acid (LCA-Met)

Using lithocholic acid and L-methionine methyl ester hydrochloride as starting materials, a final compound (Example 48; LCA-Met) was obtained in the same manner as the compound 1.

¹H-NMR (400 MHz, DMSO-d6)δ = 0.60 (s, 3H), 0.83-0.90 (m, 7H), 0.92-2.18 (m, 34H), 3.19-3.33 (m, 1H), 4.20-4.29 (m, 1H), 4.41-4.48 (m, 1H), 8.00-8.09 (m, 1H), 12.57 (bs, 1H).

### <Example 49> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-phenylpropanoic acid (LCA-Phe)

Using lithocholic acid and L-phenylalanine methyl ester hydrochloride as starting materials, a final compound (Example 49; LCA-Phe) was obtained in the same manner as the compound 1.

¹H-NMR (400 MHz, DMSO-d6)δ = 0.58 (s, 3H), 0.78-0.88 (m, 6H), 0.91-2.09 (m, 28H), 2.77-2.88 (m, 1H), 2.97-3.07 (m, 1H), 3.27-3.39 (m, 1H), 4.24-4.33 (m, 1H), 4.38-4.50 (m, 1H), 7.07-7.28 (m, 5H), 7.85-7.97 (m, 1H), 12.45 (bs, 1H)

### <Example 50> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(1H-indol-3-yl)propanoic acid (LCA-Trp)

Using lithocholic acid and L-tryptophan methyl ester hydrochloride as starting materials, a final compound (Example 50; LCA-Trp) was obtained in the same manner as the compound 1.

¹H-NMR (400 MHz, DMSO-d6)δ = 0.61 (s, 3H), 0.79-0.91 (m, 7H), 0.91-2.12 (m, 28H), 2.93-3.01 (m, 1H), 3.10-3.17 (m, 1H), 3.32-3.42 (m, 1H), 4.38-4.46 (m, 1H), 6.96 (t, J = 7.6 Hz, 1H), 7.05 (t, J = 7.6 Hz, 1H), 7.09-7.13 (m, 1H), 7.32 (d, J = 7.6 Hz, 1H), 7.51 (d, J = 8 Hz, 1H), 8.06 (d, J = 8 Hz, 1H), 10.83 (s, 1H), 12.56 (bs, 1H).

### <Example 51> Preparation of (S)-3-hydroxy-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)propanoic acid (LCA-Ser)

Using lithocholic acid and L-serine methyl ester hydrochloride as starting materials, a final compound (Example 51; LCA-Ser) was obtained in the same manner as the compound 1.

¹H-NMR (400 MHz, DMSO-d6)δ = 0.60 (s, 3H), 0.83-0.91 (m, 7H), 0.91-1.40 (m, 18H), 1.42-1.96 (m, 7H), 1.99-2.21 (m, 2H), 3.30-3.38 (m, 1H), 3.53-3.68 (m, 2H), 4.18-4.27 (m, 1H), 4.41-4.47 (m, 1H), 4.93 (bs, 1H), 7.93 (d, J = 8 Hz, 1H), 12.51 (bs, 1H).

### <Example 52> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(4-hydroxyphenyl)propanoic acid (LCA-Tyr)

Using lithocholic acid and L-tyrosine methyl ester hydrochloride as starting materials, a final compound (Example 52; LCA-Tyr) was obtained in the same manner as the compound 1.

¹H-NMR (400 MHz, DMSO-d6)δ = 0.58 (s, 3H), 0.80-0.91 (m, 7H), 0.91-1.39 (m, 17H), 1.42-1.83 (m, 7H), 1.85-2.11 (m, 3H), 2.65-2.74 (m, 1H), 2.85-2.92 (m, 1H), 3.29-3.37 (m, 1H), 4.23-4.31 (m, 1H), 4.42-4.48 (m, 1H), 6.62 (d, J = 8 Hz, 2H), 6.99 (d, J = 8 Hz, 2H), 7.99-8.04 (m, 1H), 9.19 (s, 1H), 12.57 (bs, 1H).

### <Example 53> Preparation of (S)-4-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-4-oxobutanoic acid (LCA-Asn)

Using lithocholic acid and L-asparagine tert-butyl ester hydrochloride as starting materials, a final compound (Example 53; LCA-Asn) was obtained in the same manner as the compound 1.

¹H-NMR (400 MHz, DMSO-d6)δ = 0.61 (s, 3H), 0.83-0.93 (m, 7H), 0.95-2.16 (m, 27H), 2.36-2.56 (m, 2H), 3.23-3.35 (m, 1H), 4.40-4.50 (m, 1H), 4.87-4.98 (m, 1H), 6.87 (s, 1H), 7.34 (s, 1H), 7.92-7.99 (m, 1H), 12.45 (bs, 1H).

### <Example 54> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)succinic acid (LCA-Asp)

Using lithocholic acid and L-aspartic acid dimethyl ester hydrochloride as starting materials, a final compound (Example 54; LCA-Asp) was obtained in the same manner as the compound 1.

¹H-NMR (400 MHz, DMSO-d6)δ = 0.60 (s, 3H), 0.81-0.90 (m, 7H), 0.91-2.16 (m, 28H), 2.47-2.56 (m, 1H), 2.61-2.69 (m, 1H), 3.28-3.37 (m, 1H), 4.42-4.51 (m, 1H), 8.08-8.16 (m, 1H), 12.37 (bs, 1H), 12.62 (bs, 1H).

### <Example 55> Preparation of (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)pentanedioic acid (LCA-Glu)

Using lithocholic acid and dimethyl L-glutamate hydrochloride as starting materials, a final compound (Example 55; LCA-Glu) was obtained in the same manner as the compound 1.

¹H-NMR (400 MHz, DMSO-d6)δ = 0.60 (s, 3H), 0.83-0.91 (m, 7H), 0.91-2.20 (m, 27H), 2.20-2.55 (m, 4H), 3.32-3.37 (m, 1H), 4.11-4.19 (m, 1H), 4.44-4.50 (m, 1H), 8.01-8.10 (m, 1H), 12.50 (bs, 2H).

### <Example 56> Preparation of (S)-6-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)hexanoic acid (LCA-Lys)

Using lithocholic acid and Nepsilon-benzyloxycarbonyl-L-lysine benzyl ester hydrochloride as starting materials, a final compound (Example 56; LCA-Lys) was obtained in the same manner as the compound 35.

¹H-NMR (400 MHz, DMSO-d6)δ = 0.59 (s, 3H), 0.75-2.17 (m, 42H), 3.13-3.18 (m, 2H), 3.26-3.37 (m, 1H), 4.08-4.16 (m, 1H), 4.42-4.50 (m, 1H), 7.06-7.13 (m, 1H).

The chemical structures of Examples 38 to 56 are shown in Table 3 below.

**TABLE 3**

| Example | Chemical structure | Example | Chemical structure |
|---|---|---|---|
| 38 (LCA-Pro) | 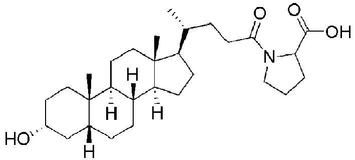 | 48 (LCA-Met) | 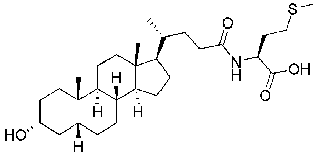 |
| 39 (LCA-Thr) | 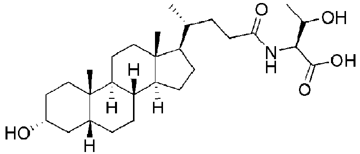 | 49 (LCA-Phe) | 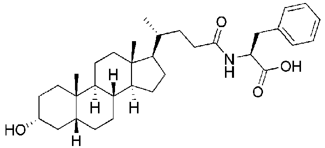 |
| 40 (LCA-Cys) | 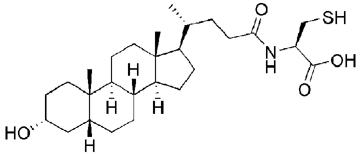 | 50 (LCA-Trp) | 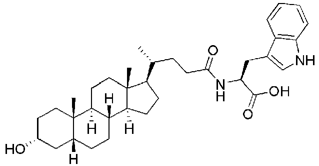 |
| 41 (LCA-Gln) | 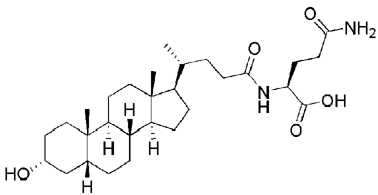 | 51 (LCA-Ser) | 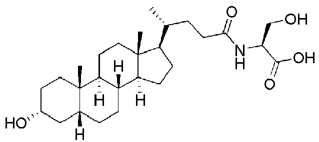 |
| 42 (LCA-Arg) | 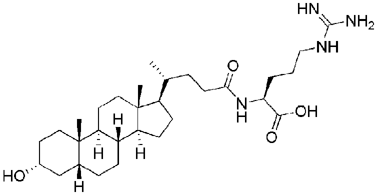 | 52 (LCA-Tyr) | 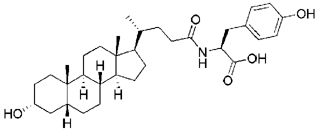 |
| 43 (LCA-His) | 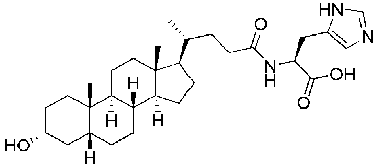 | 53 (LCA-Asn) | 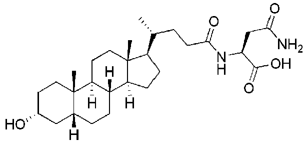 |
| 44 (LCA-Val) | 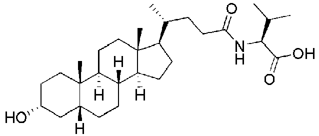 | 54 (LCA-Asp) | 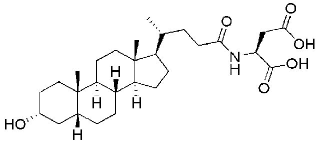 |
| 45 (LCA-Gly) | 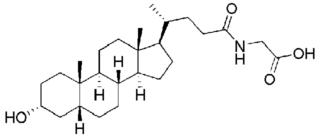 | 55 (LCA-Glu) | 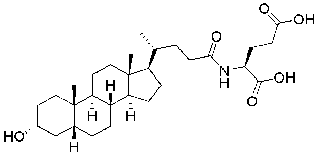 |
| 46 (LCA-Ala) | 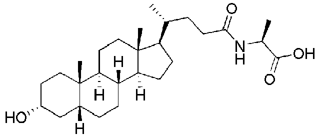 | 56 (LCA-Lys) | 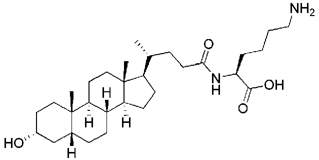 |
| 47 (LCA-Leu) | 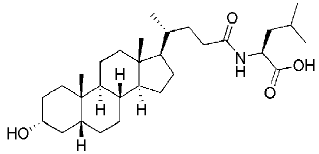 | | |

### <Comparative Examples 1 to 6> Preparation of lithocholic acid and derivatives thereof

As Comparative Example, the known compounds lithocholic acid (LCA), ursodeoxycholic acid (UDCA), cholic acid (CA), hyodeoxycholic acid (HDCA), deoxycholic acid (DCA), and chenodeoxycholic acid (CDCA) were prepared.

The chemical structures of Comparative Examples 1 to 6 are shown in Table 4 below.

**TABLE 4**

| Comparativ e Example | Chemical structure | Comparativ e Example | Chemical structure |
|---|---|---|---|
| 1 (LCA) | 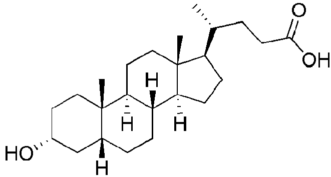 | 4 (HDCA) | 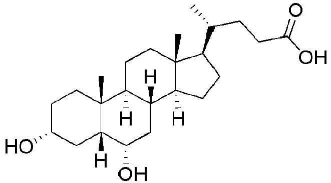 |
| 2 (UDCA) | 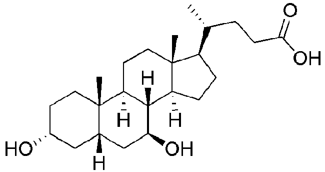 | 5 (DCA) | 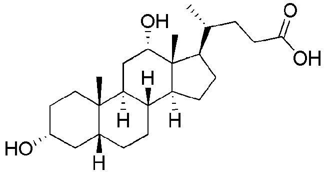 |
| 3 (CA) | 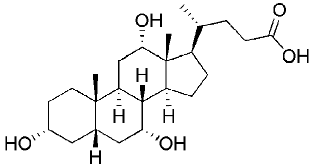 | 6 (CDCA) | 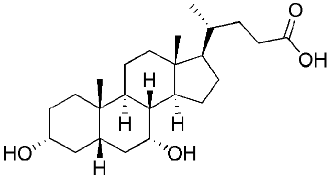 |

### <Experimental Example 1> Evaluation of an ability to inhibit the binding between preS1 and NTCP expressing cell lines

### 1-1. Comparison of Example 1, Examples 38 to 56, and Comparative Example 1

HepG2-hNTCP-C4 cell line, a cell line expressing NTCP, was seeded in a 6 well plate at a density of 2×10⁵/well and incubated at 4°C for 30 minutes when the confluency reached 80% after overnight incubation for 2 days. Thereafter, 20 nM PreS 1 (Myr-GTNLSVPNPLGFFPDHQLDPAFGANSNNPDWDFNPNKDHWPEANQV-Lys(FITC)) and the compounds in Example 1, Examples 38 to 56 or the compound in Comparative Example 1 were treated by diluting in culture media at 4°C at a concentration of 50 µM respectively, and the media was suctioned after 2 hours, followed by washing 3 times with 4°C PBS. In order to take the cells off from the plate, 500 µl of 0.5 mM EDTA PBS solution was treated, and then incubation was performed at 37°C for 15 minutes to check whether the cells fell off through an optical microscope. After taking off the cells by pipetting, the cells were collected in a 1.5 ml tube and centrifuged (4000 rpm, 4°C, 3 min). After suctioning the supernatant, the pellet was washed 3 times with 4°C PBS, and then green fluorescence was measured via flow cytometry. In this case, taurocholic acid (TCA), a compound known as a substrate of NTCP to reduce the entry of HBV or HDV, was used as a positive control group.

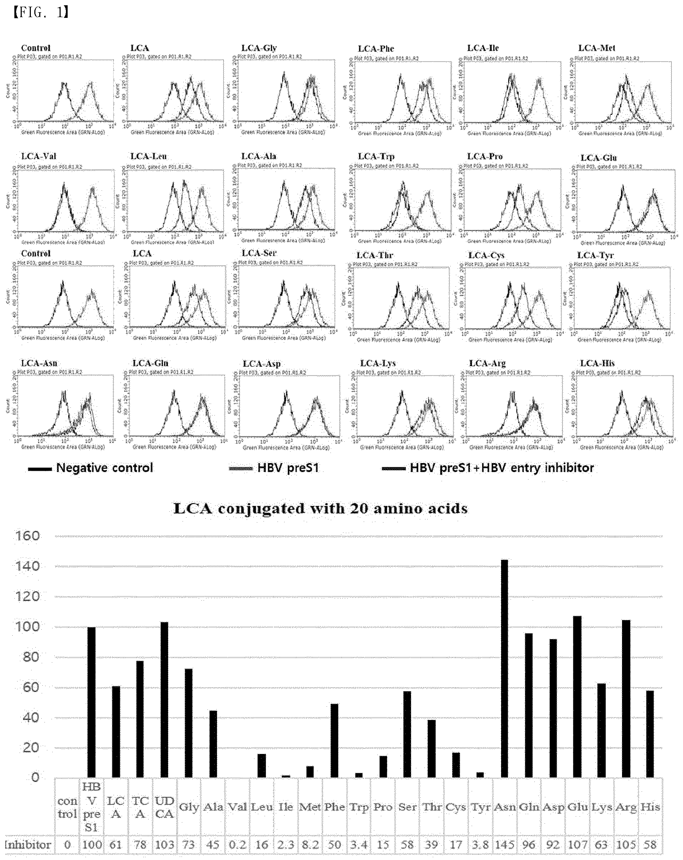

FIG. 1 is graphs showing results of identifying an inhibitory ability to inhibit the binding between PreS1 to NTCP expressing cell lines, for compounds in Example 1, Examples 38 to 56, and Comparative Example 1 according to the present disclosure.

In the graph at the top of FIG. 1, black (1) and red (2) indicate a negative control group with no treatment and an HBV-induced group (HBV preS1) solely treated with PreS1, respectively. Blue (HBV preS1+HBV entry inhibitor) (3) is the fluorescence value for each of Examples or Comparative Examples, evaluated that the closer to the negative control group, the more inhibited the binding between PreS1 and the NTCP cell line. The graph at the bottom of FIG. 1 shows the relative value (%) of fluorescence compared to the HBV-induced group (HBV PreS1) solely treated with PreS1, indicating that the lower the value, the more inhibited the binding between PreS1 and NTCP.

As a result of the analysis, compared to Comparative Example 1 (LCA), relative fluorescence intensity was low in Examples 46 (LCA-Ala), 44 (LCA-Val), 47 (LCA-Leu), 1 (LCA-Ile), 48 (LCA-Met), 49 (LCA-Phe), 50 (LCA-Trp), 38 (LCA-Pro), 51 (LCA-Ser), 39 (LCA-Thr), 40 (LCA-Cys), 52 (LCA-Tyr), Example 56 (LCA-Lys), and 43 (LCA-His), and the lower relative fluorescence intensity was observed in Examples 44 (LCA-Val), 47 (LCA-Leu), 1 (LCA-Ile), 48 (LCA-Met), 50 (LCA-Trp), 38 (LCA-Pro), 39 (LCA-Thr), 40 (LCA-Cys), and 52 (LCA-Tyr).

Thereamong, it was found that Example 44 (LCA-Val), 47 (LCA-Leu), 1 (LCA-Ile), 48 (LCA-Met), 50 (LCA-Trp), 38 (LCA-Pro), 40 (LCA-Cys), and 52 (LCA-Tyr) showed the relative fluorescence intensity less than or equal to 20% to have remarkably superior binding inhibitory ability, and in particular, the inhibitory ability of compounds in Example 44 (LCA-Val), Example 1 (LCA-Ile), Example 50 (LCA-Trp), and Example 52 (LCA-Tyr) that showed the relative fluorescence intensity less than 5% compared to the HBV-induced group (HBV PreS1) solely treated with PreS1 to be the most excellent.

### 1-2. Comparison of Examples 1, 44, 50, and 52 by treatment concentration

Additional checking was followed on whether there is concentration dependence for Example 1 (LCA-Ile), Example 50 (LCA-Trp), Example 52 (LCA-Tyr), and Example 44 (LCA-Val) which are four compounds that showed good inhibitory ability in the Experimental Example 1-1. The experimental method was the same as in the Experimental Example 1-1, and 20 nM PreS1 and each compound were treated at 4 concentrations (0.2 µM, 2 µM, 10 µM, 50 µM).

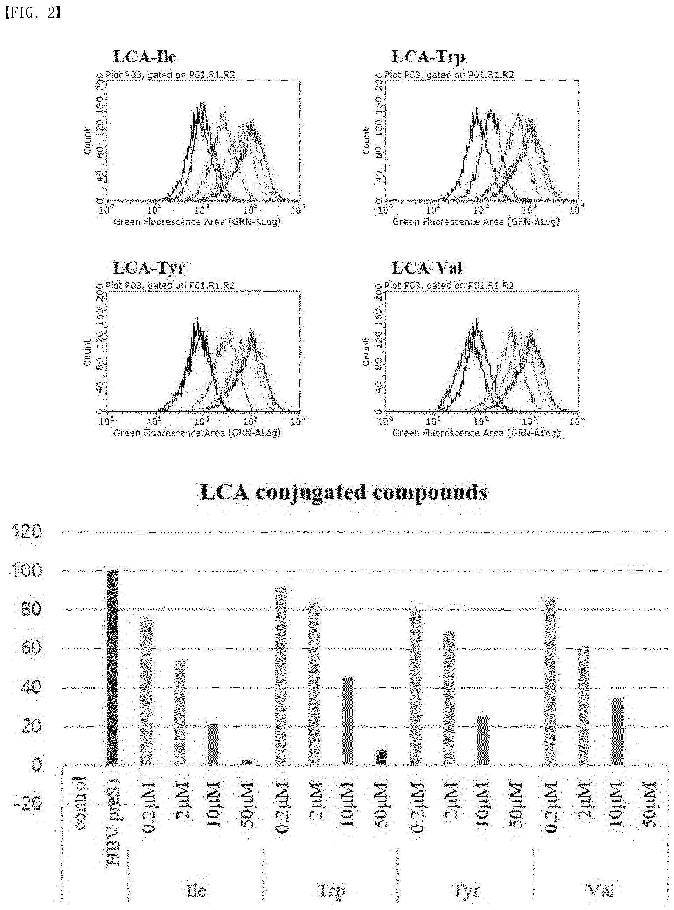

FIG. 2 is graphs showing results of identifying an ability to inhibit the binding between PreS1 and NTCP expressing cell lines for compounds in Example 1 (LCA-Ile), Example 50 (LCA-Trp), Example 52 (LCA-Tyr), and Example 44 (LCA-Val) according to the present disclosure.

In the graph, a negative control group with no treatment and an HBV-induced group (HBV PreS1) solely treated with PreS1 are shown, and a group treated with compounds in Examples (HBV PreS1+HBV entry inhibitor) is indicated by concentration.

As shown in FIG. 2, Example 1 (LCA-Ile), Example 50 (LCA-Trp), Example 52 (LCA-Tyr), and Example 44 (LCA-Val) all had the effect of inhibiting the binding between PreS1 and NTCP expressing cell lines in a concentration dependent manner.

### 1-3. Comparison of Examples 1 to 3, Examples 6 to 11, Examples 14 to 15, Example 44, and Comparative Examples 1 to 6

With an expectation that there is a structure-activity relationship among compounds composed of bile acid and amino acids, for each of bile acids in Comparative Example 1 (lithocholic acid, LCA), Comparative Example 2 (ursodeoxycholic acid, UDCA), Comparative Example 3 (cholic acid, CA), Comparative Example 4 (hyodeoxycholic acid, HDCA), Comparative Example 5 (deoxycholic acid, DCA), and Comparative Example 6 (chenodeoxycholic acid, CDCA), an evaluation experiment was conducted on an ability to inhibit the binding between PreS1 and NTCP expressing cell lines, and also for Examples 1 (LCA-Ile) and Example 44 (LCA-Val) as well as the compounds in Example 2 (UDCA-Ile), Example 3 (UDCA-Val), Example 6 (CA-Ile), Example 7 (CA-Val), Example 8 (HDCA-Ile), Example 9 (HDCA-Val), Example 10 (DCA-Ile), Example 11 (DCA-Val), Example 14 (CDCA-Ile), and Example 15 (CDCA-Val) in which bile acid parts (lithocholic acid) of Example 1 (LCA-Ile) and Example 44 (LCA-Val) were substituted with other types of bile acids in Comparative Examples 2 to 6, an evaluation experiment was conducted on an ability to inhibit the binding between PreS1 and NTCP expressing cell lines. The experimental method was the same as in the Experimental Example 1-1, and 18 compounds in the Comparative Examples 1 to 6, Examples 1 to 3, Examples 6 to 11, Examples 14 to 15, and Example 44 were treated at a concentration of 50 µM along with 20 nM PreS 1.

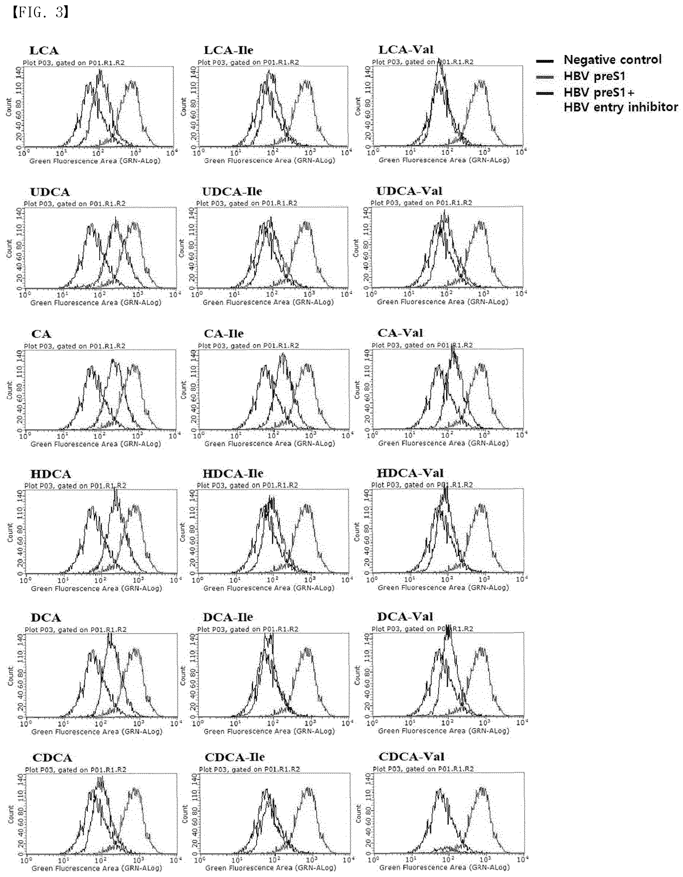

FIG. 3 is graphs showing results of identifying an ability to inhibit the binding between PreS1 and NTCP expressing cell lines, for compounds in Examples 1 to 3, Examples 6 to 11, Examples 14 to 15, Example 44, and Comparative Examples 1 to 6 according to the present disclosure.

As a result of the analysis, it was found that the compounds in Example 1 (LCA-Ile), Example 44 (LCA-Val), Example 2 (UDCA-Ile), Example 3 (UDCA-Val), Example 8 (HDCA-Ile), Example 9 (HDCA-Val), Example 10 (DCA-Ile), and Example 14 (CDCA-Ile) had relatively excellent ability to inhibit the binding between PreS1 and NTCP expressing cell lines.

### 1-4. Comparison of Examples 4 to 5, Examples 12 to 13, Examples 16 to 17, and Comparative Examples 2, 5, and 6

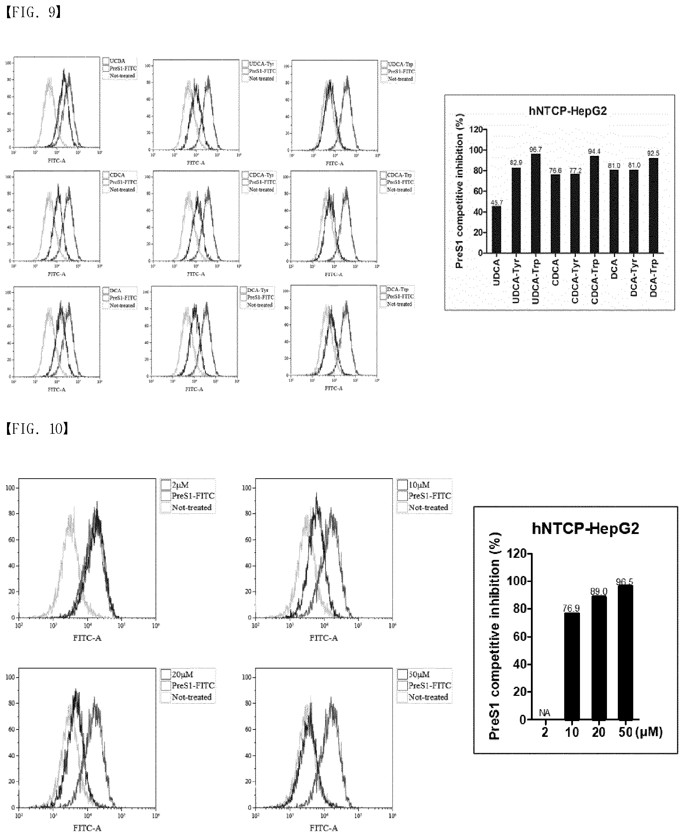

In FIG. 9, an evaluation experiment was conducted to check a degree of inhibiting the binding between PreS1 and NTCP expressing cell lines for compounds in Examples 4 to 5, Examples 12 to 13, Examples 16 to 17, and Comparative Examples 2 (ursodeoxycholic acid, UDCA), Comparative Examples 5 (deoxycholic acid, DCA), and Comparative Examples 6 (chenodeoxycholic acid, CDCA) according to the present disclosure. The experimental method was the same as the Experimental Example 1-1.

In the graph at the top of FIG. 9, gray (3) and red (2) indicate a negative control group (Not-treated) with no treatment and an HBV-induced group (PreS1-FITC) solely treated with PreS1, respectively. The blue (1) is a fluorescence value for each of Examples or Comparative Examples.

As a result of the analysis, in the comparison of Comparative Example 2 (UDCA) with Example 4 (UDCA-Trp) or Example 5 (UDCA-Tyr), the relative fluorescence intensity of Example 4 or Example 5 was low. Compared to the HBV-induced group (PreS1-FITC) solely treated with PreS1, a degree of competitive inhibition differed by about 2 times, showing excellent binding inhibitory ability.

In the comparison of Example 5 (DCA) with Example 12 (DCA-Trp) or Example 13 (DCA-Tyr), the relative fluorescence intensity of Example 12 or Example 13 was observed to be the same or lower. The degree of competitive inhibition compared to the HBV-induced group (PreS1-FITC) solely treated with PreS1 differed by 11.6% in the comparison of Comparative Example 5 and Example 12, showing excellent binding inhibitory ability.

In the comparison of Comparative Example 6 (CDCA) with Example 16 (CDCA-Trp) or Example 17 (CDCA-Tyr), the relative fluorescence intensity of Example 16 or Example 17 was low. The degree of competitive inhibition compared to the HBV-induced group (PreS1-FITC) solely treated with PreS1 differed by 17.8% in the comparison of Comparative Example 6 and Example 16, showing excellent binding inhibitory ability.

### 1-5. Comparison of Example 4 by treatment concentration

In the Experimental Examples 1-4, it was determined whether there is additionally concentration dependence for Example 4 (UDCA-Trp), a compound with good inhibitory ability. The experimental method was the same as in the Experimental Example 1-1, and 20 nM PreS 1 and each compound were treated at 4 concentrations (2 µM, 10 µM, 20 µM, 50 µM).

FIG. 10 is graphs showing results of identifying an ability to inhibit the binding between PreS1 and NTCP expressing cell lines for compound in Example 4 (UDCA-Trp) according to the present disclosure.

In the graph, gray (3) and red (2) indicate a negative control group (Not-treated) with no treatment and an HBV-induced group (PreS1-FITC)solely treated with PreS1, respectively, and blue (1) indicates values expressed by fluorescence values of compounds in Example 4 (UDCA-Trp) treated with four different concentrations, respectively.

As shown in FIG. 10, it was found that Example 4 (UDCA-Trp) had an effect of inhibiting the binding between PreS 1 and NTCP expressing cell lines in a concentration dependent manner.

### <Experimental Example 2> Evaluation of an ability to inhibit the binding of fluorescent-labeled PreS 1 peptide

Based on results of the Experimental Examples 1-1 to 1-3, for Examples 1 (LCA-Ile) and Example 44 (LCA-Val) as well as Example 2 (UDCA-Ile) and Example 3 (UDCA-Val), which are in a form in which amino acids are bound to UDCA, known to be less toxic when degraded in the human body, an evaluation experiment was conducted on the ability to inhibit the binding between PreS 1 and NTCP.

Specifically, after placing a cover slip coated with fibronectin on a 24 well plate, a HepG2-hNTCP-C4 cell line, which is a cell line expressing NTCP, was seeded at a density of 1×10⁵, followed by incubation overnight. Thereafter, 20 nM PreS 1 and the compounds in Examples 1, 8, 9, and 44 were treated by diluting in culture media at 4°C at a concentration of 50 µM, respectively. After 2 hours, the media were suctioned, followed by washing twice with 4°C PBS, fixation with 4% paraformaldehyde for 20 minutes, and washing twice with 4°C PBS. Thereafter, 300 nM 4',6-diamidino-2-phenylindole (DAPI) was treated to stain the nucleus for 20 minutes, and then washing was followed once with distilled water (DW) and twice with 4°C PBS. Subsequently, 10 µl of mounting media was loaded onto a slide glass, and a cover slip was placed thereon for observation under a fluorescence microscope.

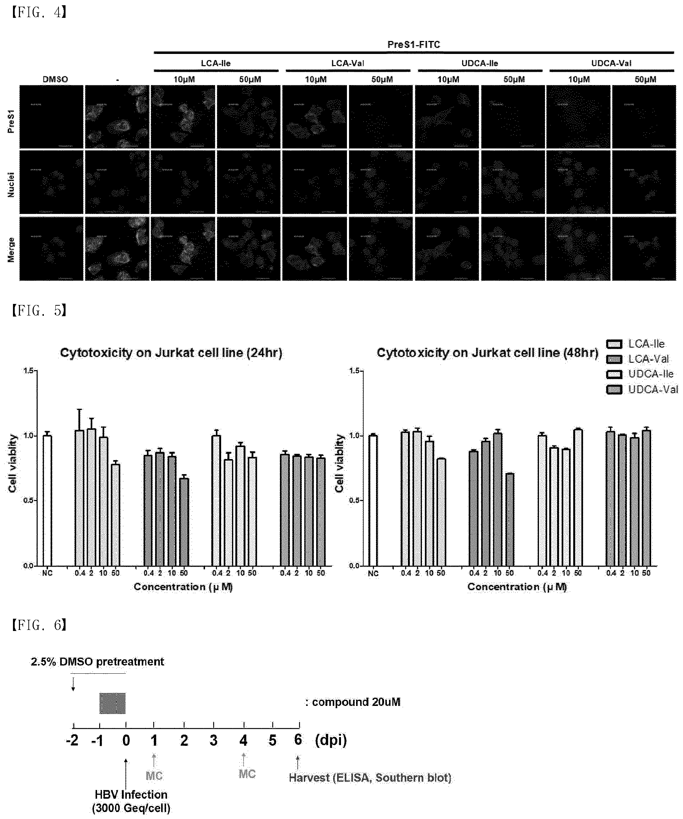

FIG. 4 is graphs showing a binding inhibitory ability of a fluorescent-labeled preS1 peptide through fluorescence imaging, for compounds of Example 1 (LCA-Ile), Example 44 (LCA-Val), Example 2 (UDCA-Ile), and Example 3 (UDCA-Val) according to the present disclosure.

Green fluorescence may be observed when PreS1 binds to a cell line expressing NTCP by FITC conjugated to PreS1. As a result of the experiment, when each compound in Example 1 (LCA-Ile), Example 44 (LCA-Val), Example 2 (UDCA-Ile), and Example 3 (UDCA-Val) was treated at two different concentrations (10 µM and 50 µM), compared to the HBV-induced group solely treated with PreS 1, it was found that the binding between PreS1 and NTCP was inhibited in a concentration dependent manner.

### <Experimental Example 3> Evaluation on cytotoxicity

For Example 1 (LCA-Ile), Example 44 (LCA-Val), Example 2 (UDCA-Ile), and Example 3 (UDCA-Val), cytotoxicity was evaluated in the Jurkat cell line, a human T lymphocyte-derived cell line. Jurkat cell lines were seeded at a density of 5 × 10⁴ on a 96 well plate for overnight incubation, and each compound was treated at a concentration of 0.2 µM ~ 50 µM for 24 or 48 hours. WST-1, a reagent for measuring cell viability, was loaded at 10 µl/well, and absorbance was measured at 440 nm using a microplate reader 2 hours later. The mean value of negative control (NC) group treated with only DMSO was calculated as 1 and the cytotoxicity for the remaining experimental group was evaluated.

FIG. 5 is graphs showing results of identifying toxicity at a cellular level, for compounds in Example 1 (LCA-Ile), Example 44 (LCA-Val), Example 2 (UDCA-Ile), and Example 3 (UDCA-Val) according to the present disclosure.

When the compounds were treated for 24 and 48 hours, cytotoxicity was barely observed. It was found that less toxicity was observed in Example 2 and Example 3 which are UDCA-based compounds than in Example 1 and Example 44 which are LCA lithocholic acid (LCA)-based compounds.

### <Experimental Example 4> Evaluation of an ability to inhibit intracellular HBV virus invasion

### 4-1. Experiment protocol for viral infection

In order to evaluate the ability to inhibit intracellular HBV virus entry (invasion), Enzyme-linked immunosorbent assay (ELISA) analysis to measure HBeAg, which is associated with high infectivity as an antigen in the HBV virus core portion, and Southern blot analysis to measure DNA of HBV were performed. The experiment protocol for viral infection to perform the above two analysis is as shown in FIG. 6.

Specifically, the HepG2-NTCP cell line was cultured on a 6-well plate, incubation was performed overnight, HBV inhibitors were treated at a concentration of 20 µM, and the positive control Myrcludex-B (MyrB) was treated at a concentration of 200 nM, followed by culture for 24 hours. After HBV infection into the cells to make HBV 3000 genome equivalent per ml/cell (Geq/cell), the cell culture medium was replaced (medium changing, MC) a day after the viral infection to remove the virus unattached to cell membranes. In order to measure an amount of virus that entered the cell, medium changing (MC) was performed once more 4 days after viral infection. 2 days after replacement, HBeAg ELISA analysis was performed using a supernatant, and Southern blot analysis was performed with cell pellets.

### 4-2. HBeAg ELISA analysis

HBeAg ELISA analysis was performed using an HBeAg ELISA kit (sanbio, Wantai HBeAg ELISA, cat#WB-2496) for the supernatant obtained by the method in Experimental Example 4-1.

First, a negative control group (Mock), positive control group (MyrB), and samples according to the present disclosure were loaded, by 50 µl each to be triplicate, onto a 96-well plate coated with capture Ab. In this case, Example 1 (LCA-ILE), Example 44 (LCA-Val), Example 52 (LCA-Tyr), Example 51 (LCA-Ser), Example 55 (LCA-Glu), Example 2 (UDCA-Ile), Example 3 (UDCA-Val), Example 25 (LCA-Val-Trp), Example 10 (DCA-Ile), Comparative Example 1 (LCA), and Comparative Example 2 (UDCA) were used as the sample.

The HRP-conjugated detection antibody was loaded into each well by 50 µl, followed by a reaction at 37°C for 30 minutes. Washing buffer (1X PBS, 0.5% Tween20) was added by 200 µl, and washing was performed 5 times. 50 µl of chromogen solution A and 50 µl of chromogen solution B were added to each well, and a reaction was carried out in the darkness at 37°C for 15 minutes. The stop solution was added to each well by 50 µl each to stop the reaction, and the amount of HBeAg was measured by measuring the absorbance (optical density, O.D.) level in the 450 nm wavelength band.

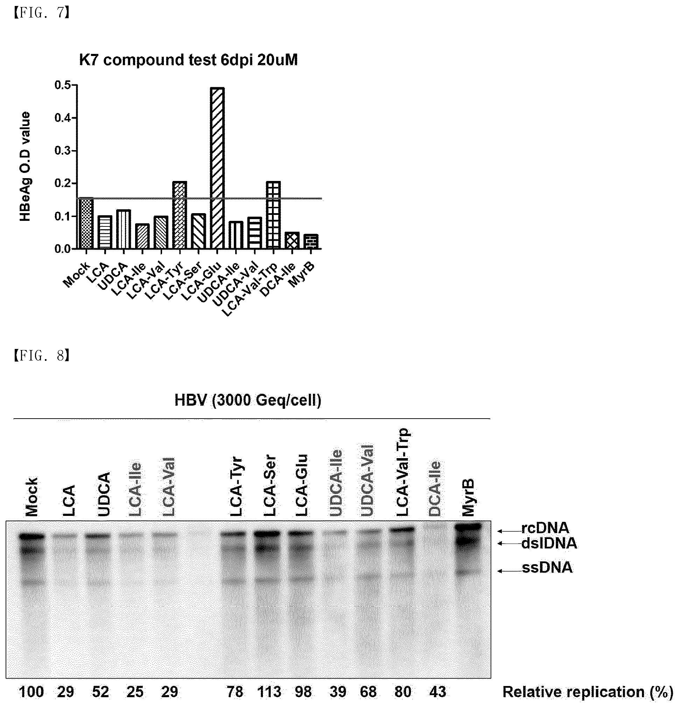

FIG. 7 is a graph showing a result of determining an HBeAg absorbance (O.D.) level through HBeAg ELISA analysis, for compounds in Example 1 (LCA-Ile), Example 44 (LCA-Val), Example 52 (LCA-Tyr), Example 51 (LCA-Ser), Example 55 (LCA-Glu), Example 2 (UDCA-Ile), Example 3 (UDCA-Val), Example 25 (LCA-Val-Trp), Example 10 (DCA-Ile), Comparative Example 1 (LCA), and Comparative Example 2 (UDCA) according to the present disclosure.

As a result of the analysis, it was found that, in the case of Example 1 (LCA-Ile), Example 44 (LCA-Val), Example 51 (LCA-Ser), Example 2 (UDCA-Ile), Example 3 (UDCA-Val), and Example 10 (DCA-Ile), the HBeAg absorbance level was relatively lower than that of the negative control group (Mock), and in the case of Example 1 (LCA-Ile), Example 44 (LCA-Val), Example 2 (UDCA-Ile), Example 3 (UDCA-Val), and Example 10 (DCA-Ile), the HBeAg absorbance level was lower than that of Comparative Example 1 (LCA) and Comparative Example 2 (UDCA), thereby having an excellent effect of inhibiting HBV viral infection.

### 4-3. Southern blot analysis

For the cell pellet obtained by the method of Experimental Example 4-1, Southern blot analysis for measurement of HBeAg DNA was performed using Example 1 (LCA-Ile), Example 44 (LCA-Val), Example 52 (LCA-Tyr), Example 51 (LCA-Ser), Example 55 (LCA-Glu), Example 2 (UDCA-Ile), Example 3 (UDCA-Val), Example 25 (LCA-Val-Trp), Example 10 (DCA-Ile), Comparative Example 1 (LCA), and Comparative Example 2 (UDCA).

First, the cell pellets were pipetted with 100 µl of HEPES lysis buffer (10 mM HEPES, 100 mM NaCl, 1 mM EDTA, 1% NP-40), placed on ice for 20 minutes, and then centrifuged with a centrifuge under the conditions at 4°C and 13000 rpm. After transferring the supernatant only to the new microtube, 4.5 µl of nuclease buffer I (10 mM CaCl₂, 12 mM MgCl₂, DNase 10 units) was added to carry out a reaction at 37°C for 2 hours, and 40 µl of 26% PEG solution was added, followed by a reaction on ice for 1 hour. After centrifuging with the centrifuge at 4°C and 13000 rpm for 30 minutes, and the supernatant was removed cleanly. After releasing the pellets with 100 µl of nuclease buffer II (10 mM Tris, 8 mM CaCl₂, 6mM MgCl₂), 287.5 µl of 0.5% SDS buffer (25 mM Tris, 10 mM EDTA, 0.5% SDS, 100 mM NaCl) was added, and 12.5 µl of 20 mg/ml proteinase K was added, followed by a reaction at 37°C for 2 hours. 400 µl of phenol-chloroform-isoamyl alcohol was added, centrifugation was performed at room temperature at 13000 rpm after voltexing, the supernatant was transferred to a new tube, 40 µl of 3 M NaOAc was added, and 800 µl of 100% ethanol was added for inverting, followed by a reaction overnight at -20°C. After performing centrifugation at 4°C and 13000 rpm for 30 minutes, 800 µl of 70% ethanol was added and mixed to the pellet obtained by removing the supernatant, centrifugation was performed at 4°C and 13000 rpm for 10 minutes, and then the supernatant was discarded. The ethanol was dried for 5 minutes to leave only the HBV DNA pellets, and then the pellets were dissolved again in 15 µl of TE buffer (10 mM Tris, 1 mM EDTA). HBV DNA samples were loaded in 1% agarose gel, and electrophoresis was performed. It was then immersed in denature buffer (0.25 N NaOH, 1 M NaCl) for 30 minutes to denature dsDNA of HBV into ssDNA. The DNA in the gel was transferred to a Nylon membrane using a transfer buffer (0.4 M NaOH), the membrane was soaked with a 2X SSC buffer (sigma, cat# 85639) for neutralization, followed by drying in air. The membrane was immersed in a church buffer (10% BSA, 0.5 M EDTA, 1 M NaPi, 20% SDS), and the radioisotopes were hybridized using a Mega prime kit (GE healthcare, RPN1607). After washing three times with washing buffer (2X SSC, 0.1% SDS), measurement was performed using radioisotope measurement equipment, and a relative replication rate (%) to the negative control group (Mock) was calculated and shown.

FIG. 8 is a graph showing results of identifying HBeAg DNA and relative replication rate (%) of quantified HBeAg DNA through southern blot analysis, for compounds in Example 1 (LCA-Ile), Example 44 (LCA-Val), Example 52 (LCA-Tyr), Example 51 (LCA-Ser), Example 55 (LCA-Glu), Example 2 (UDCA-Ile), Example 3 (UDCA-Val), Example 25 (LCA-Val-Trp), Example 10 (DCA-Ile), Comparative Example 1 (LCA), and Comparative Example 2 (UDCA) according to the present disclosure.

As a result, in the case of Example 1 (LCA-Ile), Example 44 (LCA-Val), Example 52 (LCA-Tyr), Example 55 (LCA-Glu), Example 2 (UDCA-Ile), Example 3 (UDCA-Val), Example 25 (LCA-Val-Trp), and Example 10 (DCA-Ile), relatively less amount of HBeAg DNA was detected compared to the negative control group (Mock), in a level less than 80% in Example 1 (LCA-Ile), Example 44 (LCA-Val), Example 52 (LCA-Tyr), Example 2 (UDCA-Ile), Example 3 (UDCA-Val), and Example 10 (DCA-Ile). In particular, in the case of Example 1 (LCA-Ile), Example 44 (LCA-Val), Example 2 (UDCA-Ile), Example 3 (UDCA-Val), and Example 10 (DCA-Ile), it was found that less than 70% was detected, thereby having the excellent effect of inhibiting intracellular entry of HBV virus.

Meanwhile, the compound represented by Chemical Formula 1 according to the present disclosure may be formulated in various forms depending on the purpose. The following is that illustrates several formulation methods that the compound represented by Chemical Formula 1 according to the present disclosure is included as an active ingredient, but the present disclosure is not limited thereto.

### <Preparation Example 1> Preparation of Powder

2 g of compound represented by Chemical Formula 1
1 g of lactose

The components above were mixed and filled in an airtight cloth to prepare powders.

### <Preparation Example 2> Preparation of tablets

100 mg of compound represented by Chemical Formula 1
100 mg of cornstarch
100 mg of lactose
2 mg of magnesium stearate

After mixing the components above, tablets were prepared by compression according to the conventional method of manufacturing tablets.

### <Preparation Example 3> Preparation of capsules

100 mg of compound represented by Chemical Formula 1
100 mg of cornstarch
100 mg of lactose
2 mg of magnesium stearate

After mixing the components above, the capsules were prepared by filling in a gelatin capsule according to the conventional method of manufacturing capsules.

### <Preparation Example 4> Preparation of injections

100 mg of compound represented by Chemical Formula 1
180 mg of mannitol
26 mg of Na2HPO4 · 2H2O
2974 mg of distilled water

According to a conventional method of manufacturing injections, injections were prepared by containing the components above in the suggested content.

### Health Functional Food Manufacturing Example

The compound represented by Chemical Formula 1 according to the present disclosure may be prepared in various forms of health functional foods depending on the purpose. The following illustrates methods of preparing several health functional foods in which active substances according to the present disclosure are included as active ingredients, but the present disclosure is not limited thereto.

### <Health Functional Food Manufacturing Example 1> Manufacturing of health functional food

100 mg of compound represented by Chemical Formula 1
Adequate amount of vitamin mixture
   70 µg of vitamin A acetate
   1.0 mg of vitamin E
   0.13 mg of vitamin B1
   0.15 mg of vitamin B2
   0.5 mg of vitamin B6
   0.2 µg of vitamin B12
   10 mg of vitamin C
   10 µg of biotin
   1.7 mg of niacinamide
   50 µg of folic acid
   0.5 mg of calcium pantothenate
Adequate amount of mineral mixture
   1.75 mg of iron(II) sulfate
   0.82 mg of zinc oxide
   25.3 mg of magnesium carbonate
   15 mg of potassium phosphate monobasic
   55 mg of potassium phosphate dibasic
   90 mg of potassium citrate
   100 mg of calcium carbonate
   24.8 mg of magnesium chloride

The composition ratio of the vitamin and mineral mixture above is achieved by a mixture composition of ingredients relatively suitable for health functional foods in a preferred example embodiment, but the combination ratio may be arbitrarily modified, and the above ingredients are mixed according to a conventional health functional food manufacturing method to prepare granules to be used for preparing health functional food compositions according to conventional methods.

### <Health Functional Food Manufacturing Example 2> Preparation of health functional beverages

100 mg of compound represented by Chemical Formula 1
100 mg of citric acid
100 mg of oligosaccharide
2 mg of plum concentrate
100 mg of taurine
500 mL in total by adding purified water

The above ingredients are mixed according to a conventional health beverage manufacturing method, the mixture is then stirred and heated at 85°C for about 1 hour, the resulting solution was filtered and collected in a sterile container 1 which is then sealed, sterilized, and refrigerated so as to be used to prepare the health beverage composition of the present disclosure. The composition ratio is achieved by mixing and composing ingredients relatively suitable for the favorite beverage in a preferred example embodiment, but the combination ratio may be arbitrarily modified according to regional and ethnic preferences such as demand class, demand country, and purpose of use.

Thus far, the present disclosure has been described on the preferred example embodiments. A person skilled in the art to which the present disclosure pertains will be able to understand that the present disclosure may be implemented in a modified form to the extent that it does not deviate from the essential characteristics of the present disclosure. Therefore, the disclosed example embodiments should be considered from a descriptive point of view, not a limited point. The scope of the present disclosure is not described in the foregoing, but shown particularly in the claims, and all differences within the equivalent range should be construed as included in the present disclosure.

## Claims

1. A compound represented by the following Chemical Formula 1, in which amino acids are bound to bile acid, an enantiomer thereof, a diastereomer thereof, or a pharmaceutically acceptable salt thereof: 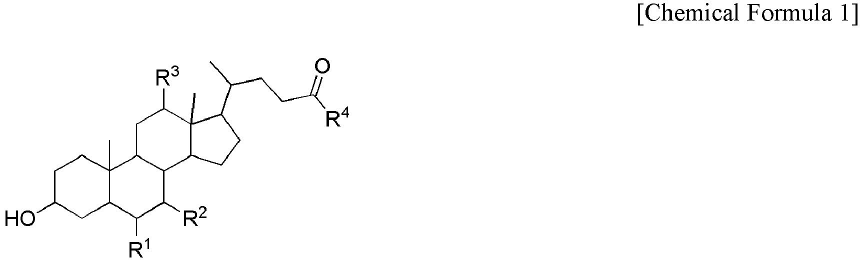
wherein, in the Chemical Formula 1,
R¹, R², and R³ are independently hydrogen or hydroxy;
R⁴ is 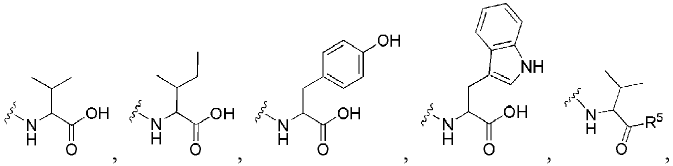 or 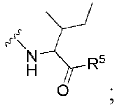 and
R⁵ is 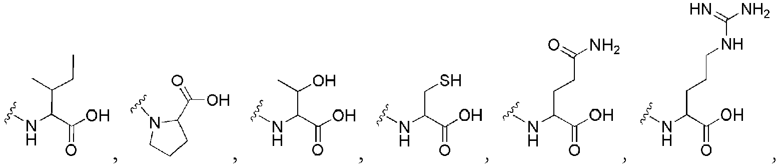 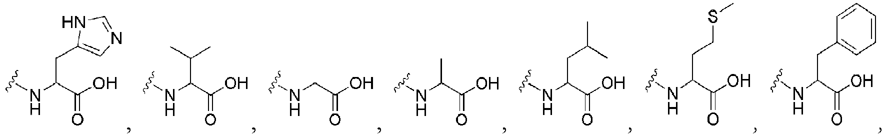 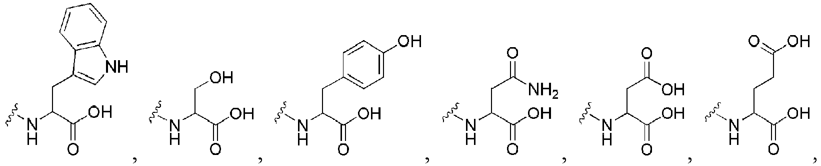 or

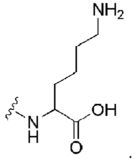

2. The compound, enantiomer thereof, diastereomer thereof, or pharmaceutically acceptable salt thereof of claim 1, wherein R⁴ of the compound represented by Chemical Formula 1 is selected from

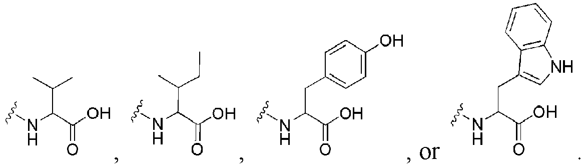

3. The compound, enantiomer thereof, diastereomer thereof, or pharmaceutically acceptable salt thereof of claim 1, wherein the bile acid is selected from the group consisting of lithocholic acid (LCA), ursodeoxycholic acid (UDCA), cholic acid (CA), hyodeoxycholic acid (HDCA), deoxycholic acid (DCA), and chenodeoxycholic acid (CDCA).

4. The compound, enantiomer thereof, diastereomer thereof, or pharmaceutically acceptable salt thereof of claim 1, wherein the compound represented by Chemical Formula 1 is any one selected from the following group of compounds:
1) (2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Ile);
2) (2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (UDCA-Ile);
3) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (UDCA-Val);
4) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (UDCA-Trp);
5) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (UDCA-Tyr);
6) (2S,3R)-3-methyl-2-((R)-4-((3R,5S, 7R,8R,9S, 10S, 12S, 13R, 14S, 17R)-3, 7,12-trihydroxy-10,13-dimethyl-hexadecahydro-1H-cyclopenta[a]phenanthren-17-yl)pentanamido)pentanoic acid (CA-Ile);
7) (S)-3-methyl-2-((R)-4-((3R,5S, 7R,8R,9S, 10S, 12S, 13R, 14S, 17R)-3, 7,12-trihydroxy-10,13-dimethyl-hexadecahydro-1H-cyclopenta[a]phenanthren-17-yl)pentanamido)butanoic acid (CA-Val);
8) (2S,3R)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylpentanoic acid (HDCA-Ile);
9) (S)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylbutanoic acid (HDCA-Val);
10) (2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid (DCA-Ile);
11) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylbutanoic acid (DCA-Val);
12) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(1H-indol-3-yl)propanoic acid (DCA-Trp);
13) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(4-hydroxyphenyl)propanoic acid (DCA-Tyr);
14) (2S,3R)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (CDCA-Ile);
15) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (CDCA-Val);
16) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (CDCA-Trp);
17) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (CDCA-Tyr);
18) 2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)acetic acid (LCA-Val-Gly);
19) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ala);
20) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylbutanoic acid (LCA-Val-Val);
21) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-methylpentanoic acid (LCA-Val-Leu);
22) (2S,3R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylpentanoic acid (LCA-Val-Ile);
23) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-(methylthio)butanoic acid (LCA-Val-Met);
24) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-phenylpropanoic acid (LCA-Val-Phe);
25) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-indol-3-yl)propanoic acid (LCA-Val-Trp);
26) 1-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoyl)pyrrolidine-2-carboxylic acid (LCA-Val-Pro);
27) (S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ser);
28) (2S,3S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)butanoic acid (LCA-Val-Thr);
29) (R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-mercaptopropanoic acid (LCA-Val-Cys);
30) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(4-hydroxyphenyl)propanoic acid (LCA-Val-Tyr);
31) (S)-4-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-oxobutanoic acid (LCA-Val-Asn);
32) (S)-5-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-5-oxopentanoic acid (LCA-Val-Gln);
33) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)succinic acid (LCA-Val-Asp);
34) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanedioic acid (LCA-Val-Glu);
35) (S)-6-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)hexanoic acid (LCA-Val-Lys);
36) (S)-5-guanidino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanoic acid (LCA-Val-Arg); and
37) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-imidazol-4-yl)propanoic acid (LCA-Val-His).

5. A pharmaceutical composition for preventing or treating hepatitis, comprising a compound represented by the following Chemical Formula 1, in which amino acids are bound to bile acid, an enantiomer thereof, a diastereomer thereof, or a pharmaceutically acceptable salt thereof: 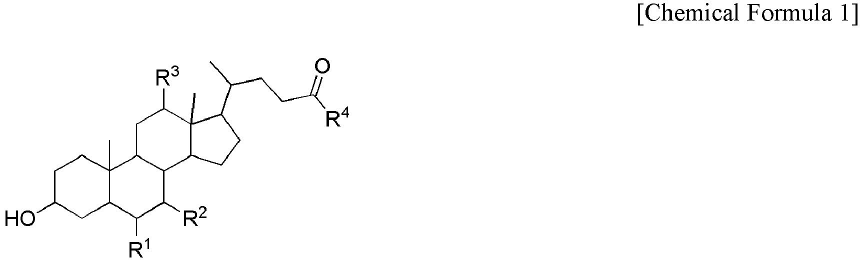
wherein, in the Chemical Formula 1,
R¹, R², and R³ are independently hydrogen or hydroxy;
R⁴ is R⁵, 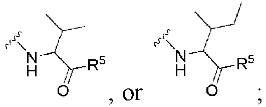 and
R⁵ is 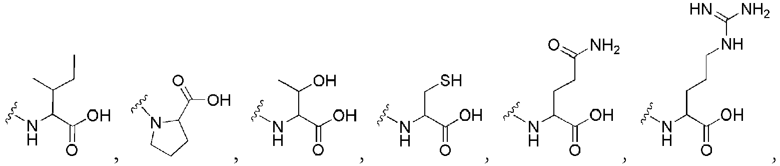 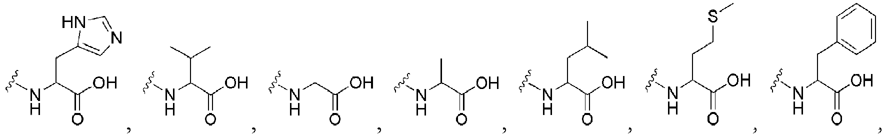 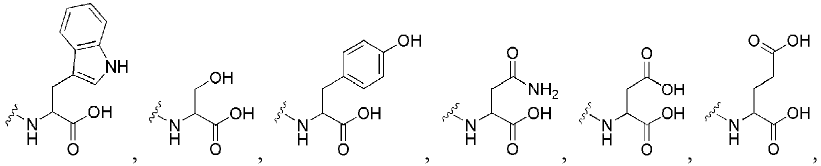 or

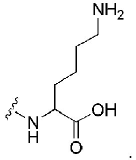

6. The pharmaceutical composition of claim 5, wherein the bile acid is selected from the group consisting of lithocholic acid (LCA), ursodeoxycholic acid (UDCA), cholic acid (CA), hyodeoxycholic acid (HDCA), deoxycholic acid (DCA), and chenodeoxycholic acid (CDCA).

7. The pharmaceutical composition of claim 5, wherein the hepatitis is hepatitis B or hepatitis D.

8. The pharmaceutical composition of claim 5, wherein the compound represented by Chemical Formula 1 is any one selected from the following group of compounds:
1) (2S,3R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Ile);
2) (2S,3R)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (UDCA-Ile);
3) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (UDCA-Val);
4) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (UDCA-Trp);
5) (S)-2-((R)-4-((3R,5S,7S,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (UDCA-Tyr);
6) (2S,3R)-3-methyl-2-((R)-4-((3R,5S, 7R,8R,9S, 10S, 12S, 13R, 14S, 17R)-3, 7,12-trihydroxy-10,13-dimethyl-hexadecahydro-1H-cyclopenta[a]phenanthren-17-yl)pentanamido)pentanoic acid (CA-Ile);
7) (S)-3-methyl-2-((R)-4-((3R,5S, 7R,8R,9S, 10S, 12S, 13R, 14S, 17R)-3, 7,12-trihydroxy-10,13-dimethyl-hexadecahydro-1H-cyclopenta[a]phenanthren-17-yl)pentanamido)butanoic acid (CA-Val);
8) (2S,3R)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylpentanoic acid (HDCA-Ile);
9) (S)-2-((R)-4-((3R,5R,6S,8S,9S,10R,13R,14S,17R)-3,6-dihydroxy-10,13-dimethyl-hexadecahydro-1H-cyclopenta[a]phenanthren-17-yl)pentanamido)-3-methylbutanoic acid (HDCA-Val);
10) (2S,3R)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylpentanoic acid (DCA-Ile);
11) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-methylbutanoic acid (DCA-Val);
12) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(1H-indol-3-yl)propanoic acid (DCA-Trp);
13) (S)-2-((R)-4-((3R,5R,8R,9S,10S,12S,13R,14S,17R)-3,12-dihydroxy-10,13-dimethyl-hexadecahydro-1H-cyclopenta[a]phenanthren-17-yl)pentanamido)-3-(4-hydroxyphenyl)propanoic acid (DCA-Tyr);
14) (2S,3R)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylpentanoic acid (CDCA-Ile);
15) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-methylbutanoic acid (CDCA-Val);
16) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(1H-indol-3-yl)propanoic acid (CDCA-Trp);
17) (S)-2-((R)-4-((3R,5S,7R,8R,9S,10S,13R,14S,17R)-3,7-dihydroxy-10,13-dimethyl-hexadecahydro-1H-cyclopenta[a]phenanthren-17-yl)pentanamido)-3-(4-hydroxyphenyl)propanoic acid (CDCA-Tyr);
18) 2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)acetic acid (LCA-Val-Gly);
19) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ala);
20) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylbutanoic acid (LCA-Val-Val);
21) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-methylpentanoic acid (LCA-Val-Leu);
22) (2S,3R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-methylpentanoic acid (LCA-Val-Ile);
23) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-(methylthio)butanoic acid (LCA-Val-Met);
24) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-phenylpropanoic acid (LCA-Val-Phe);
25) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-indol-3-yl)propanoic acid (LCA-Val-Trp);
26) 1-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoyl)pyrrolidine-2-carboxylic acid (LCA-Val-Pro);
27) (S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)propanoic acid (LCA-Val-Ser);
28) (2S,3S)-3-hydroxy-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)butanoic acid (LCA-Val-Thr);
29) (R)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-mercaptopropanoic acid (LCA-Val-Cys);
30) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(4-hydroxyphenyl)propanoic acid (LCA-Val-Tyr);
31) (S)-4-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-4-oxobutanoic acid (LCA-Val-Asn);
32) (S)-5-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-5-oxopentanoic acid (LCA-Val-Gln);
33) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)succinic acid (LCA-Val-Asp);
34) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)pentanedioic acid (LCA-Val-Glu);
35) (S)-6-amino-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)hexanoic acid (LCA-Val-Lys);
36) (S)-5-guanidino-2-((S)-2-((R)-4-((3R,5R,8R,9S, 10S, 13R, 14S, 17R)-3-hydroxy-10,13-dimethyl-hexadecahydro-1H-cyclopenta[a]phenanthren-17-yl)pentanamido)-3-methylbutanamido)pentanoic acid (LCA-Val-Arg);
37) (S)-2-((S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanamido)-3-(1H-imidazol-4-yl)propanoic acid (LCA-Val-His);
38) 1-((R)-4-((3R,5R,8R,9S,10S,13R,14S,17R)-3-hydroxy-10,13-dimethyl-hexadecahydro-1H-cyclopenta[a]phenanthren-17-yl)pentanoyl)pyrrolidine-2-carboxylic acid (LCA-Pro);
39) (2S,3S)-3-hydroxy-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)butanoic acid (LCA-Thr);
40) (R)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-mercaptopropanoic acid (LCA-Cys);
41) (S)-5-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-5-oxopentanoic acid (LCA-Gln);
42) (S)-5-guanidino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)pentanoic acid (LCA-Arg);
43) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(1H-imidazol-5-yl)propanoic acid (LCA-His);
44) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylbutanoic acid (LCA-Val);
45) 2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-acetic acid (LCA-Gly);
46) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-propanoic acid (LCA-Ala);
47) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-methylpentanoic acid (LCA-Leu);
48) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-4-(methylthio)butanoic acid (LCA-Met);
49) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-phenylpropanoic acid (LCA-Phe);
50) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(1H-indol-3-yl)propanoic acid (LCA-Trp);
51) (S)-3-hydroxy-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)propanoic acid (LCA-Ser);
52) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-3-(4-hydroxyphenyl)propanoic acid (LCA-Tyr);
53) (S)-4-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)-4-oxobutanoic acid (LCA-Asn);
54) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)succinic acid (LCA-Asp);
55) (S)-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)pentanedioic acid (LCA-Glu); and
56) (S)-6-amino-2-((R)-4-((3R,5R,8R,9S,10S,13R,14S,17R)-3-hydroxy-10,13-dimethyl-hexadecahydro-1H-cyclopenta[a]phenanthren-17-yl)pentanamido)hexanoic acid (LCA-Lys).

9. A health functional food composition for preventing or alleviating hepatitis, comprising a compound represented by the following Chemical Formula 1, in which amino acids are bound to bile acid, an enantiomer thereof, a diastereomer thereof, or a food-acceptable carrier thereof: 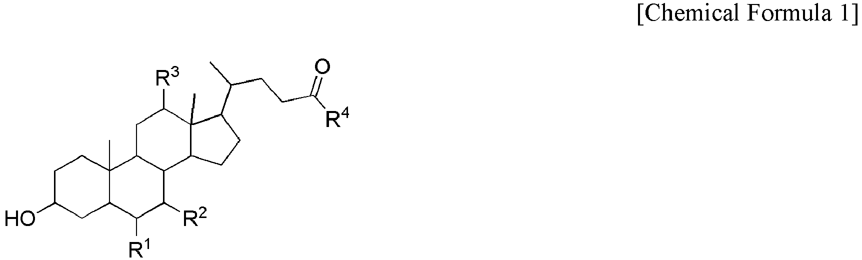
wherein, in the Chemical Formula 1,
R¹, R², and R³ are independently hydrogen or hydroxy;
R⁴ is R⁵, 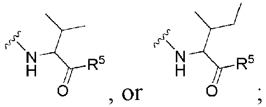 and
R⁵ is 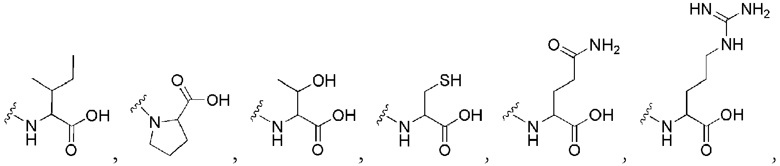 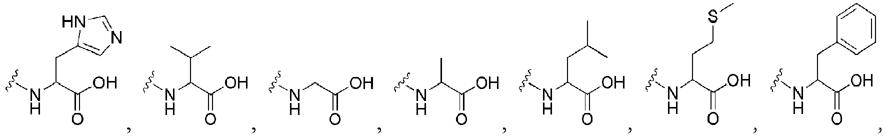 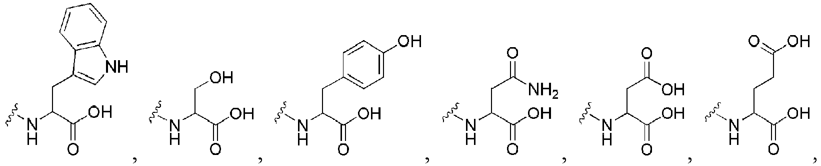 or

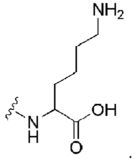

10. The health functional food composition of claim 9, wherein the bile acid is selected from the group consisting of lithocholic acid (LCA), ursodeoxycholic acid (UDCA), cholic acid (CA), hyodeoxycholic acid (HDCA), deoxycholic acid (DCA), and chenodeoxycholic acid (CDCA).

11. The health functional food composition of claim 9, wherein the hepatitis is hepatitis B or hepatitis D.
